# EUROPEAN PATENT APPLICATION

(11) **EP 3 000 820 A1**
(43) Date of publication of application: **30.03.2016**
(21) Application number: 14186597.2
(22) Date of filing: 26.09.2014
(51) Int. Cl.: C07H 15/04, A61K 39/00, A61K 31/7028, A61K 31/715, C07K 16/00, C07K 19/00, A61P 31/04

(54) **Synthetic vaccines against Streptococcus pneumoniae serotype 8**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to synthetic saccharides of general formula (**I**) that are related to *Streptococcus pneumoniae* serotype 8 capsular polysaccharide, conjugates thereof and the use of said saccharides and conjugates for raising a protective immune response in a human and/or animal host. Furthermore, the synthetic saccharide structures of general formula (**I**) are useful as marker in immunological assays for detection of antibodies against *Streptococcus pneumoniae* bacteria.

## Description

### Field of the invention

The present invention relates to synthetic saccharides of general formula (I) that are related to *Streptococcus pneumoniae* serotype 8 capsular polysaccharide, conjugates thereof and the use of said saccharides and conjugates for raising a protective immune response in a human and/or animal host. Furthermore, the synthetic saccharide structures of general formula (I) are useful as marker in immunological assays for detection of antibodies against *Streptococcus pneumoniae* bacteria

### Background of the invention

*Streptococcus pneumoniae* is a Gram-positive, encapsulated bacterium that is a main cause of infections of the respiratory tract and can lead to severe invasive pneumococcal disease (IPD). More than 90 different pneumococcal serotypes have been described to date. These are classified by the structure of their capsular polysaccharide (CPS), which is unique to each serotype. Consequently, the immune response generated against the CPS varies between different serotypes. This is used to generate specific antibodies in rabbits against the antigen of each serotype. Cross-reactivity between these specific antibodies and other serotypes than those they were raised against is often observed, due to structural similarities of the CPS of different serotypes. Due to its immunological properties, CPS is used as the main component of S. *pneumoniae* vaccines.

The first efficient vaccine that contained the CPS of four different serotypes was described in 1945. It then took over thirty years until a vaccine was introduced that covered 14 serotypes, shortly followed by a 23-valent vaccine. However, these polysaccharide vaccines had several shortcomings. They were not able to elicit a long-lasting protection and were not effective in the populations most vulnerable to infection, namely children under two years of age as well as immunodeficient and elderly patients. These shortcomings result from the immunology of carbohydrates and were overcome by the introduction of carbohydrate-protein conjugate vaccines. The first pneumococcal conjugate vaccines were the seven-valent (PCV-7) and 10-valent (PCV-10) vaccine. PCV-7 was later replaced with the most recent vaccine (PCV-13), which contains the CPS-glycoconjugates of 13 different serotypes.

The currently marketed vaccines are effective in North America and Europe for individuals of a particular age. The manufacturing process for these vaccines is complex and results in a higher price. Therefore, the vaccine is unaffordable in most developing countries. It is the object of the present invention to provide affordable synthetic saccharide vaccines that contain most of the prevalent serotypes of the developing world.

*Streptococcus pneumoniae* serotype 8 has been associated with serotype replacement and cases of invasive pneumococcal disease increased steadily in the last decades.

*Streptococcus pneumoniae* type 8 is one of the prevalent S. *pneumoniae* serotypes. The structure of the native Sp8 repeating unit is a tetrasaccharide with the sequence →4)-Glcα-(1→74)-Gala-(1→74)-GlcAβ-(1→74)-Glcβ(1→ (J. Am. Chem. Soc. 1957, 79 (11), 2787):

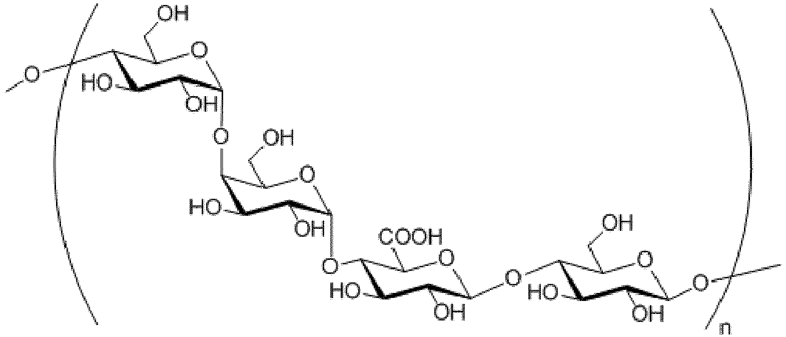

Interestingly, the native serotype 8 tetrasaccharide repeating unit harbors the disaccharide repeating unit of serotype 3. Consequently, cross-reactivity has been found between sera against serotypes 3 and 8, although precipitation of antibodies by the respective heterologous polysaccharide is incomplete. Insufficient epitope overlap may be the reason why capsular polysaccharides (CPS) from both serotypes were included in the 23-valent polysaccharide vaccine despite extensive considerations about cross-reactivity before manufacturing this vaccine.

It is the objective of the present invention to provide synthetic saccharides of general formula (I) that are related to *Streptococcus pneumoniae* serotype 8 capsular polysaccharides. Said saccharides are suitable to be conjugated to an immunogenic carrier to provide conjugates and pharmaceutical composition thereof that are useful for prevention and/or treatment of diseases associated with *Streptococcus pneumoniae,* and more specifically against diseases associated with *Streptococcus pneumoniae* serotype 8. Furthermore, the synthetic saccharides of general formula (I) are useful as marker in immunological assays for detection of antibodies against *Streptococcus pneumoniae* bacteria.

The objective of the present invention is solved by the teaching of the independent claims. Further advantageous features, aspects and details of the invention are evident from the dependent claims, the description, the figures, and the examples of the present application.

### Description of the invention

### Definitions

The term "linker" as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

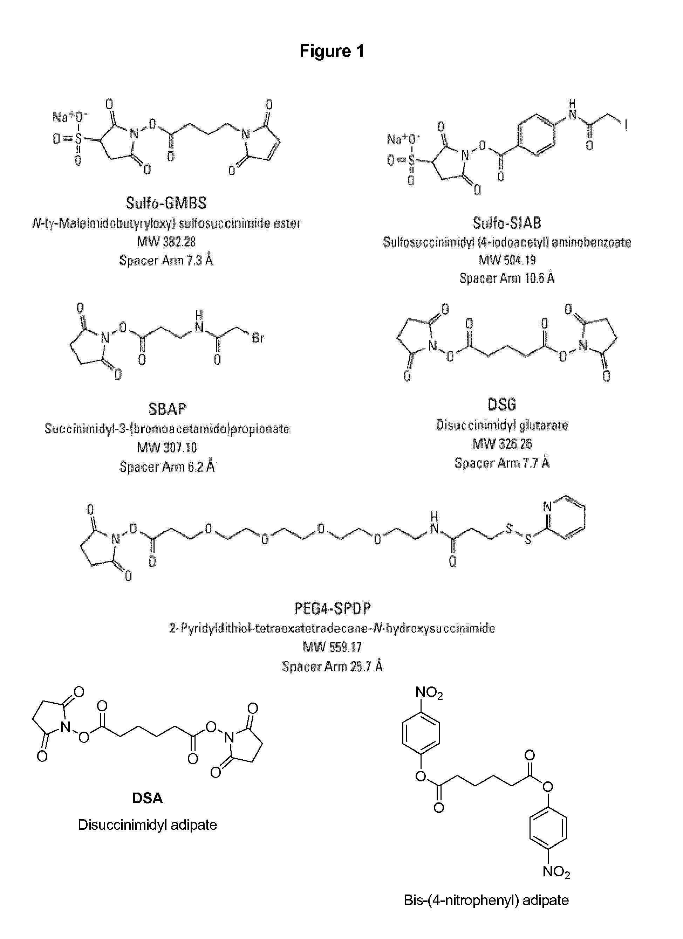

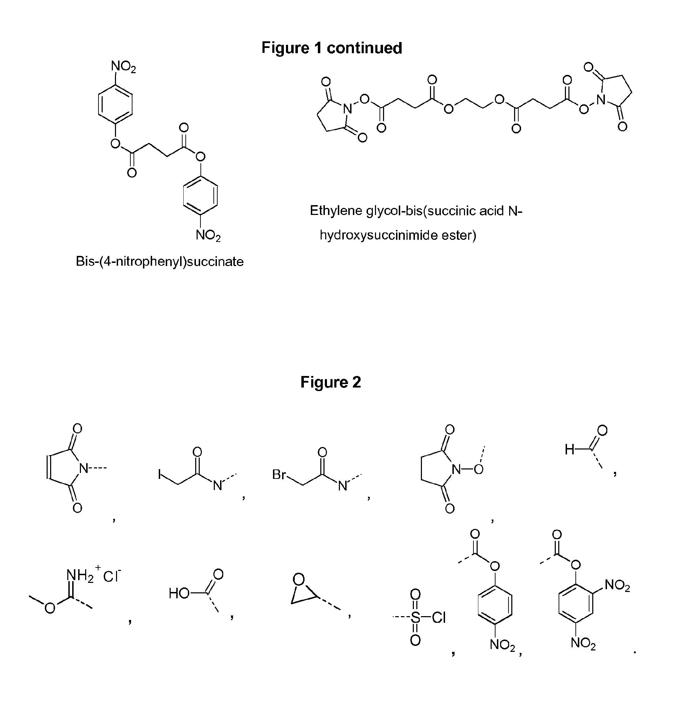

As used herein, the term "interconnecting molecule" refers to a bifunctional molecule containing functional group X and functional group Y, wherein functional group X is capable of reacting with the terminal amino group on the linker L and the functional group Y is capable of reacting with a functionality present on an immunogenic carrier or on a solid support. **Figure 1** displays examples of commercially available interconnecting molecules, but does not restrict the interconnecting molecules that can be used according to the present invention to the examples displayed herein.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the person skilled in the art, classically recognized examples of adjuvants include:
- mineral-containing compositions, including calcium salts and aluminium salts (or mixtures thereof). Calcium salts include calcium phosphate. Aluminium salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminium hydroxide and aluminium phosphate may be also used. The invention can use any of the "hydroxide" or "phosphate" adjuvants that are in general used as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;
- saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;
- microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2*S*,3*S*,4*R*)-1-*O*-(α-D-galactopyranosyl)-2-(*N-*hexacosanoylamino)-1,3,4-octadecanetriol], CRONY- 101, 3"-sulfo-galactosyl-ceramide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (e.g. Freund's adjuvant).

Theoretically, each molecule or substance that is able to favor or amplify a particular situation in the cascade of immunological events, ultimately leading to a more pronounced immunological response, can be defined as an adjuvant.
In principle, through the use of adjuvants in vaccine formulations, one can
- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.
Although little is known about their mode of action, it is currently believed that adjuvants augment immune responses by one of the following mechanisms:
- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;

- mimicking danger inducing signals from stressed or damaged cells, which serve to initiate an immune response;
- inducing the production of immunomodulatory cytokines;
- biasing the immune response towards a specific subset of the immune system; and
- blocking the rapid dispersal of the antigen challenge.

Saccharides are known by the person skilled in the art as TI-2 (T cell independent-2) antigens and poor immunogens. Therefore, to produce a saccharide-based vaccine, said saccharides are conjugated to an immunogenic carrier to provide a conjugate, which presents an increased immunogenicity in comparison with the saccharide. In this context the term "immunogenic carrier" is defined as a structure, which is conjugated to the saccharide to form a conjugate that presents an increased immunogenicity in comparison with the saccharide *per se.* Thus, the conjugation of the saccharides to the immunogenic carrier has as effect the stimulation of the immune response against said saccharide, without inducing an immune response against the said immunogenic carrier.

The present invention provides a saccharide of general formula (I)

**V*-[Uₓ₊₃-Uₓ₊₂-Uₓ₊₁-Uₓ]ₙ-V-O-L-NH₂** (**I**)

wherein
x is an integer selected from 1, 2, 3 and 4;
n is an integer selected from 1, 2 and 3; 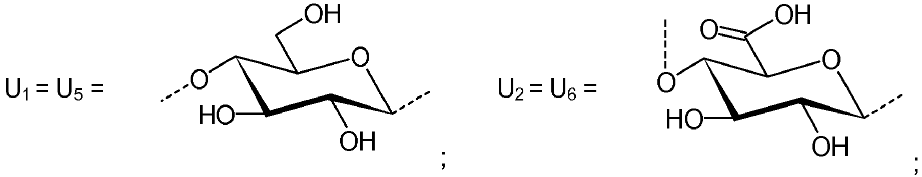 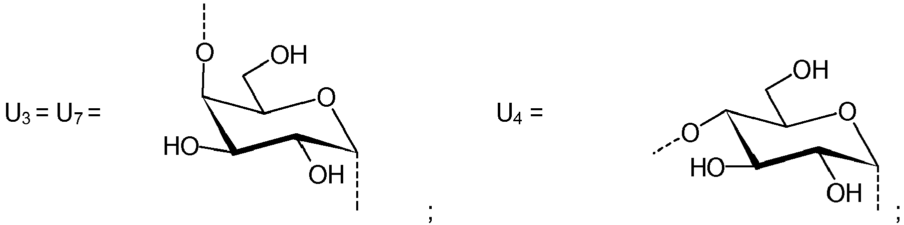
-V- represents a bond, -Uₓ₊₃-, -Uₓ₊₃-Uₓ₊₂- or -Uₓ₊₃-Uₓ₊₂-Uₓ₊₁-;
V*- represents H-, H-Uₓ-, H-Uₓ₊₁-Uₓ-, H- U_{X+2}-Uₓ₊₁-Uₓ- ;
L represents a linker.

-L- is defined as a linker and is part of the fragment -O-L-NH₂. Thus, the linker -L- is bound to an oxygen atom and to the nitrogen atom of the NH₂-group. It is preferred that at least two carbon atoms of the linker are between the oxygen atom and the NH₂-group, like -O-C-C-NH₂ and -S-C-C-NH₂. The linker -L- can be an aliphatic chain, wherein the aliphatic chain can optionally include an aromatic chain inserted in it, or a number of heteroatoms oscillating from 0 to 10.

The linker L preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-L-NH₂) and the NH₂-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the NH₂-group) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -L-, or the shortest chain is fully or partially fluorinated. The linker -L- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L- may also contain amide (-NH-CO-, -CO-NH-) and/or urea (-NH-CO-NH-) residues and preferably only one amide or urea residue. The linker may also contain substituents and preferably two substituents such as R¹⁰ and R¹¹ or four substituents such as R¹⁰, R¹¹, R¹⁵ and R¹⁴, which have the meanings as defined herein and which are preferably selected from: -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂;

In case the linker -L- is fluorinated, more than two substituents -F are preferred.

Preferably the linker -L- is selected from: -CH₂-, -(CH₂)₂-, -(CH₂)₃-, -(CH₂)₄-, -(CH₂)₅-, -(CH₂)₆-, -(CH₂)₇- -(CH₂)₈-, -(CH₂)₉-, -(CH₂)₁₀-, -CF₂-, -(CF₂)₂-, -(CF₂)₃-, -(CF₂)₄-, -(CF₂)₅- -(CF₂)₆-, -(CF₂)₇- -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂-, -CH₂-O-(CH₂)₂-, -(CH₂)₂-O-CH₂-, -(CH₂)₃-O-(CH₂)₂-, -(CH₂)₂-O-(CH₂)₃-, -(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a}-L^{d}-L⁶-;
wherein
-L^{a}- is selected from: -(CH₂)ₒ-, -(CF₂)ₒ-, -(CH₂-CH₂-O)ₒ-C₂H₄-, -(CH₂-CH₂-O)ₒ-CH₂-, -(CR¹⁰R¹¹)ₒ-, 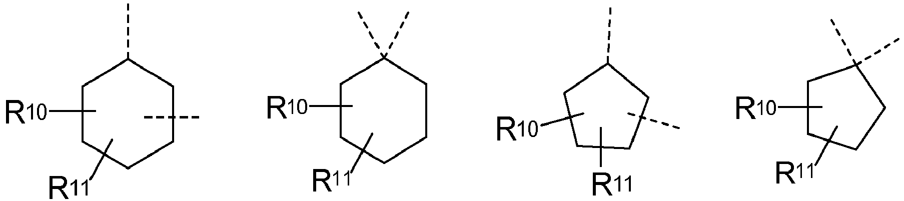 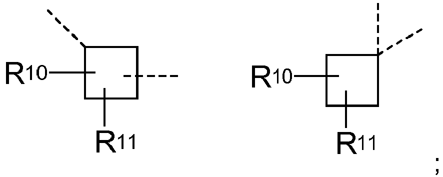
-L^{b}- and -L^{c}- are independently of each other selected from: -O-, -NH-C(O)-NH-, -NH-C(S)-NH-, -NH-C(O)-, -C(O)-NH-, -NH-C(O)-O-, -NR⁹-, -NR¹⁸-, -SO₂-, 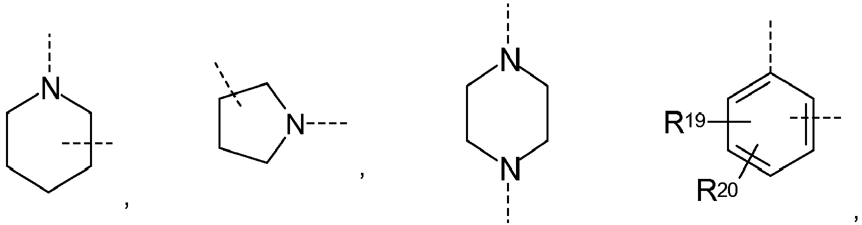 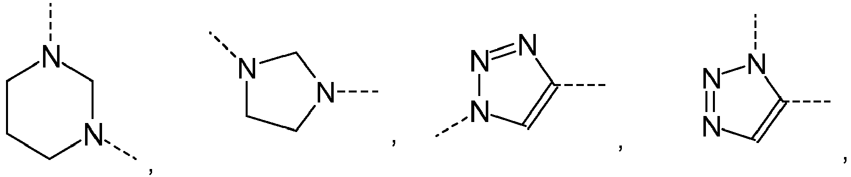 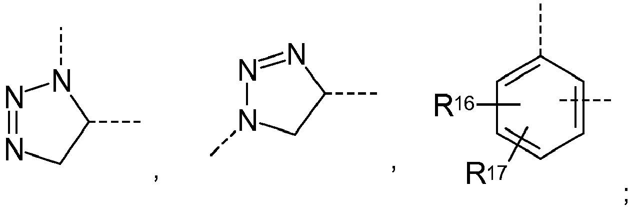
-L^{d}- represents -(CH₂)_{q}-, -(CF₂)_{q}-, -(CR¹²R¹³)_{q}-, -(CH₂-CH₂-O)_{q}-C₂H₄-, -(CH₂-CH₂-O)_{q}-CH₂-, 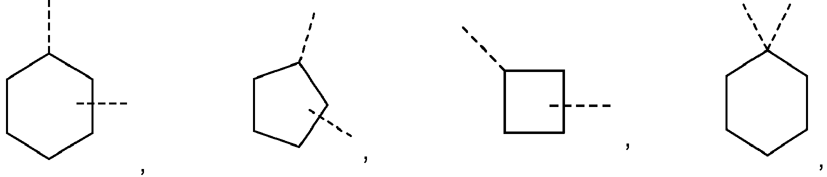 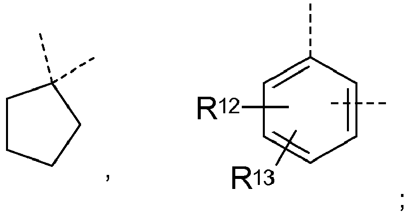
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁-, -(CH₂)ₚ₁-O-(CH₂)ₚ₂-, -(CR¹⁴R¹⁵)ₚ₁--(CR¹⁴R¹⁵)ₚ₁-O-(CR²¹R²²)ₚ₂-, 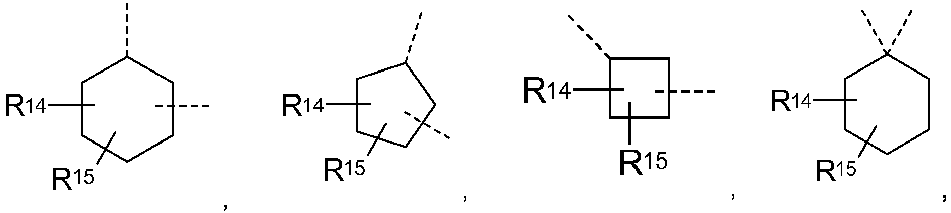 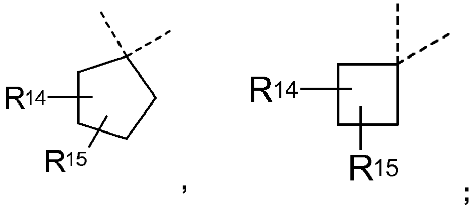
R⁹ and R¹⁸ are independently of each other selected from: -CH₃, -C₂H₅, -C₃H₇, and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o, q, p1 and p2 are independently of each other an integer selected from 1, 2, 3, 4, 5, 6, 7, 8, 9, and 10.

The saccharides of the present invention bear basic and/or acidic substituents and they may form salts with organic or inorganic acids or bases.
Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (*o*, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples of suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of a base, selected out of the group mentioned above.

It is clear for the skilled person in the art of carbohydrate chemistry that the saccharides of general (I) are not containing -O-O- bonds and or sugar fragments (Uₓ, Uₓ₊₁, Uₓ₊₂, Uₓ₊₃) connected or bound to each other *via* their anomeric or C-1 carbons.

The saccharides of general formula (I) are able to induce a protective immune response against *S*. *pneumoniae* serotype 8 bacteria in a human and/or animal host.

Another aspect of the present invention is directed to a saccharide of general formula (**II**)

**V*-[Uₓ₊₃-Uₓ₊₂-Uₓ₊₁-Uₓ]ₙ-O-L-NH₂** (II)

wherein x, n, L, Uₓ, Uₓ₊₁, Uₓ₊₂, Uₓ₊₃ and V* have the meanings as defined herein.

Thus, a saccharide of general formula **(II-a), (II-b), (II-c)** and **(II-d),** wherein n, L and V* have the meanings defined herein are especially preferred.

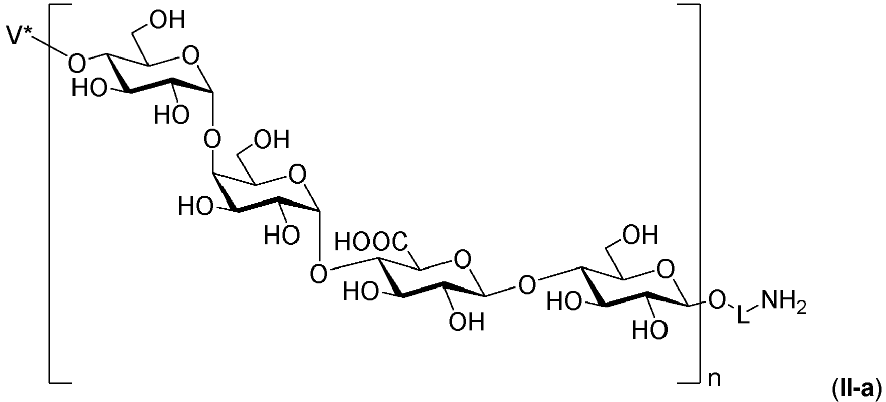

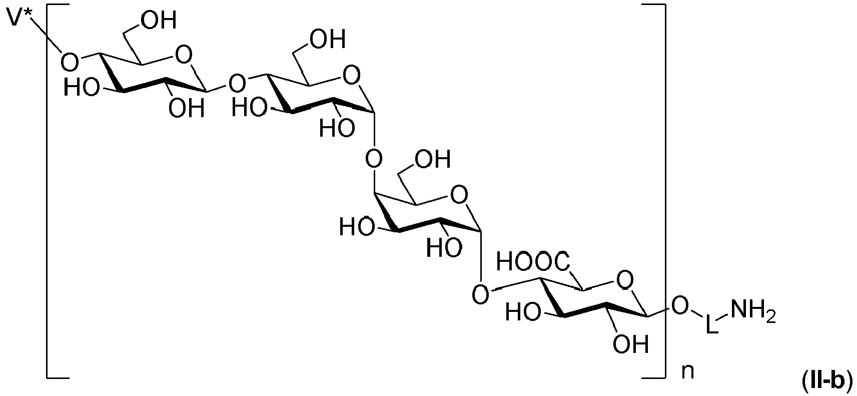

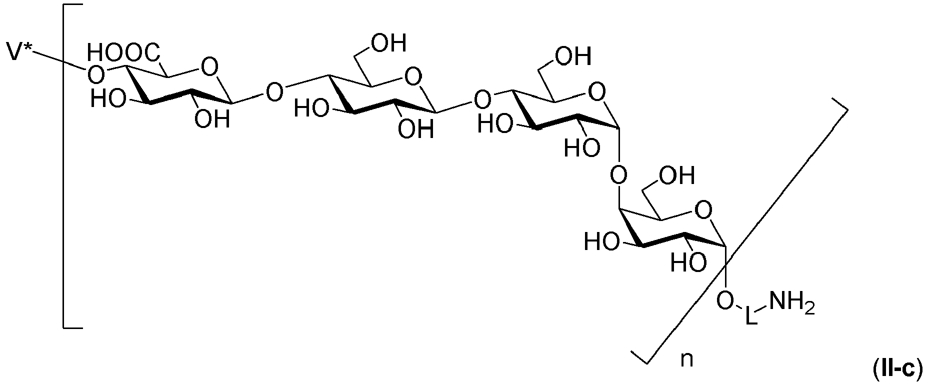

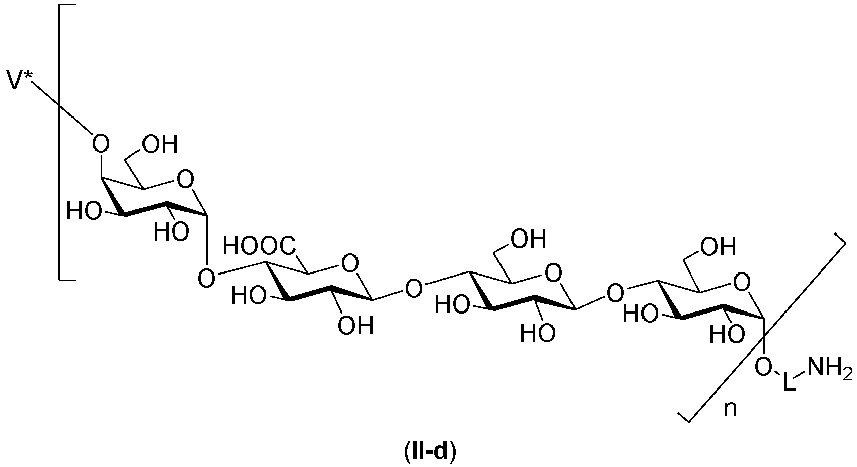

A preferred embodiment according to the present invention is directed to a saccharide of general formula **(III)**

**V*-[Uₓ₊₃-Uₓ₊₂-Uₓ₊₁-Uₓ]ₙ-Uₓ₊₃-O-L-NH₂** (III)

wherein x, n, L, Uₓ, Uₓ₊₁, Uₓ₊₂, Uₓ₊₃ and V* have the meanings defined herein.

Thus, a saccharide of general formula **(III-a), (III-b), (III-c)** and **(III-d),** wherein n, L and V* have the meanings defined herein are especially preferred.

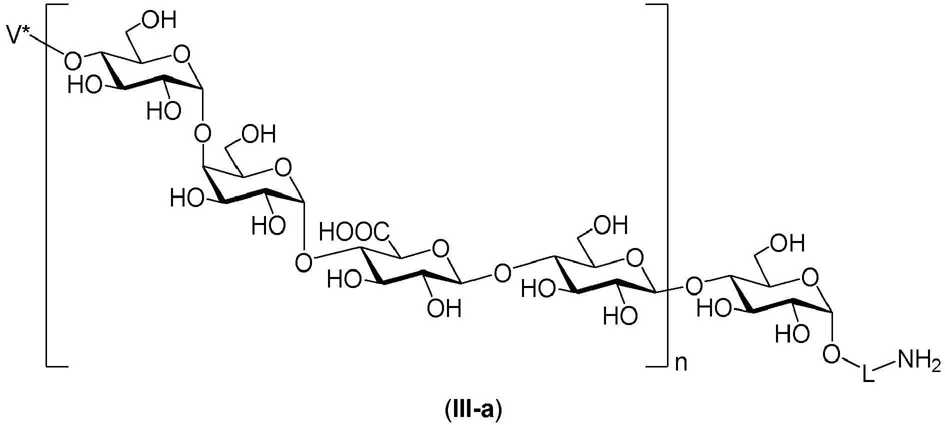

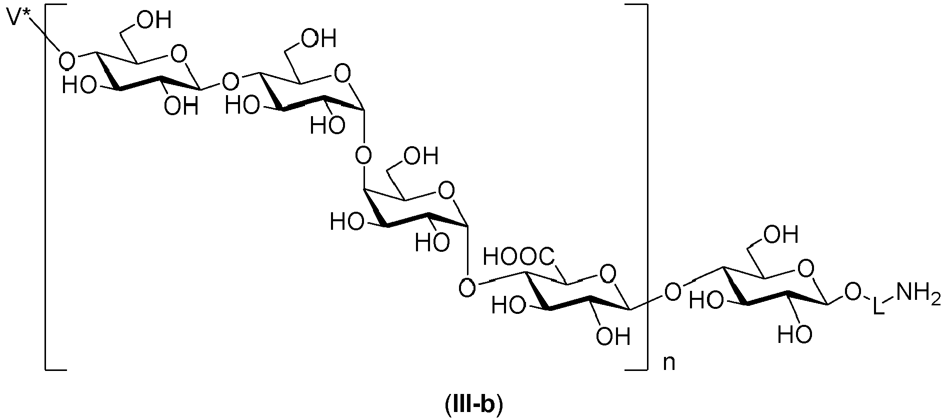

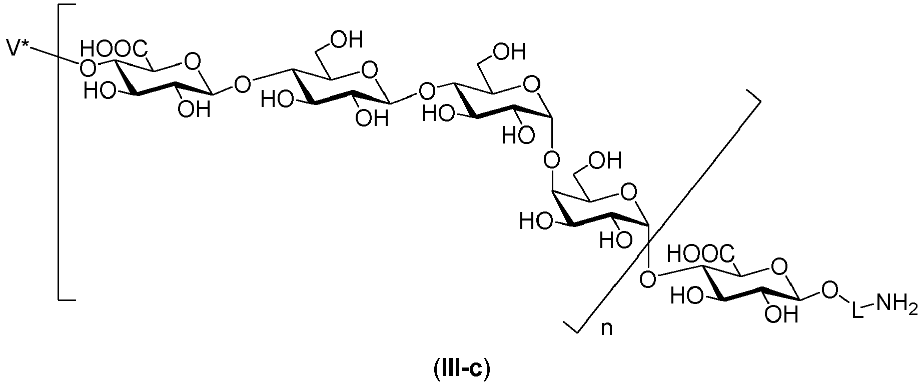

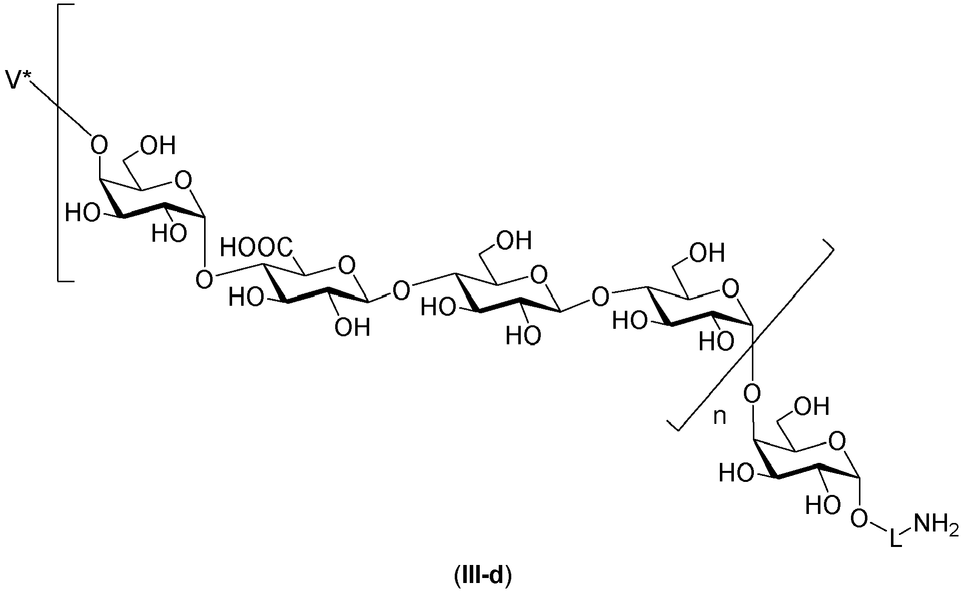

Another preferred embodiment of the present invention refers to a saccharide of general formula **(IV)**

**V*-[Uₓ₊₃-Uₓ₊₂-Uₓ₊₁-Uₓ]ₙ-Uₓ₊₃-Uₓ₊₂-O-L-NH₂** (IV)

wherein x, n, L, Uₓ, Uₓ₊₁, Uₓ₊₂, Uₓ₊₃ and V* have the meanings defined herein. Therefore, a saccharide of general formula **(IV-a), (IV-b), (IV-c)** or **(IV-d),** wherein n, V* and L have the meanings defined herein is also preferred.

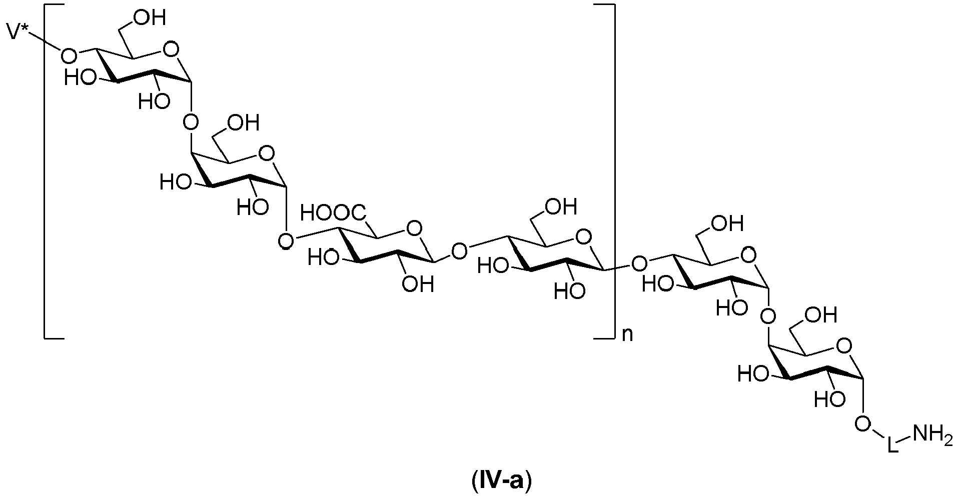

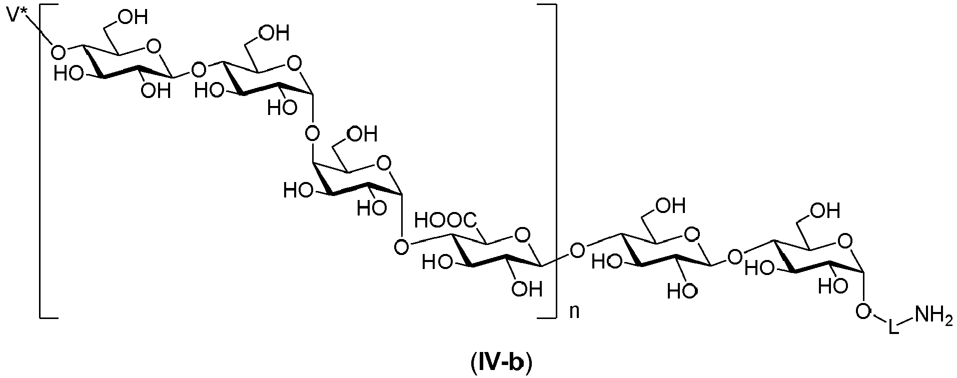

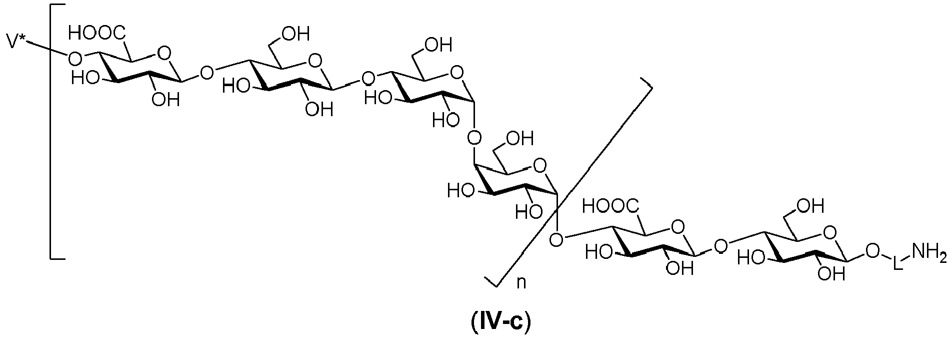

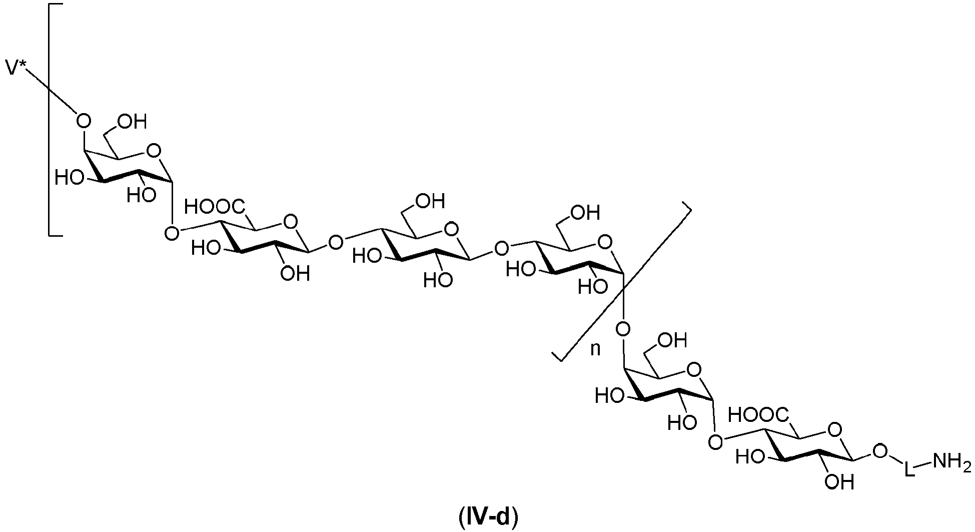

A saccharide of general formula **(V)**

**V*-[Uₓ₊₃-Uₓ₊₂-Uₓ₊₁-Uₓ]ₙ-Uₓ₊₃-Uₓ₊₂-Uₓ₊₁-O-L-NH₂** (V)

wherein x, n, L, Uₓ, Uₓ₊₁, Uₓ₊₂, Uₓ₊₃ and V* have the meanings defined herein is also preferred. A further preferred saccharide according to the present invention is a saccharide of general formula **(V-a), (V-b), (V-c)** and **(V-d),** wherein V*, n and L have the meanings defined herein.

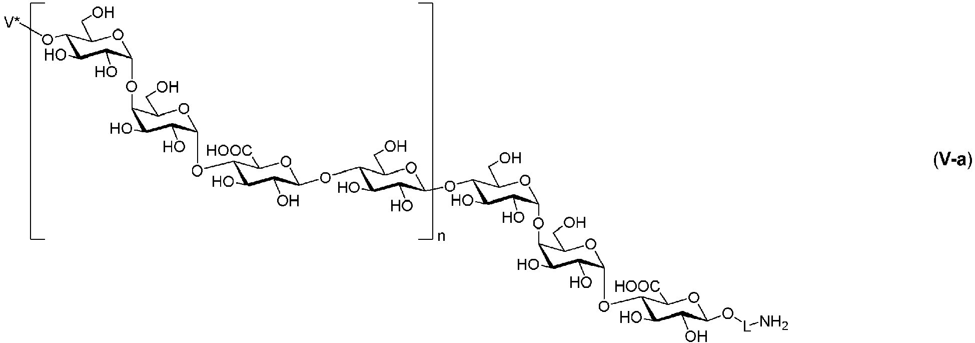

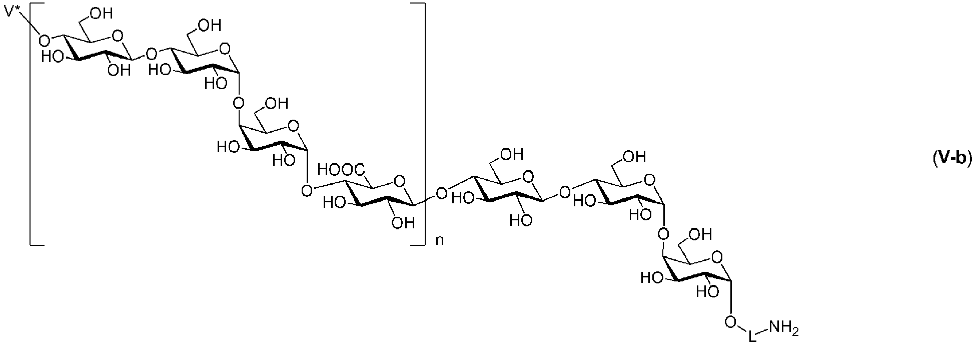

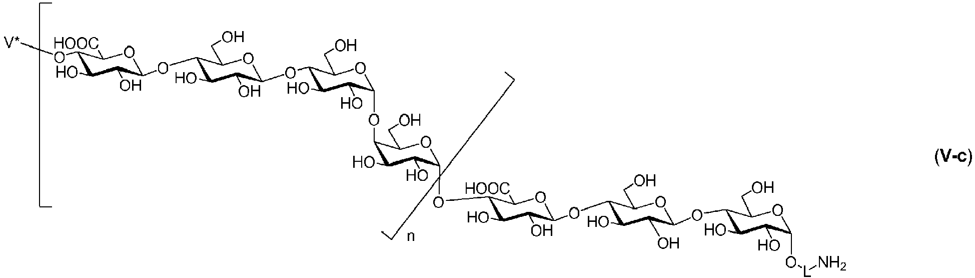

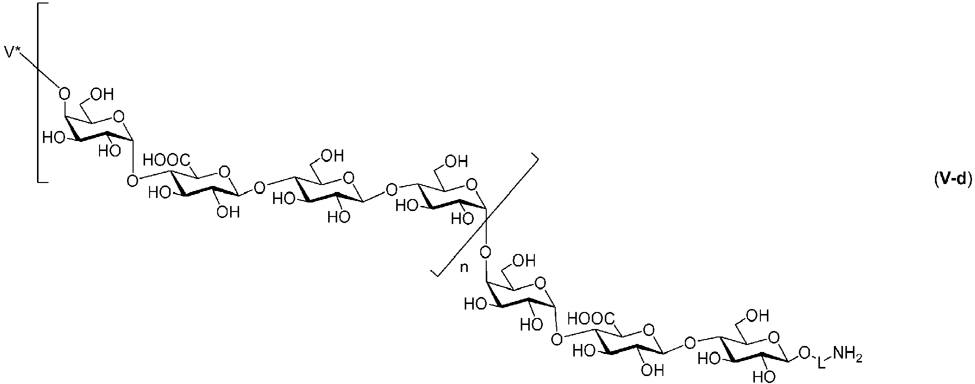

Preferably the integer x represents 3. Therefore, a compound of general formula **(I), (II), (III), (IV)** or **(V),** wherein x represents 3 is especially preferred. Even more preferred is a compound of general formula (I), **(II), (III), (IV)** or **(V),** wherein x represents 3 and V* represents H-.

Preferably the linker -L- is selected from: -L^{a}-, -L^{a}-L^{e}-, -L^{a}-L^{b}-L^{e}-, -L^{a}-L^{d}-L^{e}-;
wherein
-L^{a}- is selected from: -(CH₂)ₒ-, -(CH₂-CH₂-O)ₒ-C₂H₄-, -(CH₂-CH₂-O)ₒ-CH₂; -L^{b}- represents -O-;
-L^{d}- is selected from -(CH₂)_{q}-, -(CF₂)_{q}-, -(CH₂-CH₂-O)_{q}-C₂H₄-, and -(CH₂-CH₂-O)_{q}CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-
and o, q, p1 and p2 are independently of each other an integer selected from 1, 2, 3, 4, 5, and 6.

Therefore, a saccharide of general formula (I), **(II), (II-a), (II-b), (II-c), (II-d), (III), (III-a), (III-b), (III-c), (III-d), (IV), (IV-a), (IV-b), (IV-c), (IV-d), (V), (V-a), (V-b), (V-c)** or **(V-d)** wherein
-L- is selected from: -L^{a}-, -L^{a}-L^{e}-, -L^{a}-L^{b}-L^{e}-, and -L^{a}-L^{d}-L^{e}-;
-L^{a}- is selected from: -(CH₂)ₒ-, -(CH₂-CH₂-O)ₒ-C₂H₄-, -(CH₂-CH₂-O)ₒ-CH₂; -L^{b}- represents -O-;
-L^{d}- is selected from: -(CH₂)_{q}-, -(CF₂)_{q}-, -(CH₂-CH₂-O)_{q}-C₂H₄-, and -(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- and -(CH₂)ₚ₁-O-(CH₂)ₚ₂-
and o, q, p1 and p2 are independently of each other an integer selected from 1, 2, 3, 4, 5, and 6
is especially preferred.

Even more preferred is a saccharide of general formula **(I), (II), (II-a), (II-b), (II-c), (II-d), (III), (III-a), (III-b), (III-c), (III-d), (IV), (IV-a), (IV-b), (IV-c), (IV-d), (V), (V-a), (V-b), (V-c)** or **(V-d)** wherein -L- represents -(CH₂)ₒ- and o is an integer selected from 2, 3, 4, 5 and 6.

In yet another preferred embodiment, the saccharide according to the present invention is elected from the group consisting of:
α-D-glucopyranosyl-(1→4)-α-D-galactopyranosyl-(1→4)-β-D-glucopyranosyluronic acid-(1→4)-β-D-glucopyranosyl-(1→1)-(2-amino)ethanol **(10),**
β-D-glucopyranosyluronic acid-(1→4)-β-D-glucopyranosyl-(1→4)-α-D-glucopyranosyl-(1→4)-α-D-galactopyranosyl-(1→1)-(2-amino)ethanol **(18),**
5-amino pentanyl β-D-glucopyranosyl uronic acid-(1→4)-β-D-glucopyranosyl-(1→4)-α-D-glucopyranosyl-(1→4)-α-D-galactopyranoside **(19),**
5-amino pentanyl α-D-galactopyranosyl-(1→4)-β-D-glucopyranosyluronic acid-(1→4)-β-D-glucopyranosyl-(1→4)-α-D-glucopyranoside **(20),**
5-amino pentanyl α-D-glucopyranosyl-(1→4)-α-D-galactopyranosyl-(1→4)-β-D-glucopyranosyluronic acid-(1→4)-β-D-glucopyranoside **(21),**
5-amino pentanyl β-D-glucopyranosyl-(1→4)-α-D-glucopyranosyl-(1→4)-α-D-galactopyranosyl-(1→4)-β-D-glucopyranosyl uronic acid **(22),**
α-D-galactopyranosyl-(1→4)-β-D-glucopyranosyluronate-(1→4)-β-D-glucoyranosyl-α-D-glucopyranosyl-(1→4)-α-D-galactopyranosyl-(1→1)-(2-amino)ethanol **(55),**
α-D-glucopyranosyl-(1→4)-α-D-galactopyranosyl-(1→4)-β-D-glucopyranosyluronate-(1→4)-β-D-glucoyranosyl-(1→4)-α-D-glucopyranosyl-(1→4)-α-D-galactopyranosyl-(1→1)-(2-amino)ethanol **(57).**

### Chemical synthesis

A saccharide of the general formula (I) can be synthesized *via* several synthetic routes.

For example, a saccharide of general formula (I) can be assembled starting from thioglycoside building blocks **BB2, BB3, BB4** and **BB5** and functionalized solid support **BB1** (Angew. Chem. Int. Ed. 2013, 52, 5858.) (see **Scheme 1)** by automated solid phase synthesis.
The synthetic process, which is summarized in **Scheme 1** involves:
- assembly of the desired oligosaccharide, which includes
   glycosylation with the appropriate thioglycoside **(BB2, BB3, BB4** or **BB5)** by activation with NIS/TfOH; followed by
   removal of the temporary protecting group Fmoc by treatment with Et₃N;
- cleavage from the solid support; and
- removal of the permanent protecting groups.

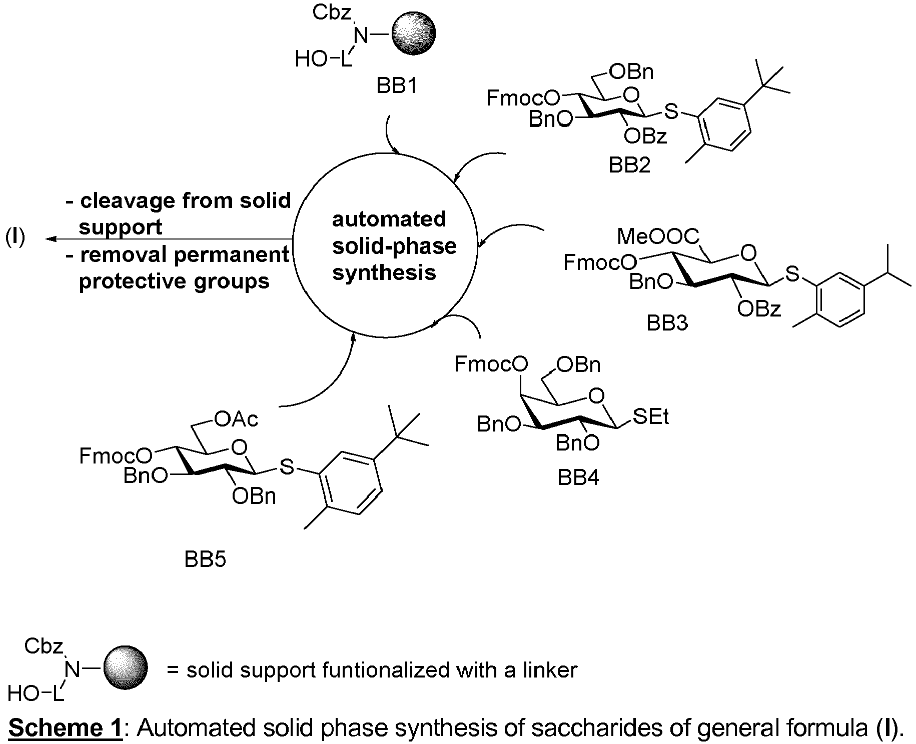

Alternatively, solution-phase synthesis of oligosaccharides can be used for accessing saccharides of general formula (I). To accelerate the synthetic process, disaccharidic building blocks such as **BB6** and **BB7** are preferably used as elongating units.

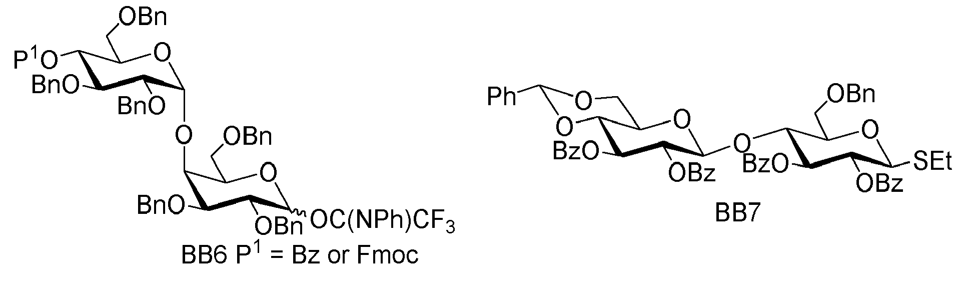

The skilled person will appreciate that for accessing higher saccharides of general formula (I), it is convenient to use higher elongating units, such as tetrasaccharide **BB8,** that can be easily obtained starting from disaccharides **BB6** and **BB7** according to **Scheme 2.** Thus, the benzylidene acetal on disaccharide **BB7** can be removed by treatment with EtSH, p-TsOH to provide an intermediate diol, which is further submitted to regioselective oxidation with BAIB and Tempo and subsequent esterification of the newly formed carboxylic acid to give secondary alcohol **BB9.** Alcohol **BB9** can be further subjected to glycosylation reaction with disaccharide **BB6** to provide tetrasaccharide **BB8** that can be directly used as elongating unit. Conveniently, the hydroxyl group at the 4^{th} position of the α-glucoside moiety can be protected with an orthogonal Fmoc protecting group that enables selective removal in anticipation of further glycosylation reactions. However, if the α-glucoside moiety constitutes the terminal monosaccharide at the non-reducing, protection of the hydroxyl group at the 4^{th} position as a benzoate ester will expedite the synthesis.

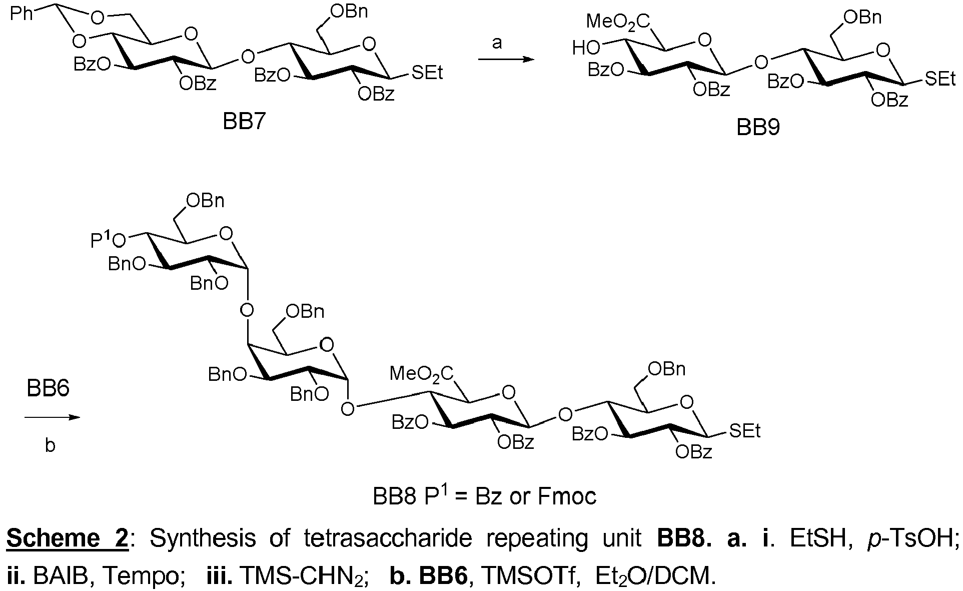

### Microarray

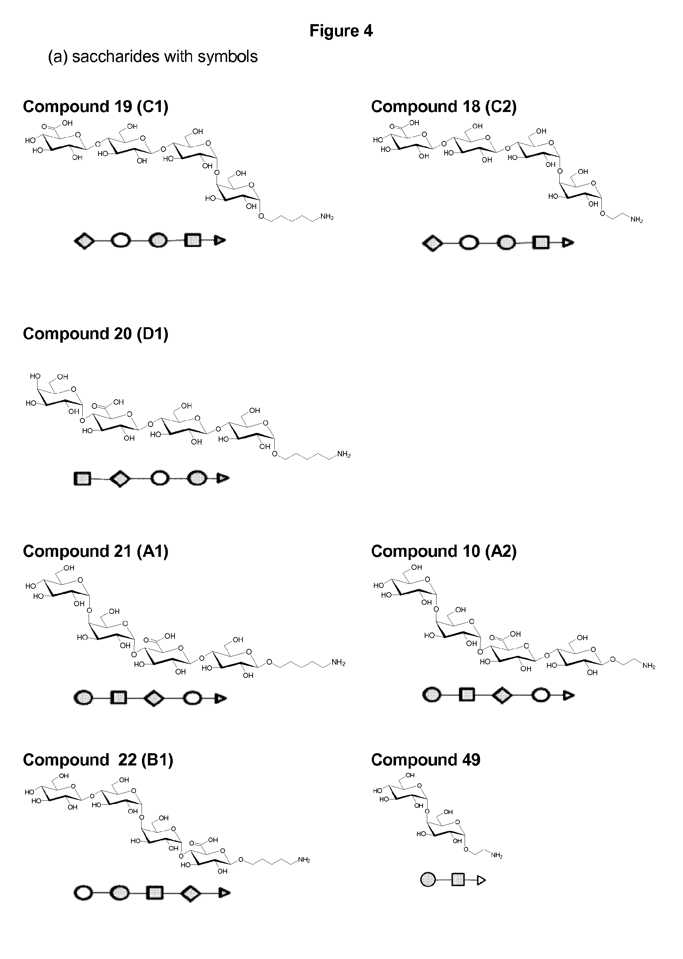

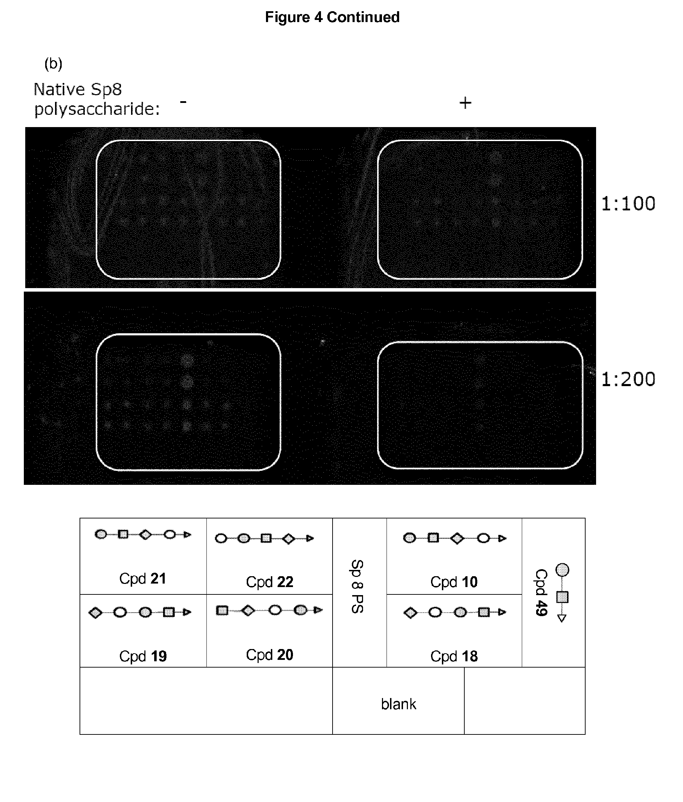

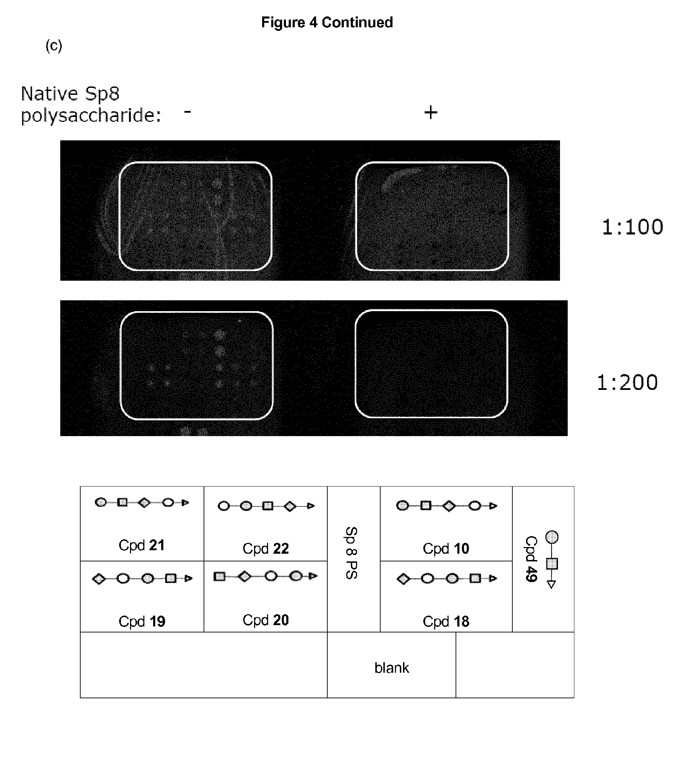

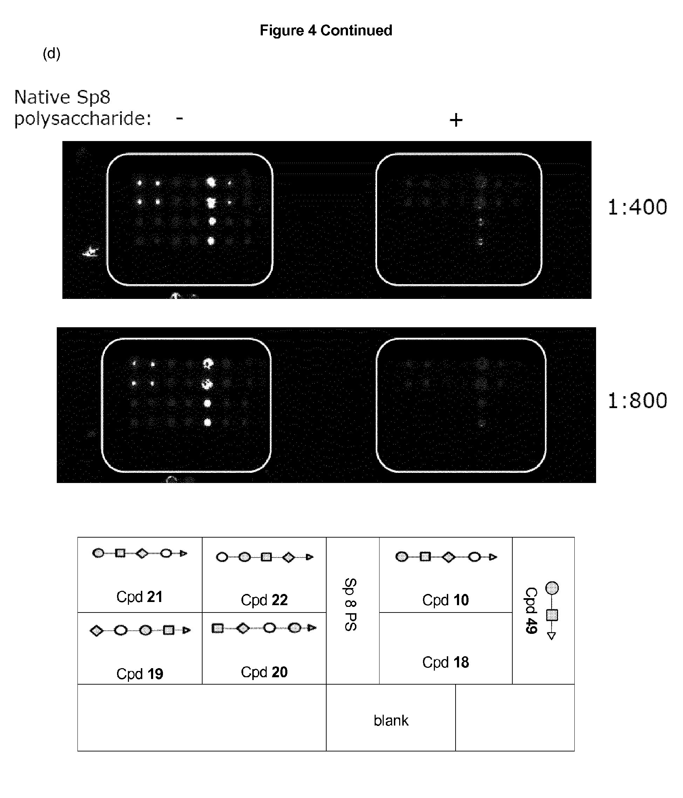

As shown in Figure 4, the inventive compounds of the formulae (I), **(II), (II-a)-(II-d), (III), (III-a)-(III-d), (IV), (IV-a)-(IV-d), (V), (V-a)-(V-d),** especially saccharides **10, 18, 19, 20, 21, 22, 55** and **57** share epitopes with the SP-8 native polysaccharide. The synthetic oligosaccharides were printed on *N*-hydroxysuccinimide-functionalized glass slides and incubated with either a rabbit antiserum against the native Sp8 bacterium (type 8 antiserum, SSI Diagnostica, **Figure 4d****)** or combined sera of humans vaccinated with Pneumovax23® ("007sp", National Institute for Biological Standards and Control, **Figure 4b****,** **c****).** While the rabbit antiserum revealed recognition of all printed tetrasaccharides (Figure **4d****),** the antiserum from human vaccinated with Pneumovax23® recognized only compounds **19, 20, 22 and 18** (see **Figures 4b** and **4c****).** Incubation with SP-8 native polysaccharide abrogated the (see **Figures 4b** and **4c****).** These data demonstrate the specificity and cross reactivity of tetrasaccharides **18, 19** and **20** towards binding to anti-SP8 CPS antibodies.

### Glycoconjugates

Another aspect of the present invention refers to compound of the general formula **(I')**

**V*-[Uₓ₊₃-Uₓ₊₂-Uₓ₊₁-Uₓ]ₙ-V-O-** (I')

covalently linked to an immunogenic carrier through the O atom, wherein V*, Uₓ₊₃, Uₓ₊₂, Uₓ₊₁, Uₓ, V, x and n have the meanings defined herein. In other words, another aspect of the present invention is directed to a saccharide of any of the general formulae **(I), (II), (II-a)-(II-d), (III), (III-a)-(III-d), (IV), (IV-a)-(IV-d), (V), (V-a)-(V-d)** conjugated with an immunogenic carrier through the nitrogen atom of the -O-L-NH₂ group. Thus, a compound of the general formula **(I')** covalently linked to an immunogenic carrier through the O atom is also defined as a conjugate obtained by reacting a saccharide of any of the general formulae (I), **(II), (II-a)-(II-d), (III), (III-a)-**(III-d), **(IV), (IV-a)-(IV-d), (V),** and **(V-a)-(V-d)** with an immunogenic carrier. Said conjugate proved to be efficient as a vaccine for immunization against diseases associated with *Streptococcus pneumoniae* serotype 3 bacteria.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, the saccharides of general formulae **(I), (II), (II-a)-(II-d), (III), (III-a)-(III-d), (IV), (IV-a)-(IV-d), (V)** and **(V-a)-(V-d)** are conjugated to an immunogenic carrier to provide conjugates presenting increased immunogenicity in comparison with the saccharide.

Said conjugate consists of at least one synthetic saccharide of the general formula (I) and an immunogenic carrier to which the at least one saccharide (I) is covalently bound.

Surprisingly it was found that immunization with a conjugate of general formula **(I')** results in the production of high titers of antibodies specific to the carbohydrate part of the saccharide of general formula **(I).** Said antibodies are cross-reacting with the natural SP-8 polysaccharides and present opsonophagocytosis and bactericidal activity, thus conferring protection against S. *pneumoniae* serotype 8 bacteria.

In this context the term "immunogenic carrier" is defined as a structure, which is conjugated to the saccharide to form a conjugate that presents an increased immunogenicity in comparison with the saccharide *per se.* Thus, the conjugation of the saccharides of the general formulae **(I), (II), (II-a)-(II-d), (III), (III-a)-(III-d), (IV), (IV-a)-(IV-d), (V)** and **(V-a)-(V-d)** to the immunogenic carrier has as effect the stimulation of the immune response against the saccharide of the general formulae **(I), (II), (II-a)-(II-d), (III), (III-a)-(III-d), (IV), (IV-a)-(IV-d), (V), (V-a)-(V-d)** without inducing an immune response against the said immunogenic carrier.

Preferred immunogenic carriers are carrier proteins or glycosphingolipids with immunomodulatory properties. For the person skilled in the art, a carrier protein is a protein selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid, a mutated tetanus toxoid, outer membrane protein (OMP), bovine serum albumin (BSA), keyhole limpet hemocyanine (KLH) or cholera toxoid (CT). The term "toxoid" as used herein refers to a bacterial toxin (usually an exotoxin), whose toxicity has been inactivated or suppressed either by chemical (formalin) or heat treatment, while other properties, typically immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group Y of the interconnecting molecule to provide a modified toxoid. Said functionality is known to the person skilled in the art and includes, but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue.

Activated esters include N-(γ-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarate (DSG), disuccinimidyl adipate (DSA), 2-pyridyldithiol-tetraoxatetradecane-N-hydroxysuccinimide (PEG-4-SPDP) (see **Figure 1****).** Preferred activated esters are disuccinimidyl adipate (DSA) and disuccinimidyl glutarate (DSG).

The cysteine residue on the carrier protein can be converted to the corresponding dehydroalanine that can be further reacted with a suitable interconnecting molecule to provide modified carrier protein having on their surface the functional group X of the interconnecting molecule.

It is especially preferred that the saccharides of general formula I are conjugated to the non-toxic mutated diphtheria toxin CRM₁₉₇ presenting as a functionality a primary amine functionality of a lysine residue.

CRM₁₉₇ like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as Prevnar.

Thus, in a preferred embodiment of the present invention the carrier protein presents on its surface primary amino functionalities of lysine residues that are able to react with the functional group Y of the interconnecting molecule to provide modified carrier protein having on their surface said functional group X of the interconnecting molecule, which is able to react with the terminal amino group of the linker of the saccharides of general formula (I).

Said functional group X of the interconnecting molecules is selected of the group comprising or consisting of maleimide; α-iodoacetyl; α-bromoacetyl; and N-hydroxysuccinimide ester (NHS), aldehyde, imidoester, carboxylic acid, alkyl sulfonate, sulfonyl chloride, epoxide, anhydride, carbonate (see **Figure 2****).**

Preferably, the saccharide of general formula **I** is conjugated to the non-toxic mutated diphtheria toxin CRM₁₉₇, which is modified by maleimide. In yet another preferred embodiment, the saccharide of general formula **I** is conjugated to the non-toxic mutated diphtheria toxin CRM₁₉₇, which is modified by α-bromoacetamide. In the most preferred embodiment, the saccharide of general formula **I** is conjugated to the non-toxic mutated diphtheria toxin CRM₁₉₇, which is modified by *N-*hydroxysuccinimide adipate.

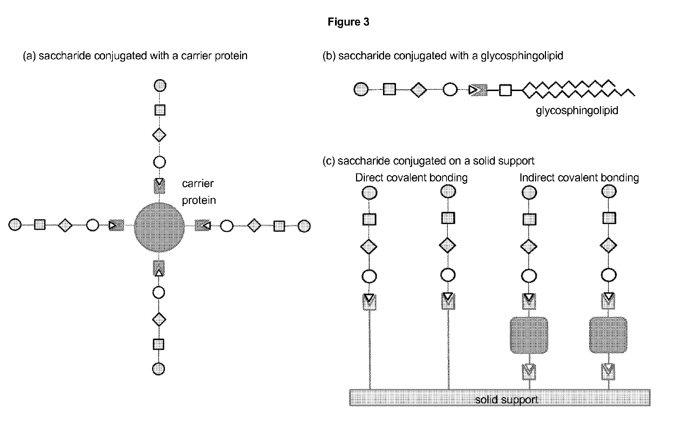

Preferably, di(*N*-succinimidyl) adipate is firstly attached to a synthetic saccharide **(I)** having a primary amino group. Activated saccharide (I) is subsequently condensed with a carrier protein such as CRM₁₉₇, to afford the conjugate. Also, the use of the related disuccinimidyl glutarate can be applied **(****Figure 3a****).**

In another embodiment, said immunogenic carrier is preferably a glycosphingolipid with immunomodulatory properties, and more preferably (2S,3S,4R)-1-(α-D-galactopyranosyl)-2-hexacosanoylaminooctadecane-3,4-diol. The term glycosphingolipid with immunomodulatory properties, as used herein, refers to a suitable glycosphingolipid capable of stimulating the immune system's response to a target antigen, but which does not in itself confer immunity as defined above.

Glycosphingolipids as used herein are compounds containing a carbohydrate moiety α-linked to a sphingolipid. Preferably, the carbohydrate moiety is a hexopyranose and most preferably is α-D-galactopyranose. For the person skilled in the art, sphingolipids are a class of lipids containing a C18 amino alcohol connected *via* an amide bond to a fatty acid. The C18 amino alcohol is preferably mono-, di- or polysubstituted with hydroxyl groups. Especially preferred, the C18 amino alcohol is phytosphingosine. The fatty acid is preferably a monocarboxylic acid having a saturated alkyl chain of a number of carbons ranging from 16 to 28 and more preferably from 18 to 26. Glycosphingolipids with immunomodulatory properties include, but they are not restricted to (2*S*,3*S*,4*R*)-1-(α-D-galactopyranosyl)-2-hexacosanoylaminooctadecane-3,4-diol, which can stimulate natural killer (NK) activity and cytokine production by natural killer T (NKT) cells and exhibits potent antitumor activity *in vivo* (Proc. Natl Acad. Sci. USA, 1998, 95, 5690).

The conjugates of the saccharides of general formula **I** with a glycosphingolipid with immunomodulatory properties have the advantage of being heat stable. To be suitable for conjugation, on the glycosphingolipid with immunomodulatory properties a functionality is introduced. Said functionality is prone to react directly with the terminal amino group of the linker of the saccharides of general formula **I** to provide conjugates of the saccharides of general formula I, or with the functional group Y of the interconnecting molecule to provide the modified glycosphingolipid with immunomodulatory properties.

Preferably, said functionality is introduced at the C6 of the carbohydrate moiety of the glycosphingolipid with immunomodulatory properties. Thus, the glycosphingolipid with immunomodulatory properties is functionalized with a functionality, which is prone of reacting with the terminal amino group of the saccharides or with the functional group Y of the interconnecting molecule. A functionality prone to react with an amino group includes, but it is not restricted to activated ester, isocyanate group, aldehyde, epoxide, imidoester, carboxylic acid, alkyl sulfonate and sulfonyl chloride. A functionality prone to react with the functional group Y of the interconnecting molecule so that to provide the modified glycosphingolipid with immunomodulatory properties presenting the functional group X of the interconnecting molecule includes, but it is not restricted to amine, alcohol, thiol, activated ester, isocyanate group, aldehyde, epoxide, vinyl, imidoester, carboxylic acid, alkyl sulfonate, sulfonyl chloride, vinyl group, alkynyl group and azido group.

Preferably, the functionality introduced at the C6 of the carbohydrate moiety of the glycosphingolipid with immunomodulatory properties is selected from the group comprising or containing an amine, a thiol, an alcohol, a carboxylic acid, a vinyl, maleimide, α-iodoacetyl, α-bromoacetyl, *N*-hydroxysuccinimide ester (NHS), 2-pyridyldithiols.

Said functional group X of the interconnecting molecules is selected of the group comprising or consisting of maleimide, α-iodoacetyl, α-bromoacetyl, *N-*hydroxysuccinimide ester (NHS), aldehyde, carboxylic acid, epoxyde, alkyl sulfonate, sulfonyl chloride, anhydride, carbonate.

Preferably, di(*N*-succinimidyl) adipate or bis(4-nitrophenyl) adipate is first reacted with a synthetic saccharide (I) having a primary amino group. Activated saccharide (I) is subsequently condensed with a glycosphingolipid which is modified at C-6 position by a interconnecting molecule having activated ester in order to afford the conjugate. **(****Figure 3b****).**

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier or on the solid support.

It was found that the saccharide of general formula **(I')** covalently linked to an immunogenic carrier through the O atom or in other words the conjugate obtained by reacting a saccharide of general formula (I), **(II), (II-a)-(II-d), (III), (III-a)-(III-d), (IV), (IV-a)-(IV-d), (V)** or **(V-a)-(V-d)** with an immunogenic carrier elicits an immune response in a human and/or animal host, and therefore is useful in the prevention and/or treatment of a disease associated with bacteria containing in their capsular polysaccharide one of the following saccharide fragments:
α-D-Glc*p*-(1→4)-α-D-Gal*p*-(1→4)-β-D-GlcA*p*-(1→4)-β-D-Glc*p*,
β-D-Glc*p*-(1→4)-α-D-Glc*p*-(1→4)-α-D-Gal*p*-(1→4)-β-D-GlcA*p*,
β-D-GlcA*p*-(1→4)-β-D-Glc*p*-(1→4)-α-D-Glc*p*-(1→4)-α-D-Gal*p*,
α-D-Gal*p*-(1→4)-β-D-GlcA*p*-(1→4)-β-D-Glc*p*-(1→4)-α-D-Glc*p*.

Preferably, the bacterium containing in the capsular polysaccharide one of the above mentioned saccharide fragments is *Streptococcus pneumoniae* serotype 8. Diseases associated with *Streptococcus pneumoniae* serotype 8 include pneumonia, meningitis, otitis media, bacteremia and acute exacerbation of chronic bronchitis, sinusitis, arthritis and conjunctivitis.

### Pharmaceutical composition

One aspect of the present invention relates to a pharmaceutical composition, especially a vaccine containing at least one synthetic saccharide of general formula (I) and/or a pharmaceutical acceptable salt thereof and/or a compound of general formula (I') covalently linked to an immunogenic carrier through the O atom together with at least one pharmaceutical acceptable adjuvant, cryoprotectant, lyoprotectant, excipient and/or diluent.

The vaccine may be prepared in the form of a suspension or may be lyophilized. The suspension form may be stored frozen. In the lyophilized form, it is preferable to add one or more stabilizers. Vaccination can be performed at any age. The vaccine many be administered subcutaneously, by spray, by injection, orally, intraocularly, intratracheally or nasally. The amount of vaccine of the invention to be administered a human or animal and the regime of administration can be determined in accordance with standard techniques well known to those of ordinary skill in the pharmaceutical and veterinary arts taking into consideration such factors as the particular antigen, the adjuvant (if present), the age, sex, weight, species and condition of the particular animal or human host, and the route of administration.

Another aspect of the present invention is directed to a method of inducing immune response against *Streptococcus pneumoniae* in a human and/or animal host, said method comprising administering of the saccharide of general formula (I) and/or salt thereof and/or a compound of general formula **(I')** covalently linked to an immunogenic carrier through the oxygen atom and/or a mixture thereof or pharmaceutical composition thereof to said human and/or animal host. A method of treating or preventing diseases caused by *Streptococcus pneumoniae* in a human and/or animal host according to the present invention comprises administering of at least one saccharide of general formula (I) and/or salt thereof and/or compound of general formula **(I')** covalently linked to an immunogenic carrier through the oxygen atom and/or a mixture thereof or pharmaceutical composition thereof to said human and/or animal host.

Intravenous and parenteral administration is preferred. Such compositions may be in admixture with a suitable carrier, diluent, or excipient such as sterile water, physiological saline, glucose or the like. For instance, lyophilized saccharides of the invention can ultimately be reconstituted with a liquid component to give material suitable for administration to a said human and/or animal host. The reconstitution will typically take place at the point of use. Thus, a saccharide of the invention and an oil-in-water emulsion adjuvant or a buffer solution of an adjuvant may be kept separately in a packaged or distributed vaccine kit, ready for final formulation at the time of use. In a kit containing two containers, one will include liquid for reconstitution and the second container includes lyophilized material. For stability reasons, the lyophilized component of the invention may include a stabilizer such as lactose, sucrose and/or mannitol, as well as mixtures thereof. Using a sucrose/mannitol mixture can speed up the drying process. A lyophilized component may also include sodium chloride. Soluble components in the lyophilized material will be retained in the composition after reconstitution, and so final liquid vaccines may thus contain lactose and/or sucrose.

Formulation of the vaccines of the present invention can be accomplished using methods known by the art. Obviously, the choice of suitable carriers and other additives will depend on the exact route of administration and the nature of the particular dosage form.

The vaccine compositions of the present invention may contain one or more adjuvants. The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples of immunological adjuvants include, but are not restricted to oil emulsions (e.g. Freund's adjuvant), saponins, aluminium or calcium salts (e.g. alum), non-ionic block polymer surfactants, and many others.

The vaccines of the present invention can contain auxiliary substances such as wetting or emulsifying agents, pH buffering agents, gelling or viscosity enhancing additives, preservatives, flavoring agents, colors, and the like, depending upon the route of administration and the preparation desired. Compositions of the invention may include an antimicrobial, particularly when packaged in multiple dose formats. Antimicrobials such as thiomersal and 2-phenoxyethanol are commonly found in vaccines, but it is preferred to use either a mercury-free preservative or no preservative at all.

Further, the inventive vaccines may include a temperature protective agent. Examples include glycerin, propylene glycol, and/or polyethylene glycol (PEG).

The pharmaceutical compositions of the present invention are conveniently provided as liquid preparations, e.g., isotonic aqueous solutions, suspensions, emulsions or viscous compositions that may be buffered to a selected pH. Pharmaceutically acceptable carriers for liquid formulations may be aqueous or non-aqueous solutions, suspensions, emulsions or oils. Examples of non-aqueous solvents are propylene glycol, polyethylene glycol, and injectable organic esters such as ethyl oleate. Aqueous carriers include water, alcoholic solutions, emulsions or suspensions, including saline and buffered media.
The pH of a composition after reconstitution is preferably between 6 and 8 and more preferably between 6.5 and 7.5 (e.g. about 7). Compositions of the invention may be maintained by the use of a buffer e.g. a Tris buffer, acetate, glutamate, lactate, maleate, tartrate, phosphate, citrate, carbonate, glycinate, histidine, glycine, succinate and triethanolamine buffer. Thus compositions of the invention will preferably include a buffer. The isotonic agent may be an ionic isotonic agent such as a salt or a non-ionic isotonic agent such as a carbohydrate. Examples of ionic isotonic agents include but are not limited to NaCl, CaCl₂, KCI and MgCl₂. Examples of non-ionic isotonic agents include but are not limited to sorbitol and glycerol.

In a preferred embodiment of the invention, the vaccine composition is formulated as a sterile liquid, pyrogene-free, phosphate-buffered physiological saline, with or without a preservative.

A pharmaceutically acceptable preservative can be employed to increase the shelf life of the compositions. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

The pharmaceutical compositions of the invention can be formulated as single dose vials, multidose vials or as pre-filled syringes.

Further preferred, the pharmaceutical composition is formulated in the form of a lyophilisate or liquid buffer solution.

The vaccine or pharmaceutical composition of the present invention is prepared in a conventional solid or liquid carrier or diluents and a conventional pharmaceutically-made adjuvant at suitable dosage level in a known way. The preferred preparations and formulations are in administrable, form which is suitable for oral application. These administrable forms, for example, include pills, tablets, film tablets, coated tablets, capsules, powders and deposits. Forms other than oral administratable forms are also possible. The inventive vaccine or pharmaceutical composition may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

The vaccine or pharmaceutical composition of the present invention, containing at least one synthetic saccharide of any of general formulae **(I), (II), (II-1**) - **(II-4), (III), (III-1) - (III-4), (IV), (IV-1) - (IV-4), (V), (V-1) - (V-4),** preferably the saccharides 10, **18, 19, 20, 21, 22, 55** and **57** or pharmaceutically acceptable salt thereof, or a compound of general formula (I') covalently linked to an immunogenic carrier thorough the oxygen atom as an active ingredient will typically be administered in admixture with suitable carrier materials suitably selected with respect to the intended form of administration, i.e. oral tablets, capsules (either solid-filled, semisolid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent of the tetrasaccharide.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavouring agents and preservatives may also be included where appropriate. Some of the terms noted above, namely disintegrants, diluents, lubricants, binders and the like, are discussed in more detail below.

Additionally, the vaccine or pharmaceutical composition of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive vaccine or pharmaceutical composition containing at least one synthetic saccharide of any of general formulae (I), **(II), (II-1**) - **(II-4), (III), (III-1**) - **(III-4), (IV), (IV-1) - (IV-4), (V), (V-1) - (V-4),** preferably the saccharides **10, 18, 19, 20, 21, 22, 55** and **57** or pharmaceutically acceptable salt thereof, or a compound of general formula **(I')** covalently linked to an immunogenic carrier through the oxygen atom may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or moulded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semisolid matrix.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1 %.

Techniques for the formulation and administration of the vaccine of the present invention may be found in "Remington's Pharmaceutical Sciences" Mack Publishing Co., Easton PA. A suitable vaccine composition comprising at least one saccharide of any one of the general formulae **(I), (II), (II-1)** - **(II-4), (III), (III-1) - (III-4), (IV), (IV-1)** - **(IV-4), (V), (V-1) - (V-4),** preferably the saccharides **10, 18, 19, 20, 21,** and **22,** or pharmaceutically acceptable salt thereof, or a compound of general formula **(I')** covalently linked to an immunogenic carrier thorough the oxygen atom, preferably the may be a solution of such saccharide(s) in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of one conjugate according to the present invention or of one saccharide of general formula (I) refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

### Antibody and immunological assays

The present invention refers also to an antibody against at least one synthetic saccharide of general formulae (I). The antibody is produced by the monoclonal hybridoma. The antibody is useful for diagnostics, prophylaxis, and treatment of pneumonia, meningitis, otitis media, bacteremia and acute exacerbation of chronic bronchitis, sinusitis, arthritis and conjunctivitis. Another embodiment of the present invention concerns the use of the antibody for manufacture of medicaments or devices for diagnosis, prophylaxis, and treatment of pneumonia, meningitis, otitis media, bacteremia and acute exacerbation of chronic bronchitis, sinusitis, arthritis and conjunctivitis caused by S. *pneumoniae,* and preferably by *S*. *pneumoniae serotype 8.*

The term "antibody" as used herein encompasses polyclonal and monoclonal antibody preparations, as well as preparations including hybrid antibodies, F(ab')₂ fragments, F(ab) molecules, single domain antibodies and functional fragments thereof which exhibit immunological binding properties of the parent antibody molecule.

The antibody according to the invention may be polyclonal or monoclonal.

The saccharide of any one of general formulae **(I), (II), (II-1**) - **(II-4), (III), (III-1**) - **(III-4), (IV), (IV-1) - (IV-4),** (**V**), **(V-1) - (V-4),** preferably the saccharides **10, 18, 19, 20, 21, 22, 55** and **57** or a compound of general formula **(I')** covalently linked to an immunogenic carrier thorough the oxygen atom or the antibody thereof can be used for preparing a pharmaceutical composition, especially a vaccine, for the treatment or prevention of diseases caused by *S*. *pneumoniae,* preferably *S*. *pneumoniae* serotype 8. The saccharide of the present invention or the antibody thereof can be used for the treatment or prevention of a disease caused by *S*. *pneumoniae,* preferably *S*. *pneumoniae* serotype 8.

Further the present invention refers to the use of at least one saccharide of general formula **(I), (II), (II-1)** - **(II-4), (III), (III-1**) - **(III-4), (IV), (IV-1) - (IV-4), (V), (V-1) - (V-4)** according to the invention or at least one antibody against at least one saccharide of the present invention in immunological assays for diagnosis of pneumonia, meningitis, otitis media, bacteremia and acute exacerbation of chronic bronchitis, sinusitis, arthritis and conjunctivitis caused by *S*. *pneumoniae,* preferably *S*. *pneumoniae* serotype 8.

Such assays comprise, for instance, microarray and ELISA useful for diagnosis of diseases caused by *S*. *pneumoniae,* preferably *S*. *pneumoniae* serotype 8. Therefore, another aspect of the present invention refers to the use of any one of saccharides of formulae **(I), (II), (II-1**) - **(II-4), (III), (III-1**) - **(III-4), (IV), (IV-1) - (IV-4), (V),** and **(V-1) - (V-4)** for diagnosis of diseases caused by S. *pneumoniae,* preferably *S*. *pneumoniae* serotype 8.

Any one of saccharides of general formulae **(I), (II), (II-1) - (II-4), (III), (III-1**) - **(III-4), (IV), (IV-1) - (IV-4), (V),** and **(V-1) - (V-4)** or a mixture of such saccharides could be immobilized on a microarray surface or any other surface and used for an *in vitro* method of detecting S. *pneumoniae* serotype 8. A method of identifying *S*. *pneumoniae serotype* 8 comprises the use of at least one saccharide of the present invention. Furthermore, the synthetic saccharide of general formulae **(I), (II), (II-1**)-**(II-4), (III), (III-1**) - **(III-4), (IV), (IV-1) - (IV-4), (V),** and **(V-1) - (V-4)** or a mixture of such saccharides can be used as an analytical standard for immunoassays.

Thus, the saccharide of general formula (I), **(II), (II-1**) - **(II-4), (III), (III-1**) - **(III-4), (IV)**, **(IV-1) - (IV-4), (V),** or **(V-1) - (V-4)** can be used as a marker in immunological assays for detection of antibodies against bacteria containing in their capsular polysaccharide one of the following saccharide fragments:
α-D-Glcp-(1→4)-α-D-Gal*p*-(1→4)-β-D-GlcA*p*-(1→4)-β-D-Glc*p*,
β-D-Glc*p*-(1→4)-α-D-Glc*p*-(1→4)-α-D-Gal*p*-(1→4)-β-D-GlcA*p*,
β-D-GlcA*p*-(1→4)-β-D-Glc*p*-(1→4)-α-D-Glc*p*-(1→4)-α-D-Gal*p*,
α-D-Gal*p*-(1→4)-β-D-GlcA*p*-(1→4)-β-D-Glc*p*-(1→4)-α-D-Glc*p*.

Thus, another aspect of the present invention is related to a solid support comprising at least one saccharide of general formula (I).

This solid support is preferable a part of a diagnostic device. The solid support and the diagnostic device are used for diagnosis of pneumonia, meningitis, otitis media, bacteremia and acute exacerbation of chronic bronchitis, sinusitis, arthritis and conjunctivitis caused by *S*. *pneumoniae,* preferably *S*. *pneumoniae* serotype 8, wherein the at least one saccharide of general formula (I) is immobilized on said solid support by preferably covalent bonding.

Preferably, the solid support is selected from the group comprising a glass slide, glass plate, a microtitre plate, microspheres, or beads.

The saccharide of general formula (I) is preferably covalently bound to the solid support using an interconnecting molecule.

Moreover, the present invention shows that the saccharide according to formula (I) can be used in immunological assays detection of for pneumonia, meningitis, otitis media, bacteremia and acute exacerbation of chronic bronchitis, sinusitis, arthritis and conjunctivitis caused by *S*. *pneumoniae,* preferably *S*. *pneumoniae* serotype 8. Such assays comprise, for instance, microarray and ELISA useful for diagnosis of pneumonia, meningitis, otitis media, bacteremia and acute exacerbation of chronic bronchitis, sinusitis, arthritis and conjunctivitis caused by *S*. *pneumoniae,* preferably *S*. *pneumoniae* serotype 8.

Therefore another aspect of the present invention refers to the use of a saccharide of formula (I) for diagnosis of pneumonia, meningitis, otitis media, bacteremia and acute exacerbation of chronic bronchitis, sinusitis, arthritis and conjunctivitis caused by *S*. *pneumoniae,* preferably *S*. *pneumoniae* serotype 8.

There are different possibilities for the choice of an assay system in which a saccharide of formula (I) is used for diagnosis of pneumonia, meningitis, otitis media, bacteremia and acute exacerbation of chronic bronchitis, sinusitis, arthritis and conjunctivitis caused by *S*. *pneumoniae,* preferably *S*. *pneumoniae* serotype 8. An assay conducted for diagnostic purposes according to the invention may be an immune assay like a solid-phase enzyme immunoassay (EIA), an enzyme linked immunosorbent assay (ELISA), especially an "indirect" ELISA or a radioimmune assay (RIA).

Preferably, the saccharide of general formula **(I)** is covalently linked on the solid support through an interconnecting molecule. Thus, the saccharide of general formula **(I)** can be covalently linked on the solid support directly or indirectly through the nitrogen atom of the -O-L-NH₂ group **(****Figure 3, (C)****).**

The solid support is preferably selected from the group comprising or consisting of: a glass slide, a microtitre plate, test tubes, microspheres, nanoparticle or beads.

It is particularly preferred that the solid support is a glass slide or a microtitre plate. A microtitre plate or microplate or microwell plate is a flat plate with multiple "wells" used as small test tubes. Typically, a microtitre plate having 6, 24, 96, 384 or even 1536 sample wells can be used. Microplates are produced from many different materials, like polycarbonate for microtitre plate used for PCR. The most common is polystyrene as used for most optical detection microplates. It can be colored white by the addition of titanium dioxide for optical absorbance or luminescence detection or black by the addition of carbon for fluorescent biological assays.

### Description of the figures

- **Figure 1:**: Commercially available interconnecting molecules according to the present invention.
- **Figure 2:**: Examples of functional group X of the interconnecting molecule according to the present invention.
- **Figure 3:**: (a) saccharide conjugated with a carrier protein. (b) saccharide conjugated with a glycosphingolipid. (c) saccharide conjugated on a solid support.
- **Figure 4:**: (a) Saccharide symbols. (b) Glycan array analysis using commercially available serum 007sp: detection was performed using a fluorescently labelled anti-human IgM secondary antibody (Alexa Fluor 488 goat anti-human IgM, Invitrogen A21215) (c) Glycan array analysis using commercially available serum 007sp: detection was performed using a fluorescently labelled anti-human IgG secondary antibody (Alexa Fluor 647 goat anti-human IgG, Invitrogen A21445); (d) Glycan array analysis using a SP-8 specific rabbit typing serum: detection was performed using a fluorescently labelled anti-rabbit IgG secondary antibody (goat anti-rabbit IgG-FITC, abcam ab6717).

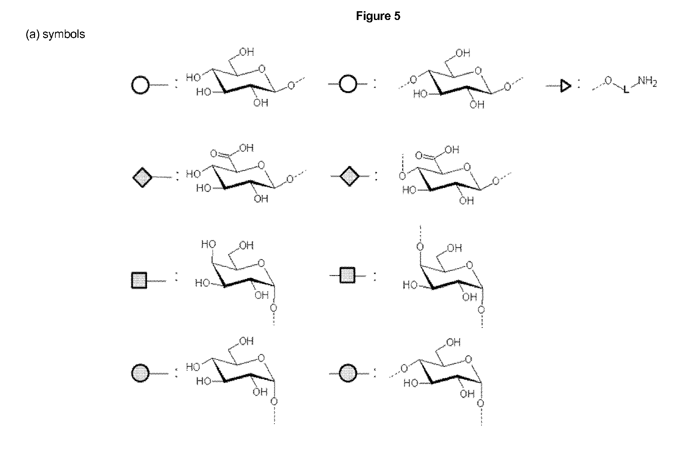

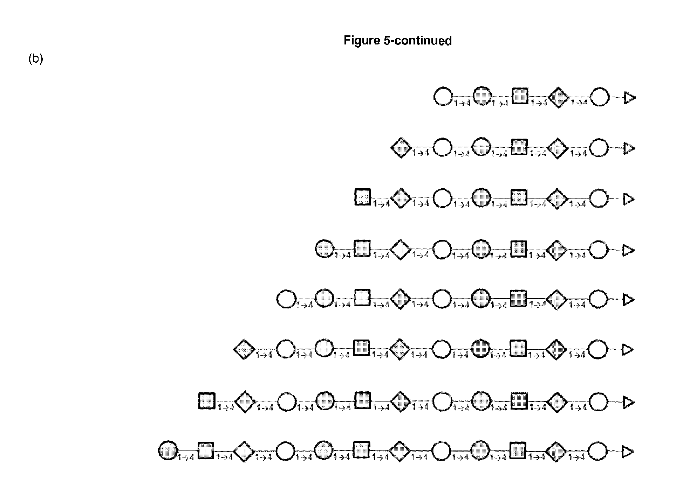

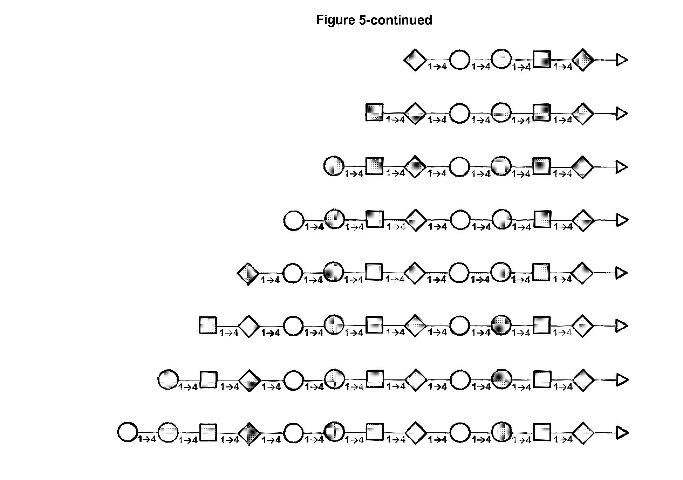

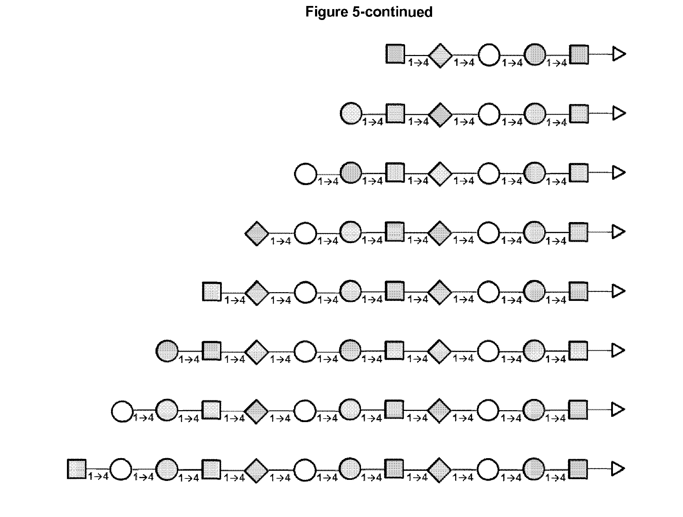

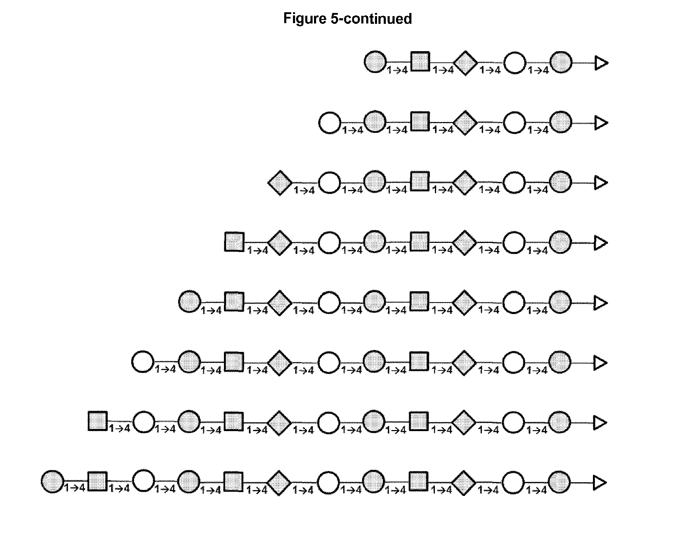

- **Figure 5:**: Preferred saccharide of general formula (I) according to the present invention. (a) saccharide symbols; (b) preferred saccharides of general formula (I) according to the present invention.
- **Figure 6:**: Immune response of mouse 1160: (a) microarray printing pattern; (b) immune response of mouse 1160 (timeframe day 0 to day 35): detection was performed using a fluorescently labelled anti-mouse IgG secondary antibody (rabbit anti-mouse IgG-FITC, F9137, Sigma).
- **Figure 7:**: Generation of anti SP-8 monoclonal antibodies: detection was performed using a fluorescently labelled anti-mouse secondary antibody (Alexa 635 goat anti-mouse IgG, Invitrogen A31574).
- **Figure 8:**: Surface plasmon resonance: monoclonal antibody mAb 1H8 specifically recognizes both synthetic saccharide **18** and the native Sp8 polysaccharide.
- **Figure 9:**: Immunofluorescence staining results: immunization with conjugate **59** induces an immune response that recognizes the native SP-8 polysaccharide and ST8 bacteria. Bacteria were labeled with FITC and incubated with the indicated primary antibodies. Antibody binding was visualized after incubation with Alexa 635-labeled goat anti-mouse IgG secondary antibody (A31574, Invitrogen). (A) *S*. *pneumoniae* ST8, mAb 1 H8; (B) *S*. *pneumoniae* ST8, mAb isotype control (anti *Y. pestis* LPS core); (C) S. pneumoniae ST1; mAb 1H8.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### 1. Chemical Synthetic Experiments

### General information for chemical synthesis.

All commercially available starting materials and reagents were used as received unless otherwise noted. All the reactions were performed under argon atmosphere. Solvents were dried. High-resolution mass spectra (HRMS) were recorded with an Agilent 6210 ESI-TOF mass spectrometer at the Freie Universität Berlin.

### Abbreviations

In the following schemes occurring abbreviations mean Ac (acetyl), BAIB ([bis(acetoxy)iodo]benzene), Bn (benzyl), t-Bu (tert-butyl), Bz (benzoyl), Cbz (carboxybenzyl), DCM (dichloromethane), DDQ (2,3-dichloro-5,6-dicyano-1,4-benzoquinone), Im (imidazole), NAP (2-naphthylmethyl), NIS (*N*-iodosuccinimide), Py/Pyr (pyridine), TEMPO ((2,2,6,6-tetramethyl-1-piperdinyl)oxy), Tf (trifluoromethanesulfonyl), THF (tetrahydrofurane), TMS(trimethylsilyl), TMSOTf (trimethylsilyl trifluoromethane sulfonate), p-Ts (para-tolylsulfonyl).

### Synthesis of saccharide by [2+2] glycosylation approach

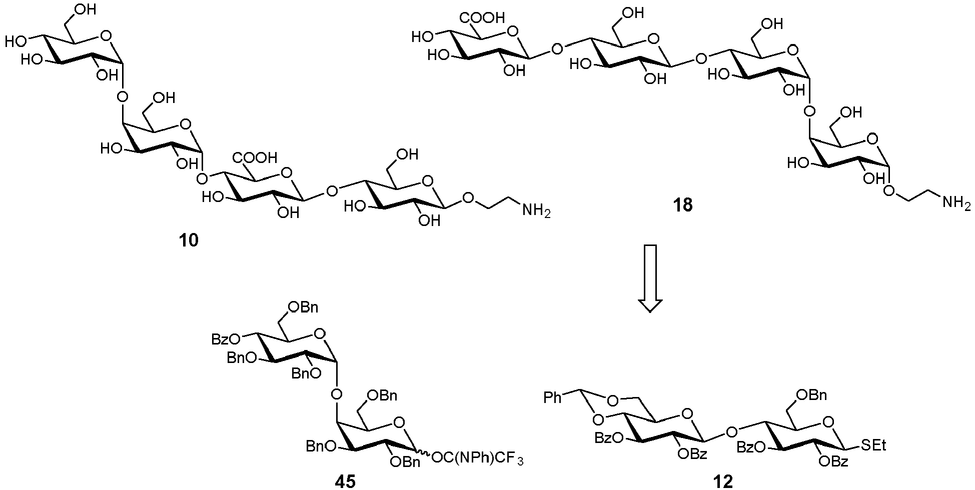

### Example 1-1: Synthesis of compound 3

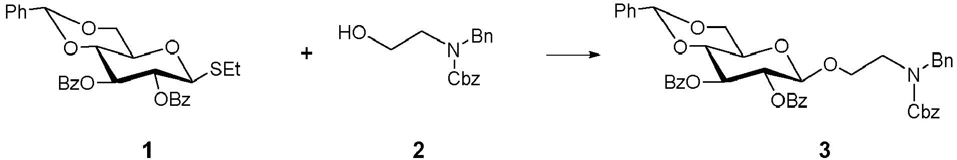

Thioglycoside donor substrate **1** (6.0 g, 11.53 mmol) and acceptor with C-2 linker **2** (dried azeotropically using toluene in rotary evaporator, 3.93 g, 13.83 mmol) were taken in dry DCM (100 mL) and added 5 g of MW dried 4 A MS to it and stirred at rt for 15 min and then cooled to -10 °C. NIS (3.83 g, 17.29 mmol) and TfOH (0.15 mL, 1.73 mmol) were then added to RM (reaction mixture) and stirred at -10 °C to -5 °C for 1 hr. Reaction completion was monitored by TLC. RM was then quenched with 10% aq. Na₂S₂O₃ solution (50 mL) and then extracted with EtOAc (25 ml X 3). Combined organic layer was then washed with brine (10 ml), dried over anhydrous Na₂SO₄, filtered and concentrated in vacuum to get pale yellow oily compound. Crude product was purified on silica gel column chromatography using 20-30% EtOAc in hexanes to get spot which on evaporation yielded desired product 3 as pale yellow colored transparent gummy liquid (7.60 g, 89%).
¹H NMR (400 MHz, CDCl₃) δ = 7.97 (dd, *J* = 8.4, 1.2 Hz, 4H), 7.59 - 6.90 (m, 21 H), 5.91 - 5.71 (m, 1 H), 5.62 - 5.41 (m, 2H), 5.22 - 4.95 (m, 2H), 4.80 (d, *J* = 7.7 Hz, 0.5H), 4.67 (d, *J* = 7.7 Hz, 0.5H), 4.56 - 4.22 (m, 3H), 4.10 - 3.52 (m, 5H), 3.50 - 3.33 (m, 2H). ¹³C NMR (101 MHz, CDCl₃) δ = 165.7, 165.4, 156.35, 156.2, 137.9, 136.9, 133.4, 133.2, 129.9, 129.5, 129.3, 129.1, 128.7, 128.5, 128.4, 128.3, 128.1, 127.8, 127.4, 127.2, 126.2, 101.9, 101.6, 78.9, 72.6, 72.1, 69.1, 68.7, 67.4, 67.2, 66.7, 51.7, 46.9, 45.8.

### Example 1-2: Synthesis of compound 4

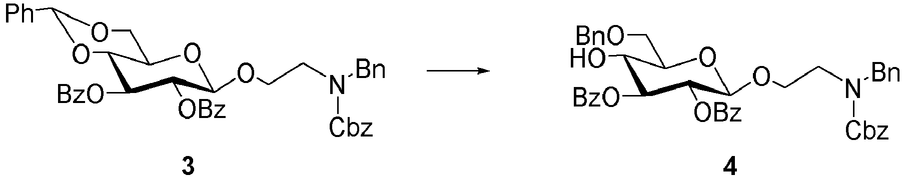

Substrate **3** (7.50 g, 10.08 mmol) was taken in DCM (75 mL) under argon with activated 3A MS for 10 min before cooling to 0 °C. Added triethylsilane (12.88 mL, 81.0 mmol) followed by TFA (4.66 mL, 60.5 mmol) drop wise and stirred the RM at rt for 16 h before quenching with water (100 mL). Extracted the aqueous with DCM (30 mL X 3), combined organics were washed thoroughly with water (20 mL X 3), brine (20 mL), dried over anhyd. Na₂SO₄, filtered, evaporated in vacuum to get colorless gummy solid. Crude product was purified by silica column chromatography using 30%-100% EtOAc in hexanes to get product **4** and evaporated in vacuum to get colorless oil (6.1 g, 81%).
¹H NMR (400 MHz, CDCl₃) δ = 8.04 - 7.84 (m, 4H), 7.60 - 6.87 (m, 21 H), 5.55 - 5.36 (m, 2H), 5.22 - 4.90 (m, 2H), 4.77 - 4.53 (m, 3H), 4.51 - 4.30 (m, 2H), 4.06 - 3.93 (m, 2H), 3.87 - 3.53 (m, 4H), 3.46 - 3.20 (m, 3H). ¹³C NMR (101 MHz, CDCl₃) δ = 167.3, 165.5, 138.0, 137.7, 133.6, 130.1, 129.9, 128.6, 128.5, 128.1, 127.9, 127.8, 127.4, 101.3, 101.2, 76.7, 74.7, 73.9, 71.6, 71.5, 71.2, 70.0, 69.0, 67.4, 67.2, 51.7, 46.8,45.8.

### Example 1-3: Synthesis of compound 5

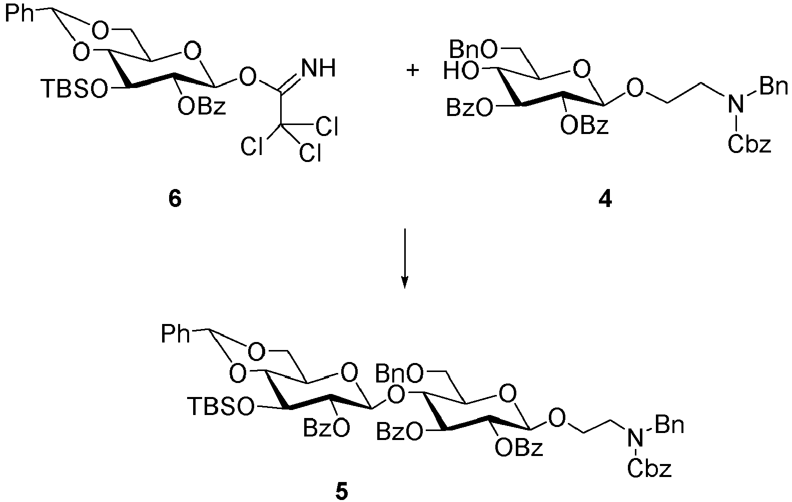

Acceptor **4** (2.0 g, 2.68 mmol) was taken in DCM (30 mL) with activated 4A AWMS and stirred at rt for 30 min before cooling to 0 °C. TMSOTf (0.49 µL, 0.27 mmol) was then added followed by the imidate donor **6** (Carbohydrate Res **2008,** *344*, 439-447.) (2.20 g, 3.89 mmol) in DCM (5 mL) over 5 min and the reaction mixture was stirred for 30 min at 0 °C. Quenched the RM with Et₃N (1mL), filtered and the solvents removed under vacuum. Crude product was purified by flash chromatography using EtOAc in hexanes to get product 5 (3.2 g, 98%).
¹H NMR (400 MHz, CDCl₃) δ=8.13 - 6.88 (m, 35H), 5.67 - 5.52 (m, 1 H), 5.46 - 5.31 (m, 1 H), 5.20 (s, 1 H), 5.16 - 4.89 (m, 3H), 4.68 (t, *J* = 11.2 Hz, 1 H), 4.55 (d, *J* = 8.1 Hz, 1.5H), 4.47 - 4.24 (m, 3.5H), 4.20 - 3.89 (m, 1.5H), 3.89 - 3.19 (m, 9.5H), 3.13 (td, *J* = 9.7, 4.9 Hz, 1 H), 2.63 (t, *J* = 10.2 Hz, 1 H), 0.63 (s, 9H), -0.12 (s, 3H), -0.19 (s, 3H). ¹³C NMR (101 MHz, CDCl₃) δ = 165.4, 165.36, 164.7, 138.2, 137.1, 133.4, 133.2, 129.94, 129.9, 129.2, 128.7, 128.6, 128.5, 128.4, 128.2, 128.0, 127.8, 127.3, 126.4, 101.7, 101.2, 101.1, 81.2, 75.5, 75.1, 74.6, 73.7, 73.4, 73.0, 68.9, 68.0, 67.3, 66.1, 51.7, 46.9, 25.6, 18.0, -4.1, -4.8.

### Example 1-4: Synthesis of compound 7

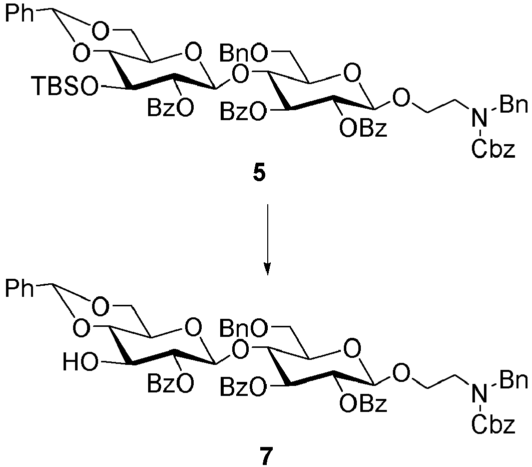

Substrate **5** (1.6 g, 1.317 mmol) was taken in pyridine (10 mL) at 0 °C and added HF-pyridine (3.56 mL, 39.5 mmol) to it and stirred at rt for 24 h. RM was washed with water and extracted with DCM (20 mL X 3). Combined organics were then washed with dil. HCl (50 mL X 2), sat. NaHCO₃ solution (50 mL), brine (10 mL), dried over Na₂SO₄, filtered and concentrated in vacuum to get crude product which on purification using silica column chromatography using 35-40% EtOAc in hexanes yielded white colored foam **7** (1.3 g, 90%).
¹H NMR (400 MHz, CDCl₃) δ = 8.15 - 6.92 (m, 1 H), 5.65 - 5.51 (m, 1 H), 5.44 - 5.30 (m, 1 H), 5.23 (s, 1 H), 5.11 - 5.04 (m, 3H), 4.77 - 4.49 (m, 3H), 4.49 - 4.24 (m, 4H), 4.25 - 3.91 (m, 2H), 3.91 - 3.59 (m, 4H), 3.57 - 3.00 (m, 7H), 2.68 (t, *J* = 10.3 Hz,1 H). ¹³C NMR (101 MHz, CDCl₃) δ = 165.4, 165.3, 156.4, 156.2, 138.2, 136.9, 133.6, 133.2, 130.3, 130.0, 129.9, 129.4, 128.7, 128.7, 128.5, 128.5, 128.4, 128.1, 128.1, 127.8, 127.4, 126.4, 101.8, 101.2, 101.1, 80.6, 75.9, 74.9, 74.7, 73.7, 73.5, 72.6, 72.0, 71.9, 68.9, 67.9, 67.4, 67.2, 66.0, 51.7, 46.9, 45.9.

### Example 1-5: Synthesis of compound 8

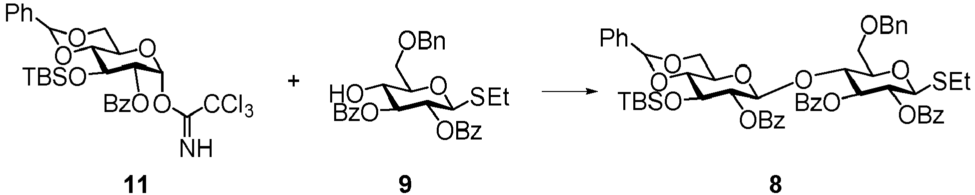

A mixture of (2S,3R,4S,5R,6R)-6-((benzyloxy)methyl)-2-(ethylthio)-5-hydroxytetrahydro-2H-pyran-3,4-diyl dibenzoate **9** (J Carbohydrate Chemistry 1993, 12, 309) (4.00 g, 7.654 mmol, 1.0 eq.) and (4aR,6R,7R,8aR)-8-((tert-butyldimethylsilyl)oxy)-2-phenyl-6-(2,2,2-trichloro-1-iminoethoxy)hexahydropyrano [3,2-d][1,3]dioxin-7-yl benzoate **11** (Carbohydrate Res, 2008, 344, 439.) (6.28 g, 9.95 mmol, 1.3 eq.) in DCM (140 mL) was stirred under an atmosphere of argon for 30 min. The reaction mixture was cooled (-20 °C) and TMSOTf (0.16 mL, 0.880 mmol, 0.115 eq.) was added. After stirring for 45 min, the reaction mixture was quenched by the addition of Et₃N (1.0 mL). The organic solution was concentrated under vacuo.

The resulting dark yellow oil was purified by flash chromatography over silica gel (EtOAc/hexanes, 1/3, v/v) to give (2S,3R,5R,6R)-5-(((4aR,6S,7R,8S,8aR)-7-(benzoyloxy)-8-((tert-butyldimethylsilyl)oxy)-2-phenylhexahydro pyranophenylhexahydropyrano[3,2-d][1,3]dioxin-6-yl)oxy)-6-((benzyloxy)methyl)-2-(ethylthio)tetrahydro-2H-pyran-3,4-diyl dibenzoate **8** (6 g, 79%) as a colorless solid: R_{f} = 0.5 (EtOAc/hexanes, 3/7, v/v). ¹H NMR (400 MHz, CDCl₃) δ = -0.19 (s, 3H), - 0.11 (s, 3H), 0.63 (s, 9H), 1.20 (t, J = 7.4 Hz, 3H), 2.67 (m, 2H), 3.15 (td, J = 9.7 Hz, 4.9 Hz, 2H), 3.28 (t, J = 9.2 Hz, 1 H), 3.53 - 3.37 (m, 1 H), 3.74 - 3.55 (m, 1 H), 3.79 (t, J = 9.0 Hz, 1H), 4.19 (t, J = 9.5 Hz, 1H), 4.37 (d, J = 12.2 Hz, 1H), 4.57 (d, J = 10.0 Hz, 1H), 4.59 (dd, J = 15.1, 9.0 Hz, 2H), 4.67 (d, J = 12.2 Hz, 1H), 5.12 (dd, J = 8.9, 8.2 Hz, 1 H), 5.21 (s, 1 H), 5.41 (t, J = 9.8 Hz, 1 H), 5.63 (t, J = 9.3 Hz, 1 H), 7.29 - 7.72 (m, 19H), 7.88 - 8.03 (m, 6H). ¹³C NMR (101 MHz, CDCl₃) δ = 165.27, 165.07, 164.45, 138.16, 137.00, 133.14, 133.10, 132.97, 130.21, 129.79, 129.77, 129.75, 129.34, 128.99, 128.51, 128.38, 128.28, 128.22, 128.05, 128.02, 127.99, 126.21, 101.57, 101.06, 83.39, 81.05, 78.70, 77.43, 77.11, 76.80, 75.41, 74.93, 74.52, 73.49, 72.90, 70.59, 67.86, 67.45, 65.97, 25.43, 24.08, 17.80, 14.85, -4.20, -4.97.

### Example 1-6: Synthesis of compound 12

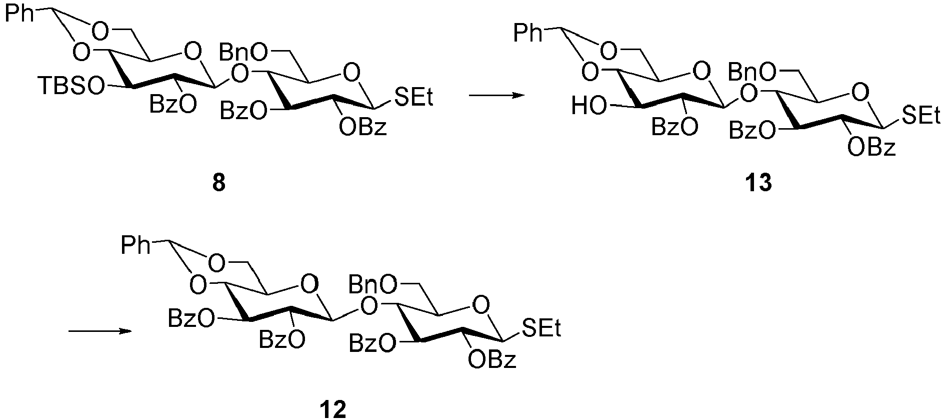

TBS substrate **8** (2.0g, 2.018 mmol, 1 equiv.) was taken in pyridine (10 mL) at 0 °C and added 70% HF-pyridine (5.45 mL, 60.5 mmo, 30 equiv.) to it and stirred at rt for 36 h.
RM was washed with water (50 mL) and extracted with DCM (50 mL X 3). Combined organics were then washed with sat. NaHCO₃ solution (50 mL), brine (20 mL), dried over Na₂SO₄, filtered and concentrated in vacuum to get crude product which on purification using silica column chromatography using 35-40% EtOAc/Hexanes to yield white colored foam 13 (1.7g, 96%).
¹H NMR (400 MHz, CDCl₃) δ = 8.08 - 7.85 (m, 6H), 7.69 - 7.28 (m, 19H), 5.62 (t, *J* = 9.3 Hz, 1 H), 5.41 (t, *J* = 9.8 Hz, 1 H), 5.22 (s, 1 H), 5.08 (dd, *J* = 9.2, 7.9 Hz, 1 H), 4.70 (d, *J =* 7.8 Hz, 1 H), 4.63 (d, *J* = 12.1 Hz, 1 H), 4.58 (d, *J* = 10.0 Hz, 1 H), 4.40 (d, *J* = 12.1 Hz, 1 H), 4.19 (t, *J* = 9.5 Hz, 1 H), 3.82 (td, *J* = 9.2, 3.6 Hz, 1 H), 3.70 (dd, *J* = 11.2, 3.4 Hz, 1 H), 3.63 (dd, *J* = 10.6, 5.0 Hz, 1 H), 3.59 - 3.55 (m, 1 H), 3.54 - 3.48 (m, 1 H), 3.32 (t, *J* = 9.3 Hz, 1 H), 3.15 (td, *J* = 9.7, 5.0 Hz, 1 H), 2.78 - 2.60 (m, 3H), 2.50 (d, *J* = 3.6 Hz, 1 H), 1.22 (t, *J* = 7.5 Hz, 3H). ¹³C NMR (101 MHz, CDCl₃) δ = 165.38, 165.29, 165.13, 138.02, 136.70, 133.43, 133.14, 133.09, 130.08, 129.87, 129.81, 129.71, 129.30, 129.28, 129.23, 128.50, 128.48, 128.29, 127.95, 127.86, 126.19, 101.59, 100.88, 83.42, 80.42, 78.71, 75.71, 74.67, 74.51, 73.46, 72.42, 70.48, 67.70, 67.51, 65.76, 24.11, 14.85.

Substrate **13** (1.6g, 1.824 mmol, 1 equiv.) was taken in anhydrous DCM (10 mL) at 0°C and added pyridine (10 mL) and BzCl (0.635 mL, 5.47 mmol, 3 equiv.) to it dropwise and RM was stirred for 16 h.

RM was then evaporated in vacuum to remove solvents and then taken again in DCM (25 mL) and washed with aq.NaHCO₃ solution(5mL X 2). Organic layer was then dried on Na₂SO₄, filtered and evaporated in vacuum. which was then triturated using methanol to get off-white solid **(12),** filtered, dried in vacuum (1.5g, 84%).
¹H NMR (400 MHz, CDCl₃₃) δ 7.95 (ddd, *J* = 16.9, 12.1, 7.3 Hz, 8H), 7.60 - 7.12 (m, 22H), 5.66 (t, *J* = 9.3 Hz, 1 H), 5.58 (t, *J* = 9.6 Hz, 1 H), 5.43 (t, *J* = 9.8 Hz, 1 H), 5.36 (dd, *J* = 9.5, 7.9 Hz, 1 H), 5.20 (s, 1 H), 4.77 (d, *J* = 7.9 Hz, 1 H), 4.60 (t, *J* = 10.3 Hz, 2H), 4.39 (d, *J* = 12.1 Hz, 1 H), 4.23 (t, *J* = 9.5 Hz, 1 H), 3.74 - 3.43 (m, 5H), 3.29 (td, *J* = 9.7, 4.9 Hz, 1 H), 2.83 - 2.55 (m, 3H), 1.22 (t, *J* = 7.4 Hz, 3H). ¹³C NMR (101 MHz, CDCl₃) δ 165.46, 165.25, 165.16, 164.72, 137.82, 136.62, 133.31, 133.12, 133.02, 130.05, 129.80, 129.73, 129.70, 129.27, 129.24, 128.99, 128.97, 128.63, 128.43, 128.29, 128.22, 128.17, 128.10, 127.98, 126.03, 101.12, 83.39, 78.65, 78.29, 75.85, 74.44, 73.44, 72.43, 71.96, 70.47, 67.71, 67.31, 66.16, 24.04, 14.84.

### Example 1-7: Synthesis of 2,3-di-O-benzoyl-β-D-glucopyranosyl-(1→4)-2,3-di-O-benzoyl-6-O-benzyl-β-D-glucoyranosyl-(1→1)-(2-N-benzyl-N-benzyloxycarbonylamino)ethanol (14)

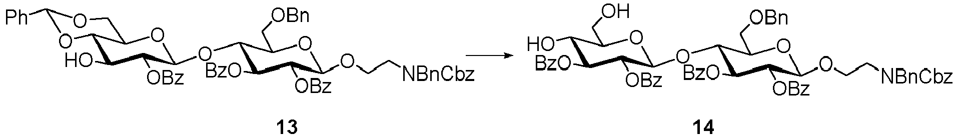

To a stirred solution of alcohol **13** (400 mg, 0.36 mmol) in pyridine (5.0 mL) was added at 0 °C benzoyl chloride (63 µL, 0.55 mmol). The reaction was slowly warmed to room temperature and stirred for 16 h at that temperature. An additional 0.5 equiv. BzCl were added to drive the reaction to completion. The mixture was stirred for 2 h at room temperature, quenched with water (30 ml) and diluted with EtOAc (50 mL). After separation, the organic fraction was washed with 0.1 M HCl (20 mL) and the aqueous fraction was re-extracted with EtOAc (30 mL). The combined organic fractions were washed with sat. aq. NaHCO₃ (20 mL) and brine (10 mL), dried over Na₂SO₄ and concentrated to give the intermediate tetrabenzoate as a yellow oil.
To a stirred solution of the intermediate tetrabenzoate in CH₂Cl₂ (6.5 mL) were added at room temperature ethanethiol (0.36 mL, 4.9 mmol) and p-toluenesulfonic acid (12 mg, 0.06 mmol). The mixture was stirred for 2 h at that temperature, quenched with Et₃N (50 µL) and concentrated. The residue was purified by flash chromatography (EtOAc/hexanes 0:1 to 1:10 to 1:8) to give diol **14** (389 mg, 0.349 mmol) as a white foam. HRMS (ESI) calcd. for C₆₄H₆₁NO₁₇ (M+Na)⁺ 1138.3837 found 1138.3850 *m*/*z.*

### Example 1-8: Synthesis of methyl(2,3-di-O-benzoyl-β-D-glucopyranosyl)uronate-(1→4)-2,3-di-O-benzoyl-6-O-benzyl-β-D-glucoyranosyl-(1→1)-(2-N-benzyl-N-benzyloxycarbonylamino)ethanol (15)

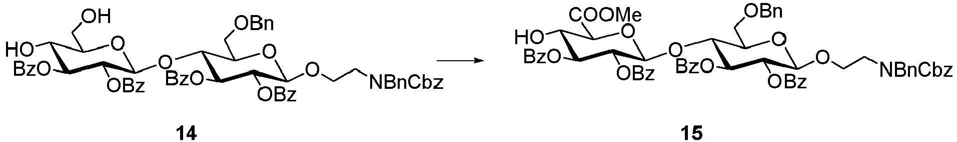

To a stirred solution of alcohol **14** (90 mg, 0.081 mmol) in CH₂Cl₂ (2.0 mL) and water (0.8 mL) were added at 0 °C TEMPO (2.5 mg, 0.016 mmol) and BAIB (55 mg, 0.170 mmol). The reaction was stirred for 20 min at that temperature and warmed to room temperature. The mixture was stirred for 2 h at that temperature and diluted with EtOAc (20 mL) and water (10 mL). After separation, the aqueous fraction was extracted with EtOAc (2x 10 mL), the combined organic fractions were dried over Na₂SO₄ and concentrated. The residue was purified by flash chromatography (EtOAc/hexanes 1:2 to 1:1, then 1:1 + 5% AcOH) to give the intermediate carboxylic acid as a white foam.
To a stirred solution of the intermediate carboxylic acid in toluene (1.6 mL) and MeOH (0.8 mL) was added at room temperature TMS-diazomethane (0.04 mL, 0.081 mmol). The reaction was stirred for 2 h at that temperature. An additional 0.25 equiv. TMS-diazomethane was added to drive the reaction to completion. The mixture was stirred for 1 h at that temperature, quenched with AcOH (0.1 mL) and concentrated. The residue was purified by flash chromatography (EtOAc/hexanes 0:1 to 1:1) to give methyl ester **15** (73 mg, 0.064 mmol, 79% over two steps) as a clear oil. HRMS (ESI) calcd. for C₆₅H₆₁NO₁₈ (M+Na)⁺ 1166.3786 found 1166.3762 *m*/*z.*

### Example 1-9: Synthesis of t-hexyl 2,3,6-tri-O-benzyl-β-D-galactopyranoside (17)

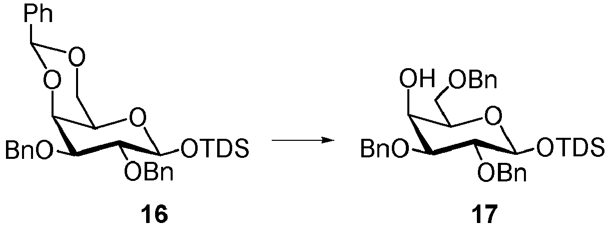

To a stirred solution of benzylidene acetal **16** (*J Carbohyd Chem* **1996,** 15 (2), 241) (1.68 g, 2.84 mmol) in CH₂Cl₂ (60 mL) over activated MS (3 A-AW) were added at 0 °C triethyl silane (2.72 mL, 17.06 mmol) and trifluoroacetic acid (1.81 mL, 17.06 mmol). The reaction was slowly warmed to room temperature and stirred for 16 h at that temperature. The reaction was quenched with Et₃N (2 mL), filtered through Celite and concentrated. The residue was purified by flash chromatography (EtOAc/hexanes 1:20 to 1:7) to give alcohol 17 (1.46 g, 2.46 mmol, 87%) as a clear oil. HRMS (ESI) calcd. for C₃₅H₄₈O₆Si (M+Na)⁺ 615.3117 found 615.3104 *m*/*z.*

### Example 1-10: Synthesis of tHexyl 4-O-benzoyl-2,3,6-tri-O-benzyl-α-D-glucopyranosyl-(1→4)-2,3,6-tri-O-benzyl-β-D-galactopyranoside (44)

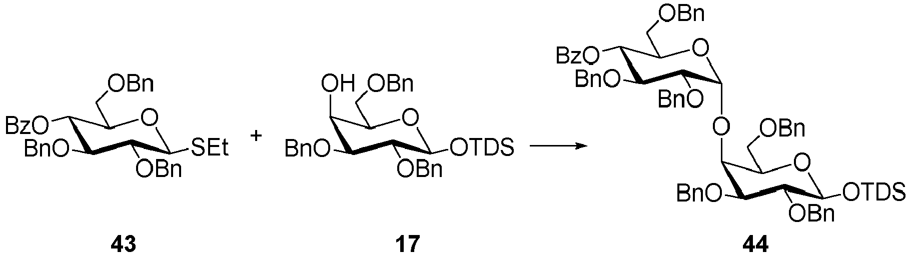

Thioglycoside **43** (J. Org. Chem. 2012, 77 (1), 291). (667 mg, 1.11 mmol) and alcohol 17 (550 mg, 0.93 mmol) were co-evaproated with dry toluene (3x10 mL) and kept under high vacuum for 30 min. The mixture was dissolved in Et₂O (14 mL) and CH₂Cl₂ (2.8 mL) and stirred over activated molecular sieves (3 A-AW) for 30 min at room temperature. The solution was cooled to -20 °C and treated with NIS (250 mg, 1.11 mmol) and triflic acid (16 µL, 0.19 mmol). The mixture was stirred for 1 h and slowly warmed to -10 °C. The reaction was quenched with Et₃N (0.05 mL), diluted with CH₂Cl₂ (20 mL), filtered through Celite and concentrated. The residue was purified by flash chromatography (EtOAc/hexanes 0:1 to 1:8 to 1:6) to give disaccharide **44** (553 mg, 0,490 mmol, 53%) along with the corresponding β-anomer (231 mg, 0.205 mmol, 22%). Analytical data for 7: Clear oil. HRMS (ESI) calcd. for C₆₉H₈₀O₁₂Si (M+Na)⁺ 1151.5316 found 1151.5293 *m*/*z.*

### Example 1-11: Synthesis of 4-O-benzoyl-2,3,6-tri-O-benzyl-α-D-glucopyranosyl-(1→4)-2,3,6-tri-O-benzyl-αβ-D-galactopyranosyl trifluoro-(N-phenyl)acetimidate (45)

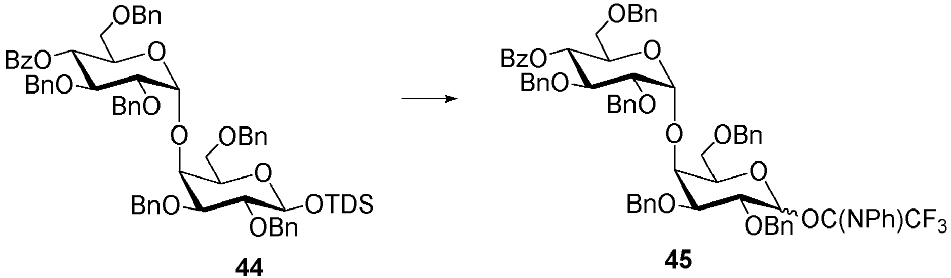

To a stirred solution of silyl ether **44** (470 mg, 0.416 mmol) in THF (8.3 mL) was added at 0 °C acetic acid (0.24 mL, 4.19 mmol) and TBAF (1.0 M solution in THF, 4.2 mL, 4.20 mmol). The reaction was slowly warmed to room temperature and stirred for 2 h at that temperature. Acetic acid (0.24 mL, 4.19 mmol) and TBAF (1.0 M solution in THF, 4.2 mL, 4.20 mmol) were added and the reaction was stirred for 16 h at room temperature. The mixture was diluted with Et₂O (50 mL), washed with water (3x30 mL) and the aqueous phase was re-extracted with Et₂O (2x20 mL). The combined organic fractions were dried over Na₂SO₄ and concentrated. The residue was filtered through a short plug of silica gel (EtOAc/hexanes 1:3 to 1:1) to give the intermediate lactol mixture as a clear oil.
To a stirred solution of the lactol mixture in CH₂Cl₂ (7.8 mL) were added at room temperature cesium carbonate (318 mg, 0.975 mmol) and F₃CC(NPh)Cl (202 mg, 0.975 mmol). The mixture was stirred for 2.5 h at that temperature, diluted with hexanes (0.5% (v/v) Et₃N, (10 mL) and filtered through Celite. The residue was purified by flash chromatography (EtOAc/hexanes 0:1 + 0.5%Et₃N to 1:3 + 0.5% Et₃N) to give imidate mixture **45** (404 mg, 0.349 mmol, 84% over two steps) as a clear oil. HRMS (ESI) calcd. for C₆₉H₆₆F₃NO₁₂ (M+Na)⁺ 1180.4434 found 1180.4458 *m*/*z*.

### Example 1-12: Synthesis of 4-O-benzoyl-2,3,6-tri-O-benzyl-α-D-glucopyranosyl-(1→4)-2,3,6-tri-O-benzyl-α-D-galactopyranosyl-(1→4)-methyl [2,3-Di-O-benzoyl-β-D-glucopyranosyl]uronate-(1→4)-2,3-di-O-benzoyl-6-O-benzyl-β-D-glucoyranosyl-(1→1)-(2-N-benzyl-N-benzyloxycarbonylamino)ethanol (46)

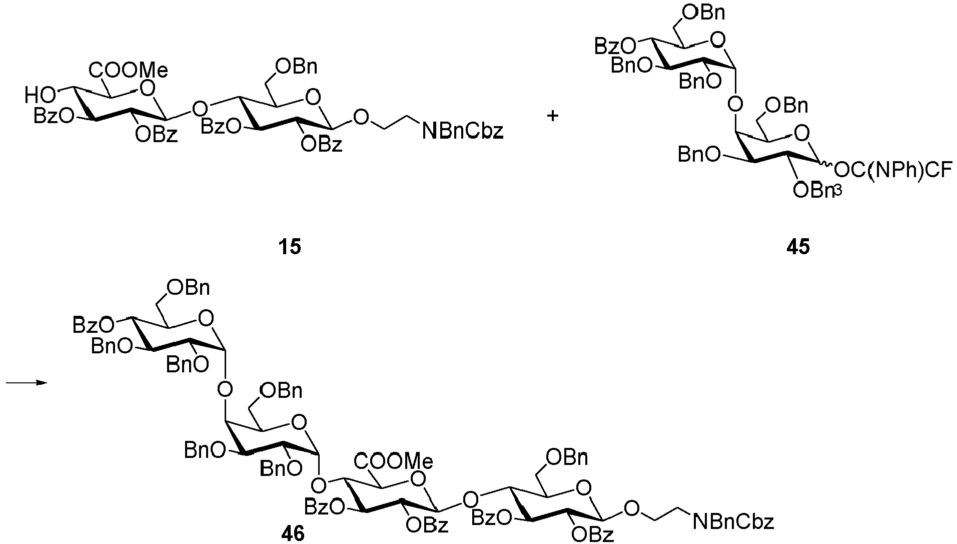

Alcohol **15** (100 mg, 87 µmol) and imidate **45** (121 mg, 105 µmol) were co-evaproated with dry toluene (3x10 mL) and kept under high vacuum for 30 min. The mixture was dissolved in Et₂O (3.3 mL) and CH₂Cl₂ (1.1 mL) and stirred over activated molecular sieves (3 A-AW) for 30 min at room temperature. The solution was cooled to -20 °C and treated with TMSOTf (3.2 µL, 17 µmol). The mixture was stirred for 1 h and slowly warmed to 0 °C. The reaction was quenched with sat. aq. NaHCO₃ (10 mL), extracted with CH₂Cl₂ (3x20 mL) and the combined organic fractions were dried over Na₂SO₄ and concentrated. The residue was purified by flash chromatography (EtOAc/hexanes/toluene 1:3:3 to 1:2:2) to give tetrsaccharide **46** (130 mg, 62 µmol, 71%) as a clear oil. HRMS (ESI) calcd. for C₁₂₆H₁₂₁NO₂₉ (M+Na)⁺ 2134.7921 found 2134.7879 *m*/*z.*

### Example 1-13: Synthesis of α-D-glucopyranosyl-(1→4)-α-D-galactopyranosyl-(1→4)-β-D-glucopyranosyluronic acid-(1→4)-β-D-glucoyranosyl-(1→1)-(2-amino)ethanol (10)

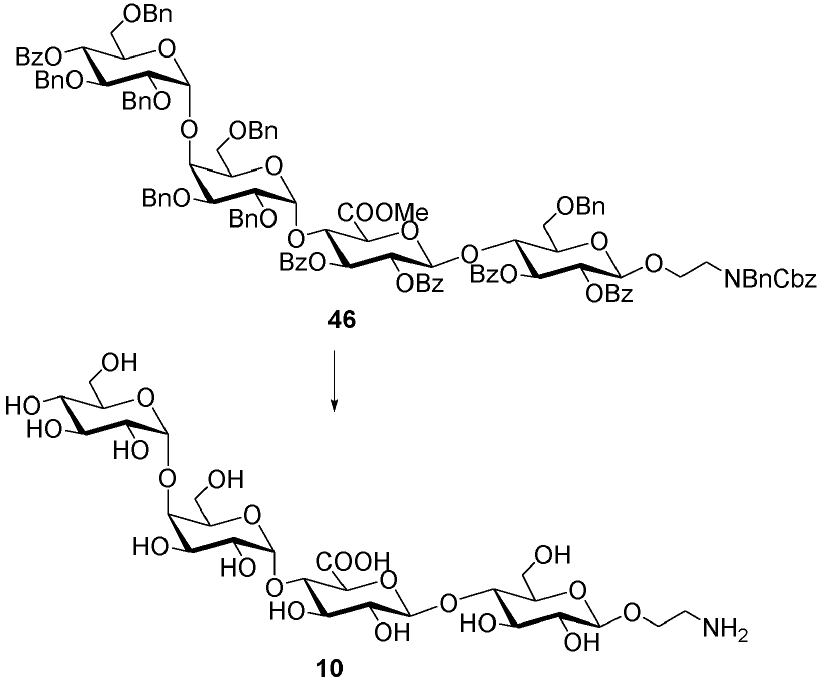

To a stirred solution of ester **46** (56 mg, 26 µmol) in THF (5 mL) and MeOH (1 mL) were added at 0 °C hydrogen peroxide (6% aq. solution, 265 µL, 530 µmol) and LiOH (1 M aq. solution, 265 µL, 132 mol). The reaction was stirred for 1 h and warmed to room temperature. The reaction was kept at that temperature and treated after 2 h with hydrogen peroxide (6% aq. solution, 265 µL, 530 µmol) and LiOH (1 M aq. solution, 265 µL, 132 mol). After 2 h, NaOH (15% aq. solution, 1 mL) was added and the mixture was stirred for 72 h at room temperature. The solvents were evaporated under reduced pressure, the residue was co-evaporated with toluene (2x5 mL) and dissolved in MeOH (5 mL). The solution was treated at room temperature with sodium methoxide (143 mg, 2.65 mmol) and stirred for 96 h at that temperature. The solvent was evaporated and the residue was dissolved in water (5 mL). The solution was neutralized at 0 °C with 0.5 M aq. NaHSO₄ and extracted with EtOAc (5x5 mL). The combined organic fractions were dried over Na₂SO₄ and concentrated to give the intermediate acid as a white foam.
The intermediate acid in MeOH (2 mL) was added at room temperature to a suspension of Pd/C (50 mg) in MeOH (1 mL), water (0.1 mL) and AcOH (5 drops). The reaction was stirred under an atmosphere of H₂ for 48 h, filtered and concentrated. The residue was purified by solid phase extraction (Chromabond C18, Macherey-Nagel) and lyophilized to give tetrasaccharide **10** (acetate salt, 13.6 mg, 18 µmol, 69% over 3 steps) as a white solid. HRMS (MALDI) calcd. for C₂₆H₄₅NO₂₂ (M+Na)⁺ 746.2330 found 746.2323 *m*/*z.*

### Example 1-14: Synthesis of 4-O-Benzoyl-2,3,6-tri-O-benzyl-α-D-glucopyranosyl-(1→4)-2,3,6-tri-O-benzyl-α-D-galactopyranosyl-(1→1)-(2-N-benzyl-N-benzyloxycarbonylamino)ethanol (47)

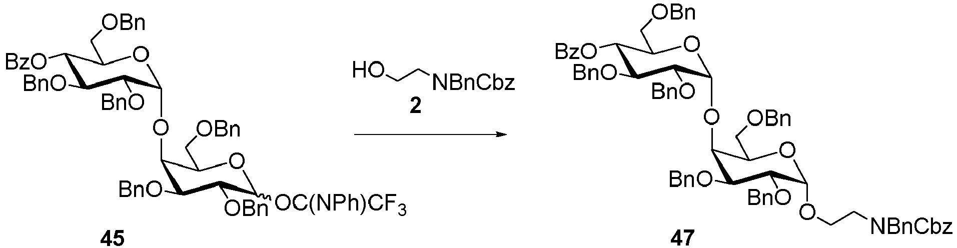

Imidate **45** (200 mg, 0.173 mmol) and alcohol **2** (74 mg, 0.259 mmol) were co-evaproated with dry toluene (2x5 mL) and kept under high vacuum for 30 min. The mixture was dissolved in Et₂O (2.8 mL) and CH₂Cl₂ (0.7 mL) and stirred over activated molecular sieves (3 A-AW) for 30 min at room temperature. The solution was cooled to -40 °C and treated with TMSOTf (6.2 µL, 35 µmol). The mixture was stirred for 10 min at that temperature and then slowly warmed to -10 °C. The reaction was quenched with sat. aq. NaHCO₃ (5 mL), extracted with CH₂Cl₂ (3x20 mL) and the combined organic fractions were dried over Na₂SO₄ and concentrated. The residue was purified by flash chromatography (EtOAc/hexanes 0:1 to 1:6 to 1:4) to give carbamate **47** (160 mg, 0.128 mmol, 74%) along with the corresponding β-anomer (32 mg, 0.026 mmol, 15%). Analytical data for **47:** Clear oil. HRMS (ESI) calcd. for C₇₈H₇₉NO₁₄ (M+Na)⁺ 1276.5398 found 1276.5405 *m*/*z.*

### Example 1-15: Synthesis of 2,3,6-tri-O-benzyl-α-D-glucopyranosyl-(1→4)-2,3,6-tri-O-benzyl-α-D-galactopyranosyl-(1→1)-(2-N-benzyl-N-benzyloxycarbonylamino)ethanol (48)

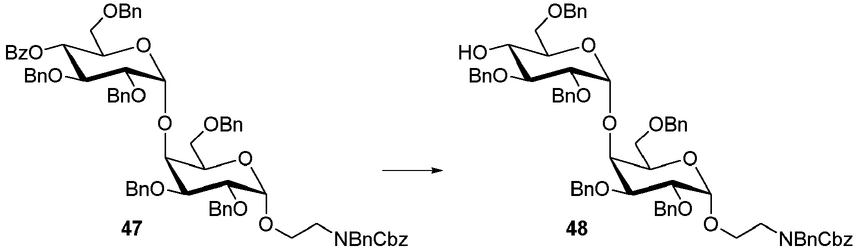

To a stirred solution of ester **47** (126 mg, 0.100 mmol) in THF (5 mL) and MeOH (5 mL) was added at 0 °C sodium methoxide (0.5 M in MeOH, 1 mL, 0.500 mmol). The reaction was slowly warmed to room temperature and kept at that temperature for 24 h. Sodium methoxide (0.5 M in MeOH, 1 mL, 0.500 mmol) was added and the reaction was warmed to 37 °C. The mixture was stirred for 7 h at that temperature, neutralized with Amberlite IR120 (H⁺ form), filtered and concentrated. The residue was purified by flash chromatography (EtOAc/hexanes 0:1 to 1:6 to 1:4) to give alcohol 48 (98 mg, 85 µmol, 85%) a s a clear oil. HRMS (ESI) calcd. for C₇₁H₇₅NO₁₃ (M+Na)⁺ 1172.5136 found 1172.5103 *m*/*z.*

### Example 1-16: Syntheis of α-D-glucopyranosyl-(1→4)-α-D-galactopyranosyl-(1→1)-(2-amino)ethanol (49)

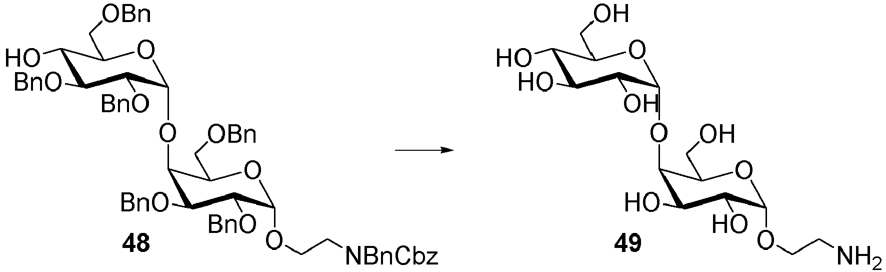

Benzyl ether **48** in EtOAc (1 mL) was added at room temperature to a suspension of Pd/C (30 mg) in MeOH (3 mL), water (0.5 mL) and AcOH (3 drops). The reaction was stirred under an atmosphere of H₂ for 24 h, filtered and concentrated to give disaccharide **49** (2.9 mg, 18 µmol, 87%) as a white solid. HRMS (ESI) calcd. for C₁₄H₂₇NO₁₁ (M+Na)⁺ 408.1481 found 408.1499 *m*/*z.*

### Example 1-17: Synthesis of 2,3-di-O-benzoyl-β-D-glucopyranosyl-(1→4)-2,3-di-O-benzoyl-6-O-benzyl-β-D-glucoyranosyl-(1→4)-2,3,6-tri-O-benzyl-α-D-glucopyranosy)-(1→4)-2,3,6-tri-O-benzy)-α-D-galactopyranosyl-(1→1)-(2-N-benzyl-N-benzyloxycarbonylamino)ethanol (50)

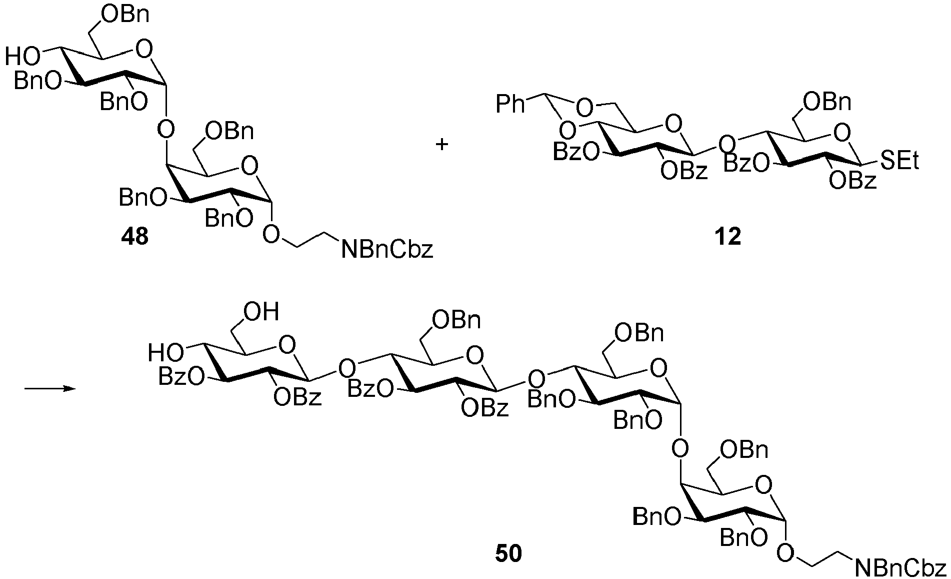

Alcohol **48** (47 mg, 41 µmol) and thioglycoside 12 (60 mg, 61 µmol) were co-evaporated with dry toluene (2x5 mL) and kept under high vacuum for 30 min. The mixture was dissolved in CH₂Cl₂ (2 mL) and stirred over activated molecular sieves (3 A-AW) for 30 min at room temperature. The solution was cooled to -10 °C and treated with NIS (13.8 mg, 61 µmol) and triflic acid (1 µL, 11 µmol). The mixture was kept for 1 h at that temperature and slowly warmed to 0 °C. The reaction was quenched with Et₃N (50 µL), filtered and concentrated to give the intermediate benzylidene acetal as a yellow oil.
To a stirred solution of the intermediate benzylidene acetal in CH₂Cl₂ (2 mL) were added at room temperature ethanethiol (0.3 mL, 4.06 mmol) and *p*-toluenesulfonic acid (10 mg, 0.053 mmol). The mixture was stirred for 1 h at that temperature, quenched with Et₃N (20 µL) and concentrated. The residue was purified by flash chromatography (EtOAc/hexanes 0:1 to 1:3 to 1:2) to give diol **50** (78 mg, 39 µmol, 96%) as a clear oil. HRMS (ESI) calcd. for C₁₁₈H₁₁₇NO₂₇ (M+Na)⁺ 2002.7710 found 2002.7731 *m*/*z.*

### Example 1-18: Synthesis of 2,3-di-O-benzoyl-β-D-glucopyranosyluronate-(1→4)-2,3-di-O-benzoyl-6-O-benzyl-β-D-glucoyranosyl-(1→4)-2,3,6-tri-O-benzyl-α-D-glucopyranosyl-(1→4)-2,3,6-tri-O-benzyl-α-D-galactopyranosyl-(1→1)-(2-N-benzyl-N-benzyloxycarbonylamino)ethanol (51)

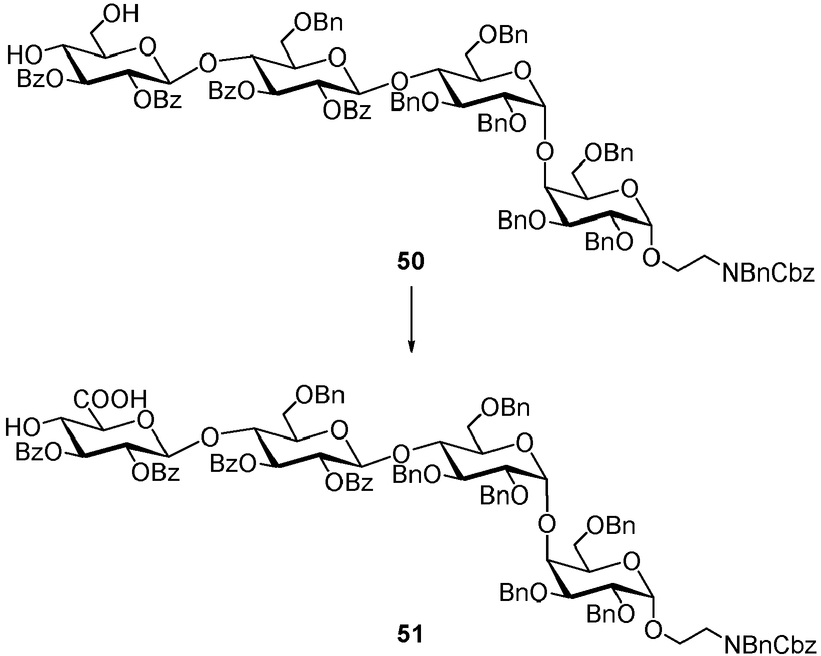

To a vigorously stirred solution of alcohol **50** (45 mg, 23 µmol, 73%) in CH₂Cl₂ (2 mL) and water (0.8 mL) were added at 0 °C TEMPO (3 crystals) and BAIB (15.4 mg, 48 µmol). The reaction was stirred for 20 min at that temperature and slowly warmed to room temperature. After 1 h, TEMPO (2 crystals) and BAIB (10 mg, 31 µmol) were added and the mixture was stirred for 2 h at room temperature. The reaction was diluted with CH₂Cl₂ (5 mL) and quenched with 10% aq. Na₂S₂O₃ (5 mL). Following separation, the aqueous phase was extracted with EtOAc (2x10 mL), the combined organic fractions were dried over Na₂SO₄ and concentrated. The residue was purified by flash chromatography twice (EtOAc/hexanes 0:1 to 1:2 to 8:1, then EtOAc/hexanes 1:1+1% AcOH) and co-evaporated with heptane repeatedly to give acid 51 (33 mg, 17 µmol, 74%) as a clear oil. HRMS (ESI) calcd. for C₁₁₈H₁₁₅NO₂₈ (M+Na)⁺ 2016.7503 found 2016.7558 *m*/*z.*

### Example 1-19: Synthesis of β-D-Glucopyranosyluronic acid-(1→4)-β-D-glucoyranosyl-(1 →4)-α-D-glucopyranosyl-(1→4)-α-D-galactopyranosyl-(1→1)-(2-amino)ethanol (18)

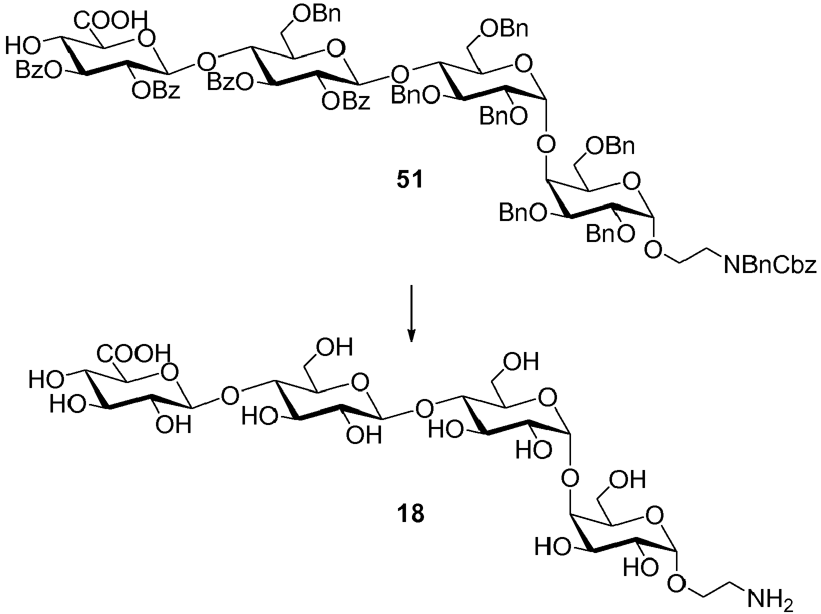

To a stirred solution of ester 51 (45 mg, 23 µmol) in THF (4 mL) and MeOH (0.5 mL) were added at 0 °C NaOH (1 M aq. solution, 1 mL). The reaction was slowly warmed to room temperature and stirred for 16 h at that temperature. The solution was neutralized at 0 °C with 0.5 M aq. NaHSO₄ and extracted with EtOAc (5x5 mL). The combined organic fractions were dried over Na₂SO₄ and concentrated to give the intermediate alcohol as a white foam.
The intermediate alcohol in MeOH (3 mL) was added at room temperature to a suspension of Pd/C (20 mg) in MeOH (6 mL), water (6 drops) and AcOH (3 drops). The reaction was stirred under an atmosphere of H₂ for 96 h, filtered and concentrated. Since the reaction had not proceeded to completion, the residue was subjected to the same conditions again and stirred for 72 h at room temperature. The reaction was filtered and concentrated, the residue was purified by solid phase extraction (Chromabond C18, Macherey-Nagel) and lyophilized to give tetrasaccharide **18** (11.3 mg, 16 µmol, 68% over 2 steps) as a white solid. HRMS (MALDI) calcd. for C₂₆H₄₅NO₂₂ (M+Na)⁺ 746.2330 found 746.2416 *m*/*z.*

### Example 1-20: Synthesis of methyl[2,3-di-O-benzoyl-β-D-glucopyranosyl]uronate-(1→4)-2,3-di-O-benzoyl-6-O-benzyl-β-D-glucoyranosyl-(1 →4)-2,3,6-tri-O-benzyl-α-D-glucopyranosyl-(1→4)-2,3,6-tri-O-benzyl-α-D-galactopyranosyl-(1→1)-(2-N-benzyl-N-benzyloxycarbonylamino)ethanol (52)

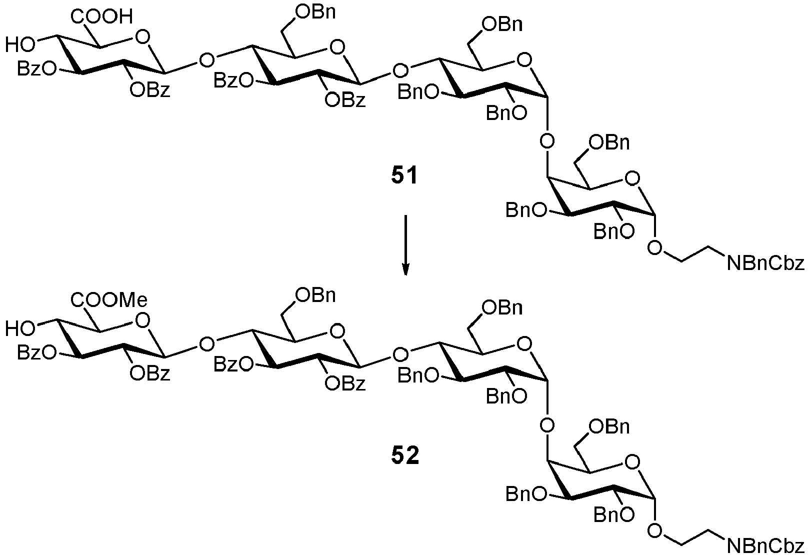

To a stirred solution of carboxylic acid **51** (100 mg, 50 µmol) in DMF (2.5 mL) were added at room temperature Cs₂CO₃ (24.5 mg, 75 µmol) and methyl iodide (10.7 mg, 75 µmol) and the reaction was stirred at that temperature. After 2 h, methyl iodide (10.7 mg, 75 µmol) was added and the mixture was stirred for another 2 h at room temperature. The reaction was quenched with sat. aq. NH4Cl (5 mL), extracted with EtOAc (4x10 mL), the combined organic extracts were dried over Na₂SO₄ and concentrated. The residue was purified by flash chromatography (EtOAc/hexanes 0:1 to 2:3) to give methyl ester **52** (81 mg, 40 µmol, 80%) as a white foam. HRMS (MALDI) calcd. for C119H117NO28 (M+Na)+ 2030.7659 found 2030.7660 m/z.

### Example 1-21: Synthesis of 2,3,4,6-tetra-O-benzyl-α-D-galactopyranosyl-(1→4)-methyl[2,3-di-O-benzoyl-β-D-glucopyranosyl]uronate-(1→4)-2,3-di-O-benzoyl-6-O-benzyl-β-D-glucoyranosyl-(1→4)-2,3,6-tri-O-benzyl-α-D-glucopyranosyl-(1 →4)-2,3,6-tri-O-benzyl-α-D-galactopyranosyl-(1→1)-(2-N-benzyl-N-benzyloxycarbonylamino)ethanol (53)

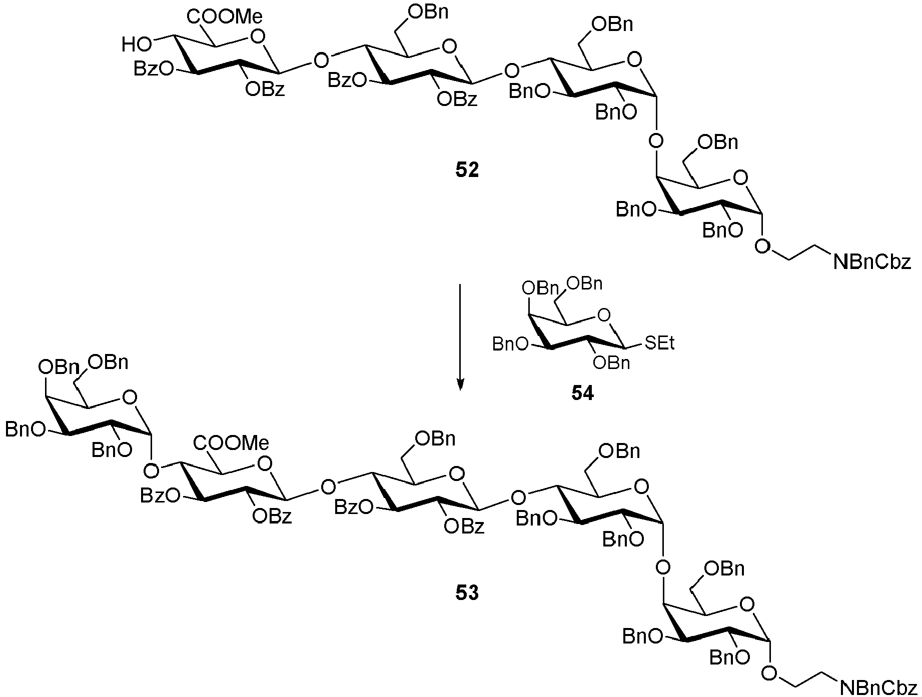

Alcohol **52** (14 mg, 7 µmol) and thioglycoside **54** (J Org Chem 1990, 55, 2860.) (16.3 mg, 28 µmol) were co-evaporated with dry toluene (3x10 mL) and kept under high vacuum for 30 min. The mixture was dissolved in Et₂O (1.05 mL) and CH₂Cl₂ (0.35 mL) and stirred over activated molecular sieves (3 A-AW) for 30 min at room temperature. The solution was cooled to -20 °C and treated with NIS (6.3 mg, 28 µmol) and TMSOTf (1 µL, 5.5 µmol). The mixture was stirred for 1 h and slowly warmed to 0 °C. The reaction was quenched with a 1:1 (v/v) mixture of sat. aq. NaHCO₃ (10 mL) and 10% (w/v) Na₂SO₃ (5 mL) and extracted with CH₂Cl₂ (4x10 mL). The combined organic fractions were dried over Na₂SO₄ and concentrated. The residue was purified by flash chromatography (EtOAc/hexanes 0:1 to 1:4 to 1:3) to give pentasaccharide 53 (12.5 mg, 4.9 µmol, 71%) as a clear oil. HRMS (MALDI) calcd. for C₁₂₆H₁₂₁NO₂₉ (M+Na)⁺ 2553.0066 found 2553.0066 *m*/*z.*

### Example 1-22: Synthesis of α-D-galactopyranosyl-(1→4)-β-D-glucopyranosyluronate-(1→4)-β-D-glucoyranosyl-α-D-glucopyranosyl-(1→4)-α-D-galactopyranosyl-(1→1)-(2-amino)ethanol (55)

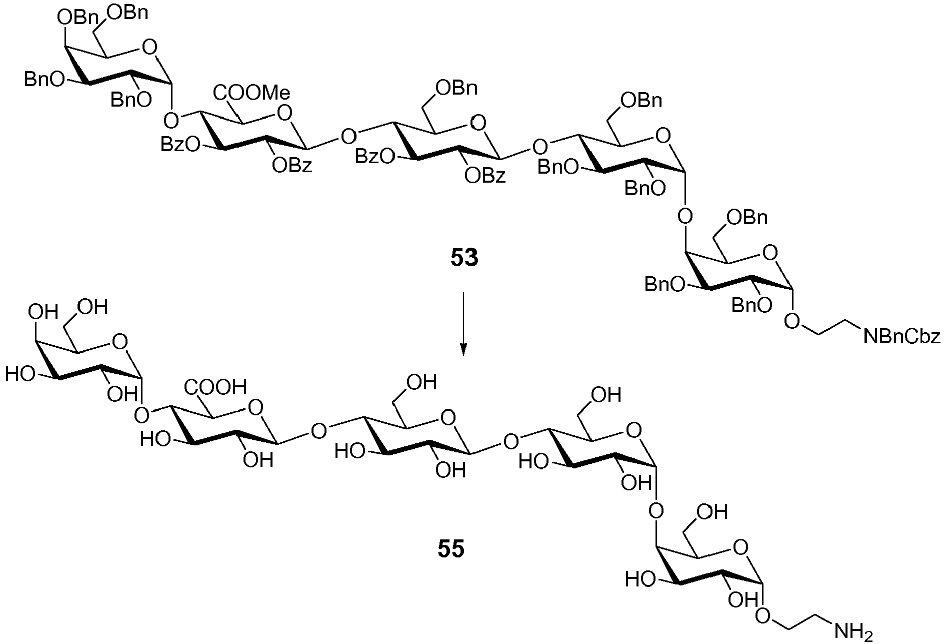

To a stirred solution of ester **53** (6 mg, 2.4 µmol) in THF (0.6 mL) and MeOH (0.6 mL) was added at 0 °C a 1:1 (v/v) mixture (94 µL) of hydrogen peroxide (2 M aq. solution, 96 µmol) and LiOH (0.5 M aq. solution, 24 µmol). The reaction was warmed to room temperature and treated after 2 h and 4 h with another 94 µL of the same LiOOH solution, respectively. The mixture was stirred for 16 h at room temperature and treated with NaOH (1 M aq. solution, 0.5 mL). The reaction was stirred for 6 h at that temperature, quenched with 10% aq. Na₂SO₃ (0.2 mL) and concentrated under reduced pressure. The residue was dissolved in MeOH (2 mL) and water (0.5 mL), neutralized with Amberlite IR-120 (H+ form), filtered and concentrated. The residue was purified by flash chromatography (EtOAc/hexanes 0:1 to 1:2 to 1:2/2% (v/v) AcOH) to give the intermediate carboxylic acid as a clear oil.
The intermediate carboxylic acid in CH₂Cl₂/tBuOH/water (1:16:8, 1 mL) was purged with argon and treated at 0 °C with a suspension of Pd(OH)₂ on carbon (20% (w/w) loading, 20 mg) in the same solvent mixture (0.5 mL). The suspension was purged with hydrogen and stirred under a hydrogen atmosphere for 18 h. The suspension was filtered and concentrated. The residue was purified by high performance liquid chromatography (Hypercarb 35005-159070, Thermo Fisher Scientific, Waltham, US; flow rate 1.7 mL/min, solvent A: water/0.1% formic acid (FA), solvent B: MeCN/0.1% FA; isocratic 0% B for 5 min, linear gradient to 35% (v/v) B in 10 min, linear gradient to 55% (v/v) B in 30 min, total retention time of about 18 min) and lyophilized to give pentasaccharide **55** as a white solid. ¹H NMR (400 MHz, D₂O) δ 5.48 (d, J = 3.5 Hz, 1H), 5.02 (d, J = 3.2 Hz, 1H), 4.88 (d, J = 3.9 Hz, 1H), 4.51 (m, 2H), 4.20 (d, J = 9.9 Hz, 1 H), 4.04 (m, 1 H), 4.02 - 3.52 (m, 26H), 3.43 - 3.19 (m, 4H).

### Example 1-23: Synthesis of 4-O-benzoyl-2,3,6-tri-O-benzyl-α-D-glucopyranosyl-(1→4)-2,3,6-tri-O-benzyl-α-D-galactopyranosyl-(1→4)-methyl[2,3-di-O-benzoyl-β-D-glucopyranosyl]uronate-(1→4)-2,3-di-O-benzoyl-6-O-benzyl-β-D-glucoyranosyl-(1→4)-2,3,6-tri-O-benzyl-α-D-glucopyranosyl-(1→4)-2,3,6-tri-O-benzyl-α-D-galactopyranosyl-(1→1)-(2-N-benzyl-N-benzyloxycarbonylamino)ethanol (56)

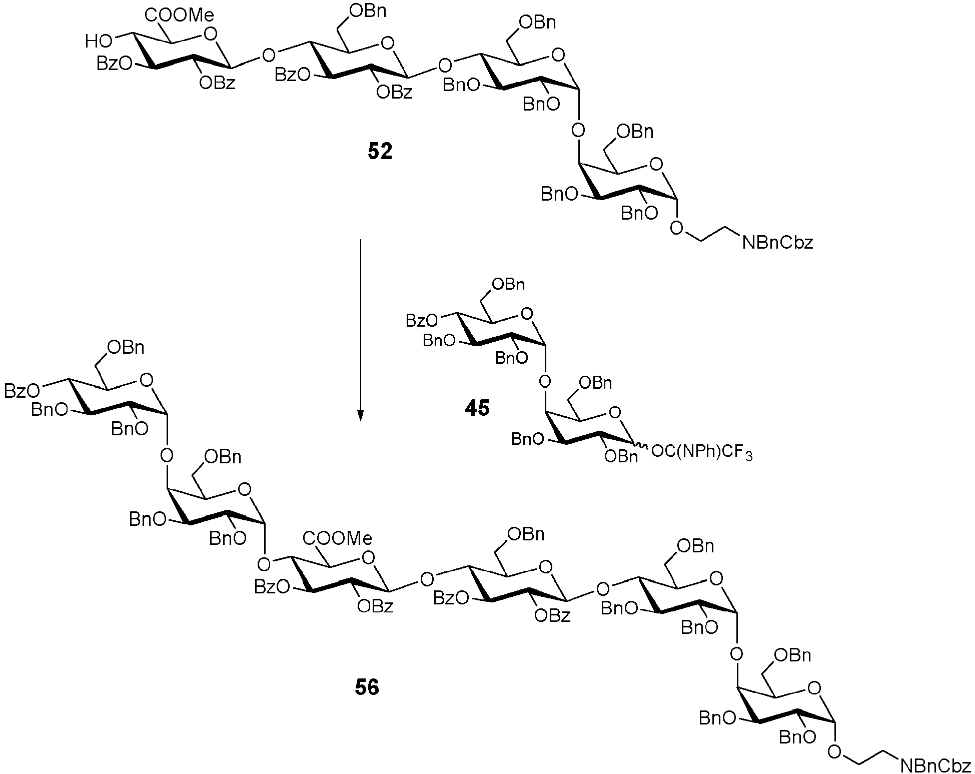

Alcohol 52 (50 mg, 25 µmol) and imidate 45 (72.1 mg, 62 µmol) were co-evaproated with dry toluene (3x10 mL) and kept under high vacuum for 30 min. The mixture was dissolved in Et₂O (2 mL) and CH₂Cl₂ (0.67 mL) and stirred over activated molecular sieves (3 A-AW) for 30 min at room temperature. The solution was cooled to -20 °C and treated with TMSOTf (2 µL, 11 µmol). The mixture was stirred for 1 h and slowly warmed to 0 °C. The reaction was quenched with sat. aq. NaHCO₃ (10 mL) and extracted with CH₂Cl₂ (4x10 mL). The combined organic fractions were dried over Na₂SO₄ and concentrated. The residue was purified by flash chromatography (EtOAc/hexanes 0:1 to 1:3 to 3:7 to 1:2) to give hexasaccharide **56** (51 mg, 17 µmol, 69%) as a clear oil. HRMS (MALDI) calcd. for C₁₈₀H₁₇₇NO₃₉ (M+2Na)²⁺ 1511.0847 found 1511.0576 *m*/*z.*

### Example 1-24: Synthesis of α-D-glucopyranosyl-(1→4)-α-D-galactopyranosyl-(1 →4)-β-D-glucopyranosyluronate-(1→4)-β-D-glucoyranosyl-(1→4)-α-D-glucopyranosyl-(1→4)-α-D-galactopyranosyl-(1→1)-(2-amino)ethanol (57)

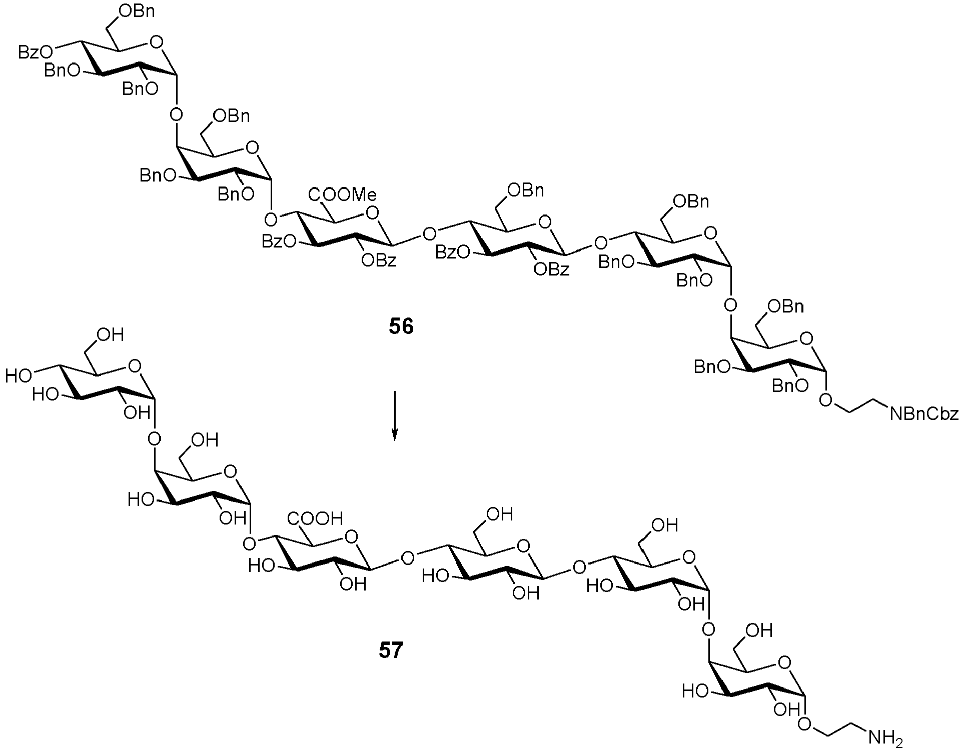

To a stirred solution of ester **56** (20 mg, 6.7 µmol) in THF (1 mL) and MeOH (1 mL) was added at 0 °C a 1:1 (v/v) mixture (268 µL) of hydrogen peroxide (2 M aq. solution, 96 µmol) and LiOH (0.5 M aq. solution, 24 µmol). The reaction was warmed to room temperature and treated after 2 h and 4 h with another 268 µL of the same LiOOH solution, respectively. The mixture was stirred for 16 h at room temperature and treated with NaOH (1 M aq. solution, 0.5 mL). The reaction was stirred for 18 h at that temperature, quenched with 10% aq. Na₂SO₃ (0.8 mL) and concentrated under reduced pressure. The residue was dissolved in water (4 mL), neutralized with NaHSO₄ (0.5 M aq. solution), extracted with EtOAc (4x10 mL), dried over Na₂SO₄ and concentrated. The residue was treated with sodium methoxide (0.5 M solution in MeOH, 1.5 mL, 750 µmol), warmed to 40 °C and stirred for 5 h at that temperature. The reaction was cooled to room temperature, stirred for another 16 h at that temperature and treated with water (0.5 mL). The mixture was neutralized with Amberlite IR-120 (H⁺ form), filtered and concentrated. The residues was purified by flash chromatography (EtOAc/hexanes 0:1:0 to 1:4/2% (v/v) AcOH to 1:1/2% (v/v) AcOH) to give the intermediate carboxylic acid as a clear oil.
The intermediate carboxylic acid in CH₂Cl₂/*t*BuOH/water (1:16:8, 2 mL) was purged with argon and treated at 0 °C with a suspension of Pd(OH)₂ on carbon (20% (w/w) loading, 30 mg) in the same solvent mixture (1 mL). The suspension was purged with hydrogen and stirred under a hydrogen atmosphere for 18 h. The suspension was filtered and concentrated. The residue was purified by size exclusion chromatography (Sephadex G-25, 4:1 (v/v) 5 mM aq. NH₄OAc/MeOH) and lyophilized to give hexasaccharide **57** (acetate salt) as a white solid. HRMS (ESI) calcd. for C₃₈H₆₅NO₃₂ (M+Na)⁺ 1070.3387 found 1070.3391 *m*/*z.*

### Synthesis of saccharide by stepwise automated glycosylation

### Example 1-25: Synthesis of glucose building block 32

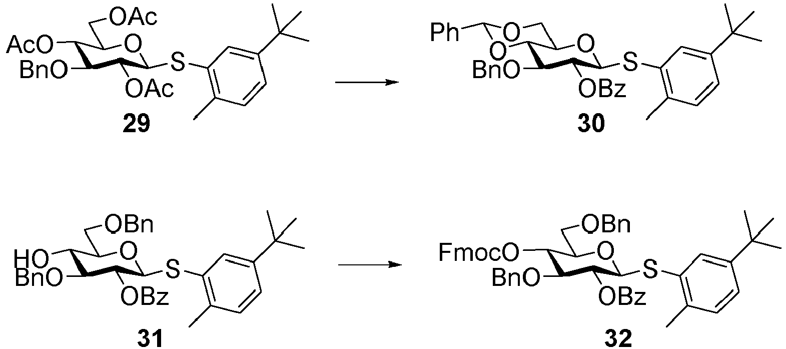

### (2-Methyl-5-tert-butylphenyl) 2-O-benzoyl-3-O-benzyl-4,6-O-benzylidene-1-thio-β-D-glucopyranose (30)

(2-Methyl-5-tert-butylphenyl) 2,4,6-tri-O-acetyl-3-O-benzyl-1-thio-β- D glucopyranose (29) was dissolved in MeOH, NaOMe (1.0 eq) was added and the reaction mixture was stirred overnight. The mixture was neutralized with IR-120-H⁺ amberlite resin, filtered off, concentrated and co-evaporated with toluene. The crude triol was dissolved in DMF. Benzaldehyde dimethyl acetal (2.0 eq) and a catalytic amount of para-toluene sulfonic acid were added and the mixture was stirred at 80 °C for 2 h. After the mixture was cooled to room temperature, saturated aqueous NaHCO₃ was added. After phase separation, the organic phase was extracted three times with ethyl acetate. The combined organic layers were washed with brine, dried over MgSO₄, filtered and concentrated. The crude was dissolved in DCM, cooled to 0 °C and Bz₂O (2.0 eq), DMAP (0.5 eq) and triethylamine (4.0 eq) were added. After complete conversion, the reaction was quenched with saturated aqueous NaHCO₃ and the combined organic layers were washed with brine, dried over MgSO₄, filtered and concentrated. The residue was purified by flash column chromatography on silica gel to provide compound (88% over three steps). Rf: 0.25 (Hexane:EtOAc = 9:1). [α]D = 68.96 (c = 3.18, CHCl₃). ); IR (thin film, chloroform): u = 2962, 1729, 1265, 1093 cm-1; 1 H-NMR (400 MHz, CDCl3) δ = 8.01 (ddd, J = 5.8, 5.3, 0.8 Hz, 2H, H Ar), 7.60 (ddd, J = 7.1, 2.6, 1.3 Hz, 1 H, H Ar), 7.51 (ddd, J = 6.0, 5.6, 2.3 Hz, 3H, H Ar), 7.49 - 7.43 (m, 2H, H Ar), 7.43 - 7.35 (m, 3H, H Ar), 7.19 (dt, J = 6.3, 3.1 Hz, 1 H, H Ar), 7.13 (dd, J = 6.7, 4.0 Hz, 3H, H Ar), 7.10 - 7.04 (m, 3H, H Ar), 5.63 (s, 1 H CHO2Ph), 5.42 - 5.32 (m, 1 H, H-2), 4.82 (d, J = 11.9 Hz, 1 H, CHHPh), 4.81 (d, J = 10.2 Hz, 1 H, H-1), 4.69 (d, J = 12.0 Hz, 1 H, CHHPh), 4.39 (dd, J = 10.5, 5.0 Hz, 1 H, H-6'), 3.94 - 3.86 (m, 3H, H-3, H-4, H-6), 3.56 (dt, J = 14.6, 4.9 Hz, 1 H, H-5), 2.19 (s, 3H), 1.27 (s, 9H). 13C-NMR (100 MHz, CDCl3) δ 165.23 (C=O), 149.66, 137.84, 137.28, 137.03, 133.34, 132.37, 130.07, 130.04, 129.93, 129.90, 129.19, 128.51, 128.43, 128.29, 128.22, 127.71, 126.13, 125.38 (Ar), 101.44 (CHO₂Ph), 88.03 (C-1), 81.64 (C-3), 79.43 (C-4), 74.36 (CH2Ph), 72.25 (C-2), 70.66 (C-5), 68.81 (C-6), 34.56 (Cq tBu thio), 31.36 (tBu), 20.36 (CH3 thio); MS ESI+-HRMS m/z [M+Na]+ calcd for C₃₈H₄₀O₆SNa 647.2462, found 647.

### (2-Methyl-5-tert-butylphenyl) 2-O-benzoyl-3,6-di-O-benzyl-1-thio-β-D-glucopyranose (31)

To a solution of compound 1 was co-evaporated with toluene, and dissolved in DCM (6.5 mL) under an argon atmosphere. Triethylsilane (0.62 mL, 3.88 mmol) and trifluoroacetic anhydride (0.27 mL, 1.94 mmol) were added and the solution was cooled to 0 °C. Trifluoroacetic acid (0.30 mL, 3.88 mmol) were added dropwise, and the reaction was stirred and allowed to warm to room temperature. After complete conversion of the starting material, the solution was diluted with DCM, and quenched with saturated aqueous NaHCO₃. The combined organic layer was dried over MgSO₄ and the solvent was removed in vacuo. The residue was purified by column chromatography on silica gel (hexane/ethyl acetate, 9:1 to 7:3) to give a white foam (92%). MS ESI+-HRMS m/z [M+Na]+ calcd for C₃₈H₄₂O₆SNa 649.2600 found 649.2585.

### (2-Methyl-5-tert-butylphenyl) 2-O-benzoyl-3,6-di-O-benzyl-4-O-fluorenylmethoxycarbonyl-1-thio-β-D-glucopyranoside (32)

Compound **31** was dissolved in DCM (6.5 mL) under an argon atmosphere. 9-fluorenylmethyl chloroformate (8.3 g, 31.9 mmol) and pyridine (3.44 mL, 42.5 mmol) were added into the solution at 0 °C. After complete conversion of the starting material, the solution was diluted with DCM, extracted with 1 M aqueous HCl and saturated aqueous NaHCO₃. The combined organic phase was dried over MgSO₄ and the solvent was removed in vacuo. The crude product was purified by silica gel flash column chromatography affording the title compound
MS ESI+-HRMS m/z [M+Na]+ calcd for C₅₃H₅₂O₈SNa 871.3281 found 871.3311.

### Example 1-26: Synthesis of glucose building block 35

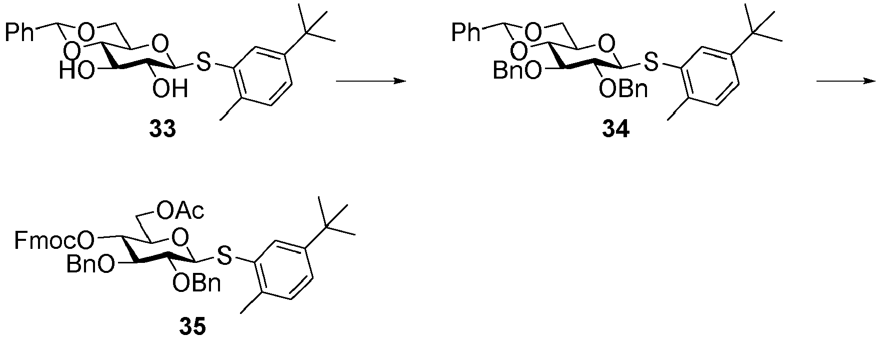

### (2-Methyl-5-tert-butylphenyl) 2,3-di-O-benzyl-4,6-O-benzylidene-1-thio-β-D-glucopyranoside (34)

(2-Methyl-5-*tert*-butylphenyl) 4,6-*O*-benzylidene-1-thio-β-D-glucopyranoside (33) was dissolved in DCM (6.5 mL) under an argon atmosphere. Benzyl bromide, and NaH were added into the solution at 0 °C. After complete conversion of the starting material, the reaction mixture was quenched with methanol, and diluted with ether, extracted with saturated NH₄Cl. The combined organic phase was dried over MgSO₄ and the solvent was removed *in vacuo.* The crude product was purified by silica gel flash column chromatography affording the title compound.
MS ESI+-HRMS m/z [M+Na]+ calcd for C₃₈H₄₂O₅SNa 633.2651 found 633.2644.

### (2-Methyl-5-tert-butylphenyl) 6-O-acetyl-2,3-di-O-benzyl-4-O-fluorenylmethoxycarbonyl-1-thio-β-D-galactopyranoside (35)

To the solution of (2-Methyl-5-*tert*-butylphenyl) 2,3-di-*O*-benzyl-4,6-*O*-benzylidene-1-thio-β-D-glucopyranoside (34) in DCM (6.5 mL) were added TFA and water. After completion, the crude was decanted with hexane to remove byproduct. The crude was used to the next reaction. To the solution of the crude was added acetic acid, 2-chloro-1-methylpyridium iodide, and DABCO at -15 °C. After complete conversion of the starting material, the solution was diluted with DCM, extracted with 1 M aqueous HCl and saturated aqueous NaHCO₃. The combined organic phase was dried over MgSO₄ and the solvent was removed in vacuo. The crude product was purified by silica gel flash column chromatography affording the title compound. The crude was dissolved in DCM (6.5 mL) under an argon atmosphere. 9-fluorenylmethyl chloroformate (8.3 g, 31.9 mmol) and pyridine (3.44 mL, 42.5 mmol) were added into the solution at 0 °C. After complete conversion of the starting material, the solution was diluted with DCM, extracted with 1 M aqueous HCl and saturated aqueous NaHCO₃. The combined organic phase was dried over MgSO₄ and the solvent was removed *in vacuo.* The crude product was purified by silica gel flash column chromatography affording the title compound.
MS ESI+-HRMS m/z [M+Na]+ calcd for C₄₈H₅₀O₈SNa 809.3124 found 809.3137.

### Example 1-27: Synthesis of (2-methyl-5-tert-butylphenyl) 3,6-di-O-acetyl-2,4-di-O-benzyl-1-thio-β-D-galactopyranoside (37)

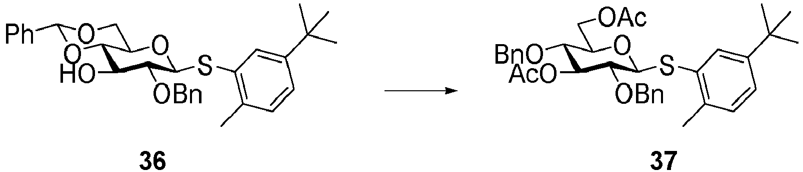

(2-Methyl-5-tert-butylphenyl)4,6-O-benzylidene-2-O-benzyl-1-thio-β-D-glucopyranoside **(36)** *(*Tetrahedron Letter, 1999, 40, 6523) was co-evaporated toluene and dissolved under an Ar atmosphere in DCM (170 mL). A 1 M solution of BH₃ in THF (108 mL, 108 mmol) was added and the solution was cooled to 0 °C. After 10 min, trimethylsilyl triflate (1.66 mL, 9.2 mmol) was added and the reaction was stirred. After completion, the solution was diluted with DCM, and quenched with saturated aqueous NaHCO₃ dropwise. The organic phase was dried over MgSO₄ and the solvent was removed in vacuo. The crude product was used without further purification for the next step.
To a solution of the crude in DCM was added acetic anhydride (8.3 g, 31.9 mmol) and pyridine (3.44 mL, 42.5 mmol). After complete conversion of the starting material, the solution was diluted with DCM, extracted with saturated aqueous NaHCO₃. The combined organic phase was dried over MgSO₄ and the solvent was removed *in vacuo.* The crude product was purified by silica gel flash column chromatography affording the title compound (37).
MS ESI+-HRMS m/z [M+Na]+ calcd for C₃₅H₄₂O₇SNa 629.2546 found 629.2522.

### Example 1-28: Synthesis of protected galactose building blocks

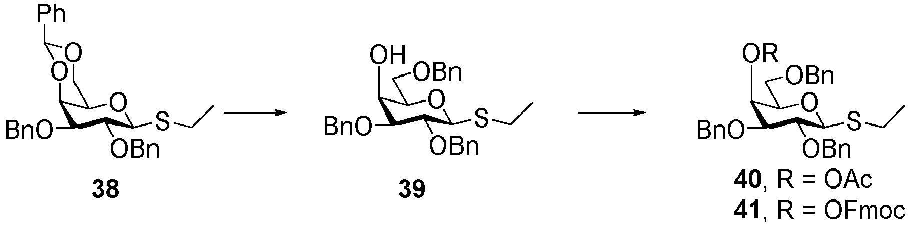

Ethyl 2,3-di-*O*-benzyl-4,6-*O*-benzylidene-1-thio-β-D-galactopyranoside **(38)** was co-evaporated with toluene, and dissolved in DCM (6.5 mL) under an Ar atmosphere. Triethylsilane (0.62 mL, 3.88 mmol) and trifluoroacetic anhydride (0.27 mL, 1.94 mmol) were added and the solution was cooled to 0 °C. Trifluoroacetic acid (0.30 mL, 3.88 mmol) were added dropwise, and the reaction was stirred and allowed to warm to room temperature. After complete conversion of the starting material, the solution was diluted with DCM, and quenched with saturated aqueous NaHCO₃. The combined organic layer was dried over MgSO₄ and the solvent was removed in vacuo. The residue was purified by column chromatography on silica gel (hexane/ethyl acetate, 9:1 to 7:3) to give a white foam (94%). [Christopher E. Martin, Markus W. Weishaupt, and Peter H. Seeberger, Chem. Commun., 2011, 47, 10260-10262.]

Ethyl 2,3,6-tri-*O*-benzyl-1-thio-β-D-galactopyranoside was dissolved in DCM (6.5 mL) under an argon atmosphere. Acetic anhydride (8.3 g, 31.9 mmol) and pyridine (3.44 mL, 42.5 mmol) were added into the solution at 0 °C. After complete conversion of the starting material, the solution was diluted with DCM, extracted with 1 M aqueous HCl and saturated aqueous NaHCO₃. The combined organic phase was dried over MgSO₄ and the solvent was removed in vacuo. The crude product was purified by silica gel flash column chromatography affording the title compound **(40).** (Chem. Commun., 2011, 47, 10260)

### Ethyl 2,3,6-tri-O-benzyl-4-O-fluorenylmethoxycarbonyl-1-thio-β-D-galactopyranoside (41)

To a solution of compound **39** were added 9-fluorenylmethyl chloroformate (8.3 g, 31.9 mmol) and pyridine (3.44 mL, 42.5 mmol) at 0 °C. After complete conversion of the starting material, the solution was diluted with DCM, extracted with 1 M aqueous HCl and saturated aqueous NaHCO₃. The combined organic phase was dried over MgSO₄ and the solvent was removed in vacuo. The crude product was purified by silica gel flash column chromatography affording the title compound. MS ESI+-HRMS m/z [M+Na]+ calcd for C₄₄H₄₄O₇SNa 739.2705 found 739.2673.

### Example 1-29: Preparation of glucuronic acid building block 42

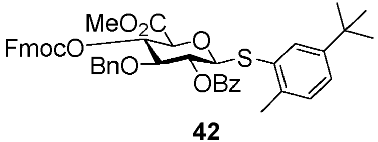

**Methyl (2-methyl-5-tert-butyl-phenyl)-2-*O*-benzoyl-3-*O*-benzyl-4-*O-*fluorenylmethoxycarbonyl-1-thio-β-D-glucopyranosyluronate (42)** was synthesized according to the procedure described in Angew. Chem. Int. Ed. 2013, 52, 5858.

### Example 1-30: Synthesis of the functionalized solid support

Functionalized resin **58** was synthesized according to the procedure described in Angew. Chem. Int. Ed. 2013, 52, 5858.

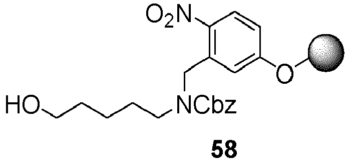

### Example 1-31: General Procedure 1: Automation Module

### Preparation of Stock Solutions

**Activator Solution:** N-Iodosuccinimide (1.48 g, 6.66 mmol) and TfOH (60 µL, 0.66 mmol) was dissolved in a mixture of DCM (20 mL) and dioxane (20 mL).

**Fmoc Deprotection Solution:** A solution of 20% triethylamine in DMF (v/v) was prepared.

**Thioglycoside Building Block Solution:** 0.25 mmol of building block was dissolved in 2 ml of DCM.

**Module 1: Glycosylation:** The resin is swollen in 2 mL DCM and the temperature of the reaction vessel is adjusted to T1. For the glycosylation reaction, the DCM is drained and a solution of building block (5 eq in 1.0 mL DCM) is delivered to the reaction vessel. After the set temperature is reached, the reaction is started by the addition of activator (5 eq in 1.0 mL of solution). The glycosylation is performed for 5 min at temperature T1 and for 45 min at T2. This procedure is repeated twice.
After the reaction was complete, the solution is drained and the resin is washed with DCM (six times each with 2 mL for 25 s). This procedure is repeated two times more.

**Module 2: Fmoc Deprotection:** The resin is washed with DMF (six time), swollen in 2 mL DMF and the temperature of the reaction vessel is adjusted to 25 °C. For Fmoc deprotection the DMF is drained and 2 mL of a solution of 20% triethylamine in DMF is delivered to the reaction vessel. After 5 min the reaction solution is collected in the fraction collector of the oligosaccharide synthesizer and 2 mL of a solution of 20% triethylamine in DMF is delivered to the resin. This procedure is repeated three times. For the next glycosylation the resin is washed with DMF, THF, DCM (six times each). For Fmoc quantification the reaction solutions are combined and a 100 µL aliquot is taken. This aliquot is diluted with a solution of 20% triethylamine in DMF to 5 mL and the UV absorption at λ = 294 nm is determined.

### Cleavage from Solid Support (Angew. Chem. Int. Ed. 2013, 52, 5858-5861):

**Continuous photocleavage flow reactor-general procedure:** The flow reactor setup consists of a medium pressure Hg lamp (Hanovia) with arc lengths of 27.9 cm and power of 450 W surrounded by a UV filter (Pyrex, 50% transmittance at 305 nm) in a quartz glass cooling system connected to a chiller to maintain a reaction temperature of 25 °C. A fluorinated ethylene propylene (FEP) tubing (inner diameter: 0.03 inch; volume: 12 mL) is wrapped around the cooling system. A syringe pump is connected to the FEP tubing and is used to flush solvents and resin via the inlet through the reactor. The solid support is filtered off by a frit and the product solution is pooled and the solvents are removed *in vacuo.* The UV lamp is located in a box that is additionally cooled by a fan.

To finish the automated synthesis process, the resin is washed with DCM (six times), swollen in 2 mL DCM and transferred into a disposable syringe (20 mL). To prepare the photoreactor, the FEP tubing is washed with 15 mL MeOH, then subsequently with 15 mL DCM using a flow rate of 4 mL·min-1. For the cleavage, the resin is slowly injected from the disposable syringe (20 mL) into the reactor and pushed through the tubing with 15 mL DCM (flow rate: 500 µL·min-1). To wash out remaining resin, the tubing is washed with 20 mL DCM (flow rate: 500 µL·min-1). The suspension leaving the reactor is directed into a filter where the resin is filtered off and washed with DCM. The tubing is re-equilibrated with 15 mL DCM using a flow rate of 4 mL·min-1. The entire procedure is performed twice. The resulting solution is evaporated *in vacuo* and the crude product is purified by HPLC (column: Luna silica; flow rate: 5 mL·min-1).

### General Procedure 2: Global Deprotection

To a solution of purified saccharide in a mixture of THF and MeOH (1.2 ml, v/v = 4:1) were added 1 M LiOH-35% H₂O₂ (150 uL, v/v = 2:1) at 0 °C. The reaction was warmed up to room temperature, kept. After 4hr, 1 M KOH (0.5 mL) was added and the mixture was stirred. After completion, the mixture was neutralized with IR-120-H⁺ amberlite resin, filtered off, concentrated. The crude was dissolved in MeOH, ethyl acetate, and acetic acid (5 mL: 0.75 mL: 0.25 mL), followed by Pd/C (20mg). The mixture was bubbled under an atmosphere of argon for 30 min, then at atmosphere of H₂ for 12 hr, filtered and concentrated. The residue was purified by HPLC (Hyper-carbon) and lyophilized to give saccharide (formic acid salt) as a white solid.

### General procedure to run automation:

Functionalized resin **58** (65 mg; loading 0.385 mmol/g; 0.025 mmol) was loaded into the reaction vessel of the synthesizer and swollen in 2 mL DCM. To start the synthesis sequence, the resin was washed consecutively with DMF, THF, then DCM (three times each with 2 mL for 25 s). Module 1 for each building block and module 2 for Fmoc deprotection were performed to produce each saccharide structure.

### Purification on HPLC

After synthesis of the desired compound, the resin was cleaved from the solid support. The crude product was purified by semi-preparative HPLC (column: Luna-Silica (21×250 mm; 5 µm); flow rate: 5 mL/min; eluents: Hexane/Ethyl acetate; gradient: 20% (5 min) → 60% (in 45 min) → 100% (in 5 min); detection: 210 and 280 nm) affording the target oligosaccharide.

After global deprotection of the desired compound, the crude product was purified by semi-preparative HPLC (column: Luna-Hyper-Carbon (21×250 mm; 5 µm); flow rate: 5 mL/min; eluents: 0.1 % formic acid in Water / 0.1 % formic acid in acetonitrile; gradient: 10% (5 min) → 40% (in 30 min) → 100% (in 5 min); detection: ELSD) affording the target oligosaccharide.

### Example 1-32: Synthesis of N-benzyloxycarbonyl-5-amino-pentanyl methyl 2-O-benzoyl-3-O-benzyl-β-D-glucopyranosyluronate-(1→4)-2-O-benzoyl-3,6-di-O-benzyl-β-D-glucopyranosyl-(1→4)-6-O-acetyl-2,3-di-O-benzyl-α-D-glucopyranosyl-(1→4)-2,3,6-tri-O-benzyl-α-D-galactopyranoside (19a)

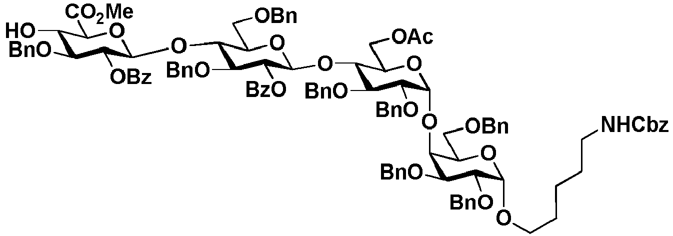

Tetrasaccharide **21a** was prepared according to the reactions sequence:

| **Sequence** | Module | Details | Condition |
|---|---|---|---|
| I | 1 | **Building block 41** | T1 = -30°C, T2 = -10°C |
| | 2 | Fmoc Removal | r.t for 5 min |
| II | 1 | **Building block 35** | T1 = -30°C, T2 = 0°C |
| | 2 | Fmoc Removal | r.t for 5 min |
| III | 1 | **Building block 32** | T1 = - 40 °C, T2 = - 20 °C |
| | 2 | Fmoc Removal | r.t for 5 min |
| IV | 1 | **Building block 42** | T1 = -30°C, T2 = 0°C |
| | 2 | Fmoc Removal | r.t for 5 min |

8 % from the resin, MS ESI+-HRMS m/z [M+Na]+ calcd for C₁₁₀H₁₁₇NO₂₇Na 1906.7711, found 1906.7590.

### Example 1-32: Preparation of 5-amino pentanyl β-D-glucopyranosyl uronic acid-(1 →4)-β-D-glucopyranosyl-(1→4)-α-D-glucopyranosyl-(1→4)-α-D-galactopyranoside (19)

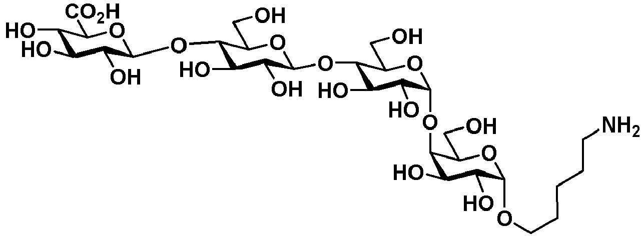

Tetrasaccharide **19a** was subjected to the general deprotection procedure to afford tetrasaccharide **19:** 36%; MS ESI+-HRMS m/z [M+H]+ calcd for C₂₉H₅₂NO₂₂ 766.2975, found 766.2988.

### Example 1-33: Synthesis of N-benzyloxycarbonyl-5-amino-pentanyl 4-O-acetyl-2,3,6-tri-O-benzyl-α-D-galactopyranosyl-(1→4)-methyl 2-O-benzoyl-3-O-benzyl-β-D-glucopyranosyluronate-(1→4)-2-O-benzoyl-3,6-di-O-benzyl-β-D-glucopyranosyl-(1→4)-6-O-acetyl-2,3-di-O-benzyl-α-D-glucopyranoside (20a)

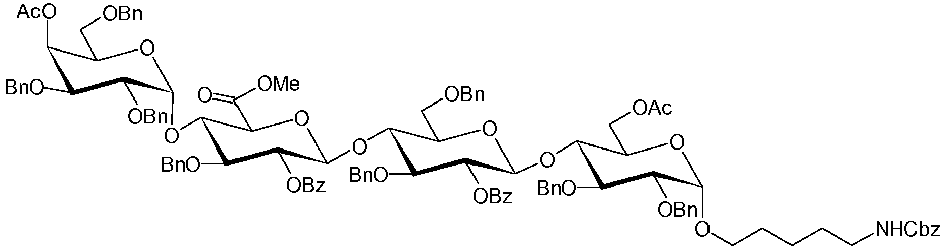

Tetrasaccharide **20a** was prepared according to the following reactions sequence:

| **Sequence** | Module | Details | Condition |
|---|---|---|---|
| I | 1 | **Building block 35** | T1 = - 30 °C, T2 = 0 °C |
| | 2 | Fmoc Removal | r.t for 5 min |
| II | 1 | **Building block 32** | T1 = - 30 °C, T2 = -10 °C |
| | 2 | Fmoc Removal | r.t for 5 min |
| III | 1 | **Building block 42** | T1 = - 30 °C, T2 = 0 °C |
| | 2 | Fmoc Removal | r.t for 5 min |
| IV | 1 | **Building block 40** | T1 = - 40 °C, T2 = - 20 °C |
| | 2 | Fmoc Removal | r.t for 5 min |

16% from the resin, MS ESI+-HRMS m/z [M+Na]+ calcd for C₁₁₂H₁₁₉NO₂₈Na 1948.7816, found 1948.7796.

### Example 1-34: Preparation of 5-amino pentanyl α-D-galactopyranosyl-(1→4)-β-D-glucopyranosyluronic acid-(1→4)-β-D-glucopyranosyl-(1→4)-α-D-glucopyranoside (20)

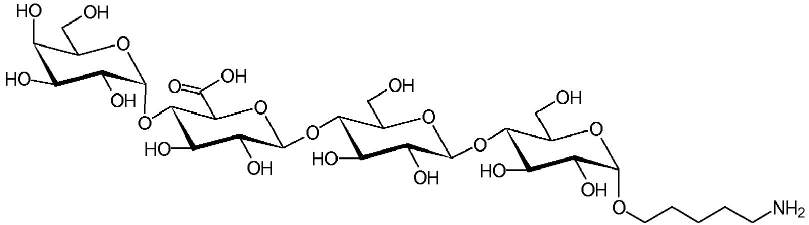

Tetrasaccharide **20a** was subjected to the general deprotection procedure to afford tetrasaccharide **20:** 40% MS ESI+-HRMS m/z [M+H]+ calcd for C₂₉H₅₂NO₂₂ 766.2975, found 766.2964.

### Example 1-35: preparation of N-benzyloxycarbonyl-5-amino-pentanyl 3,6-di-O-acetyl-2,4-di-O-benzyl-α-D-glucopyranosyl-(1→4)-2,3,6-tri-O-benzyl-α-D-galactopyranosyl-(1→4)-methyl 2-O-benzoyl-3-O-benzyl-β-D-glucopyranosyluronate-(1→4)-2-O-benzoyl-3,6-di-O-benzyl-β-D-glucopyranoside (21a):

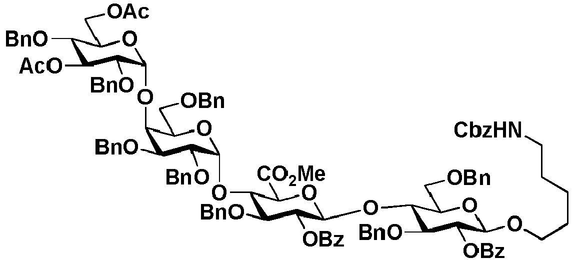

| **Sequence** | Module | Details | Condition |
|---|---|---|---|
| I | 1 | **Building block 32** | T1 = -30°C, T2 = -10°C |
| | 2 | Fmoc Removal | r.t for 5 min |
| II | 1 | **Building block 42** | T1 = -30°C,T2 = 0°C |
| | 2 | Fmoc Removal | r.t for 5 min |
| III | 1 | **Building block 41** | T1 = - 40 °C, T2 = - 20 °C |
| | 2 | Fmoc Removal | r.t for 5 min |
| IV | 1 | **Building block 37** | T1 = -30°C, T2 = 0°C |
| | 2 | Fmoc Removal | r.t for 5 min |

20% from the resin, MS ESI+-HRMS m/z [M+Na]+ calcd for C₁₁₂H₁₁₉NO₂₈Na 1948.7816, found 1950,7906

### Example 1-36: preparation of 5-Amino pentanyl α-D-glucopyranosyl-(1→4)-α-D-galactopyranosyl-(1→4)-β-D-glucopyranosyluronic acid-(1→4)-β-D-glucopyranoside (21)

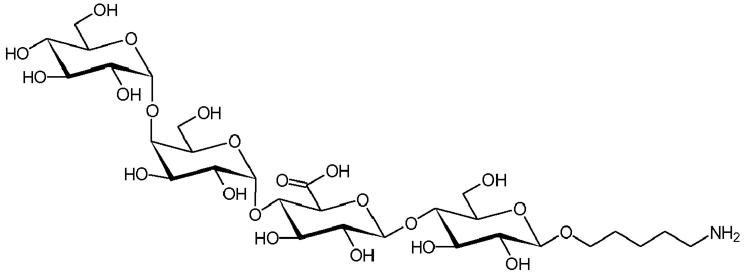

Tetrasaccharide 21a was subjected to the general deprotection procedure to afford tetrasaccharide 21: 42% MS ESI+-HRMS m/z [M+H]+ calcd for C₂₉H₅₂NO₂₂ 766.2975, found 766.2977.

### Example 1-37: Synthesis of N-benzyloxycarbonyl-5-amino-pentanyl 2-O-benzoyl-3,6-di-O-benzyl-β-D-glucopyranosyl-(1→4)-6-O-acetyl-3,4-di-O-benzyl-α-D-glucopyranosyl-(1→4)-2,3,6-tri-O-benzyl-α-D-galactopyranosyl-(1→4)-methyl 2-O-benzoyl-3-O-benzyl-β-D-glucopyranosyl uronate (22a)

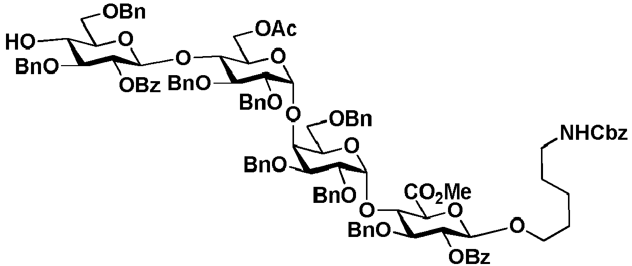

Tetrasaccharide **22a** was synthesized according to the following reactions sequence:

| **Sequence** | Module | Details | Condition |
|---|---|---|---|
| I | 1 | **Building block 42** | T1 = -30°C, T2 = 0°C |
| | 2 | Fmoc Removal | r.t for 5 min |
| II | 1 | **Building block 41** | T1 = - 40 °C, T2 = - 20 °C |
| | 2 | Fmoc Removal | r.t for 5 min |
| III | 1 | **Building block 35** | T1 = -30°C, T2 = 0°C |
| | 2 | Fmoc Removal | r.t for 5 min |
| IV | 1 | **Building block 32** | T1 = - 30 °C, T2 = -10 °C |
| | 2 | Fmoc Removal | r.t for 5 min |

21% from the resin, MS ESI+-HRMS m/z [M+Na]+ calcd for C₁₁₀H₁₁₇NO₂₇Na 1906.7711, found 1906.7624.

### Example 1-38: Synthesis of 5-amino pentanyl β-D-glucopyranosyl-(1→4)-α-D-glucopyranosyl-(1→4)-α-D-galactopyranosyl-(1→4)-β-D-glucopyranosyl uronic acid (22)

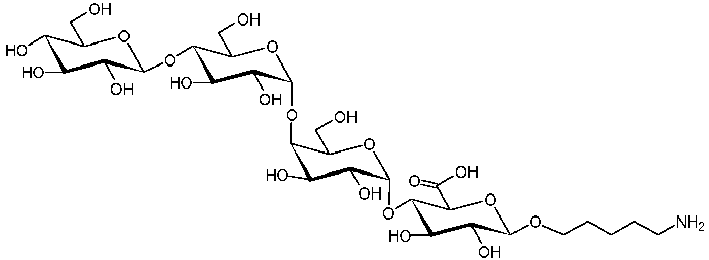

Tetrasaccharide **22a** was subjected to the general deprotection procedure to afford tetrasaccharide **22:** 52% MS ESI+-HRMS m/z [M+H]+ calcd for C₂₉H₅₂NO₂₂ 766.2975, found 766.2988.

### Biological Experiments

### Example 2.1: Synthesis of microarrays using CodeLink NHS slides

The indicated glycans were spotted onto CodeLink NHS slides using an automatic piezoelectric arraying robot (Scienion, Berlin, Germany) and incubated for 24 h (1% w/v in PBS) at room temperature. Slides were incubated in blocking buffer (100 mM ethanolamine in 50 mM NaPi pH > 9) for 30 min at room temperature, washed three times each with water and ethanol, and dried. Slides were then blocked with 1% (w/v) bovine serum albumin in phosphate buffered saline for 1 h at 37 °C, washed with water three times and dried.

### Example 2.2: Binding experiments using the microarrays synthesized according to the procedure described at example 2.1

Binding experiments were performed by incubating microarray slides coated with the saccharides of general formula (I) with either a rabbit anti-SP8 typing serum or human pneumococcal reference serum 007sp (pooled sera of 287 humans immunized with Pneumovax® vaccine purchase from National Institute for Biological Standards and Control) in the dilutions indicated in the presence or absence of native SP8 polysaccharide, and using fluorescently labeled anti-rabbit (goat anti-rabbit IgG-FITC, abcam ab6717) or anti-human secondary antibodies (Alexa Fluor 488 goat anti-human IgM, Invitrogen A21215; Alexa Fluor 647 goat anti-human IgG, Invitrogen A21445).

### Example 2.3: Conjugation of synthetic tetrasaccharides 10 and 18 to CRM197:

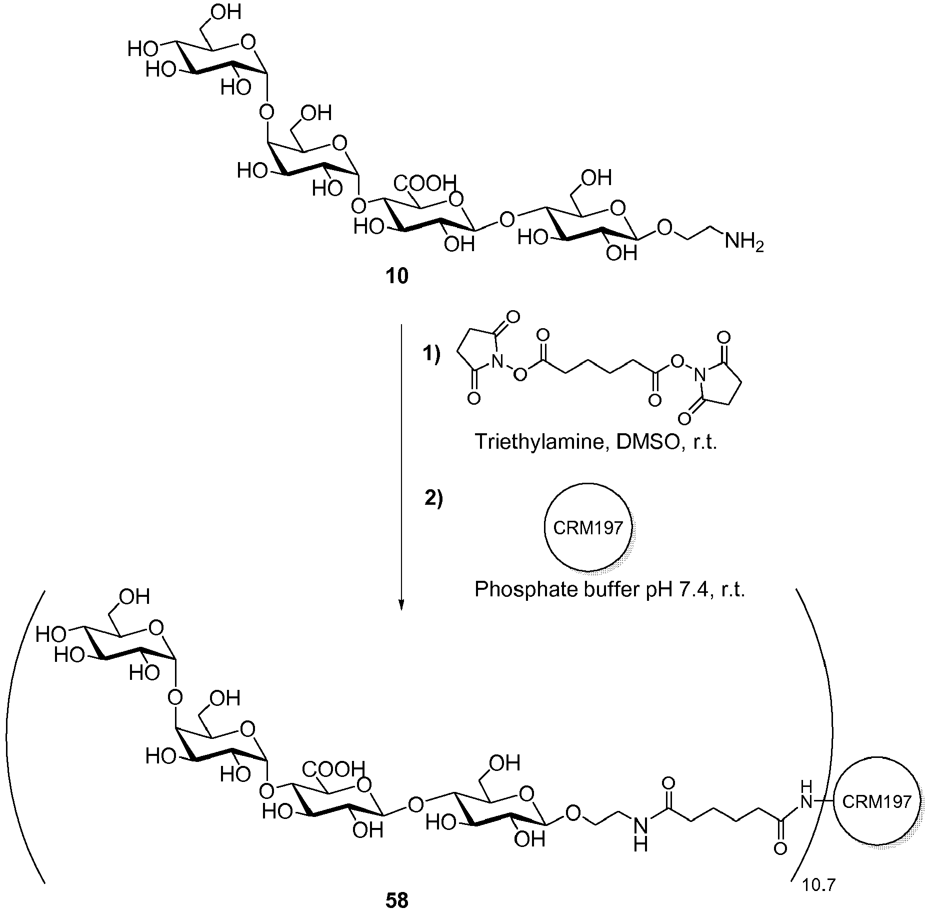

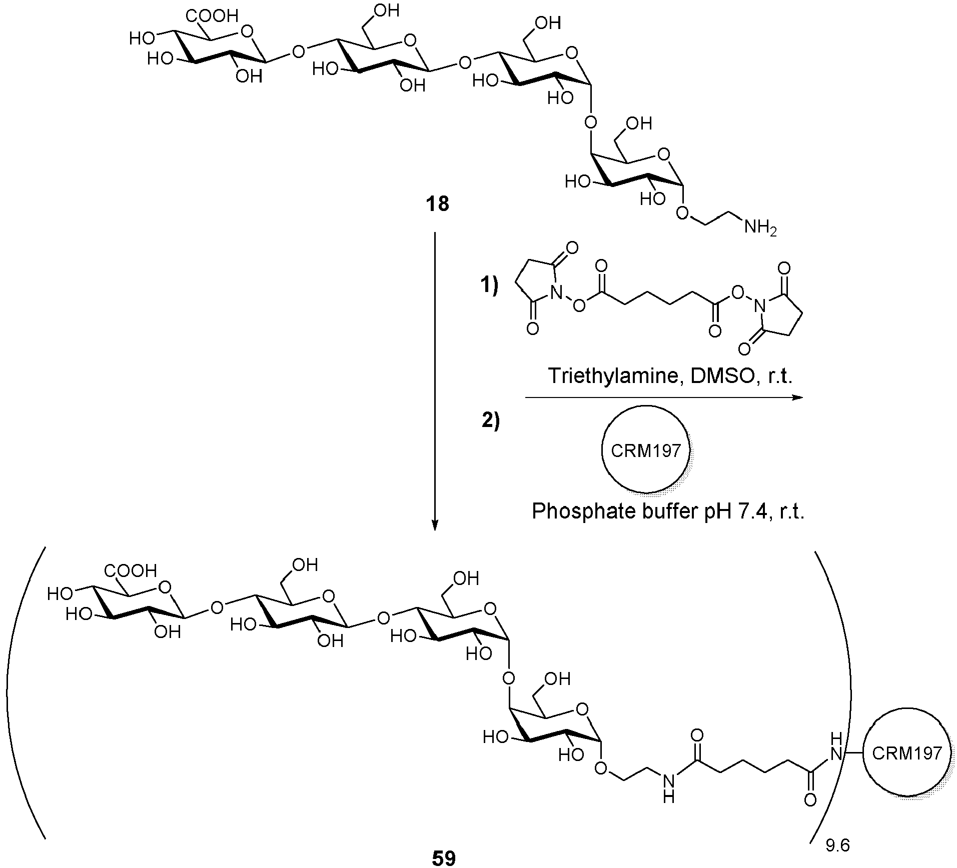

To a stirred solution of disuccinimidyl adipate (10 mg, 29 µmol) and triethylamine (10 µL, 72 µmol) in anhydrous DMSO (150 µL) was added at room temperature dropwise a suspension of tetrasaccharide **10** or **18** (approx. 2 mg, 2.8 µmol) in anhydrous DMSO (150 µL). The reaction was stirred for 2 h at that temperature under an Argon atmosphere and treated with a 100 mM sodium phosphate buffer pH 7.4 (NaPi, 200 µL). The mixture was extracted with chloroform (10 mL) and the phases separated by centrifugation (2 min, 1800 g, room temperature). The organic phase was discarded and the extraction step was repeated two times. The aqueous layer was clarified by centrifugation in a 1.5 mL reaction tube (1 min, 14500 g, room temperature) and added to a stirring solution of CRM197 (1 mg, 17.3 nmol) in NaPi (1 mL). The mixture was stirred for 16 h at room temperature and lyophilized using a centrifugal filter (10 kDa MWCO, Millipore, Darmstadt, Germany). The conjugate was characterized by MALDI-MS:
Conjugate **58:** ca. 67000 m/z (incorporation of 10.7 tetrasaccharide molecules on average)
Conjugate **59:** ca. 66000 m/z (incorporation of 9.6 tetrasaccharide molecules on average)

### Example 2.4: Immunization procedure:

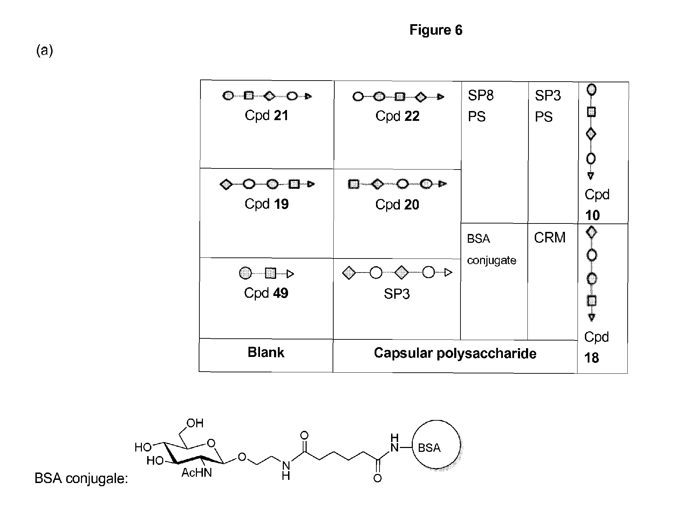

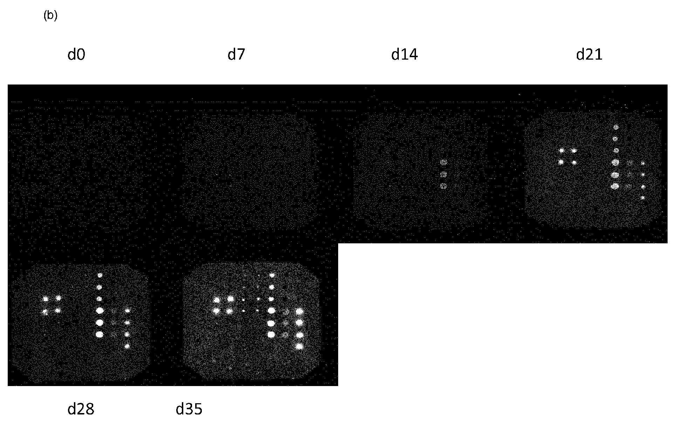

Mice (6-8 week old female Balb/c mice, Charles River) were immunized s. c. with CRM-Sp8 conjugates obtained at **example 2.3** (corresponding to 4 µg synthetic glycan) formulated either with Freund's adjuvant (Sigma-Aldrich, St. Louis, US), Alum (Alhydrogel, Brenntag) or without adjuvant at a total volume of 100 µL at days 0, 14 and 28. The immune response was monitored by glycan array. The conjugate **59** induced an oligosaccharide-specific immune response in mouse #1160 receiving the said conjugate formulated with Freund's adjuvant. Importantly, a robust immune response was observed in immune serum from mouse #1160 against the native Sp8 polysaccharide (see **Figure 6****).**

### Example 2.5: Generation of monoclonal antibodies:

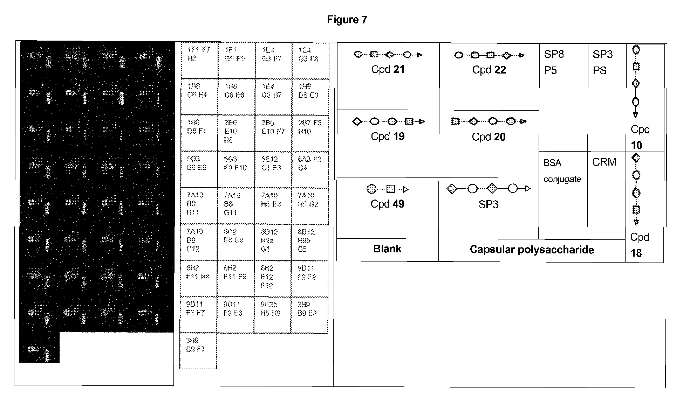

Monoclonal antibodies were prepared using BM-Condimed H1 (Roche, Penzberg, Germany) according to the manufacturer's instructions. Following fusion, single clones were generated using limited dilution and two subsequent rounds of subcloning. Antibody production was monitored by glycan array and ELISA. 33 clones were eventually isolated that produced mAbs recognizing both tetrasaccharide **19** and Sp8 native polysaccharide. Clone 1H8 was expanded in serum-free medium and mAb 1H8 was purified from the cell culture supernatant using a Protein G Antibody Purification kit (Pro-Chem, Littleton, USA) (see **Figure 7****).**

### Example 2.6: Enzyme-linked immunosorbent assay (ELISA):

ELISA was performed using high-binding polystyrene 96-well plates (Corning, Corning, US). Plates were coated using native Sp8 polysaccharide (SSI Diagnostica, Kopenhagen) at a concentration of 10 µg/mL in PBS for 20 h at 4 °C. Plates were blocked with 10% (v/v) fetal calve serum in PBS for 1 h at 37 °C and washed once with PBS containing 0.1% (v/v) Tween 20 (PBS-T). Cell culture supernatants of anti-Sp8 mAbs (50 µL) were applied. Plates were incubated for 1 h at 37 °C, washed with PBS-T three times and treated with a horseradish peroxidase (HRP)-labeled secondary antibody (goat anti-mouse IgG HRP conjugate, dianova, Hamburg, Germany). Plates were washed with PBS-T three times and HRP activity was measured with TMB substrate (BD Biosciences, San Jose, US) according to the manufacturer's instructions. Monoclonal antibodies generated from #1160 specifically recognized both synthetic saccharide **19** and the native Sp8 polysaccharide, as assessed by ELISA.

### Example 2.7: Surface Plasmon Resonance:

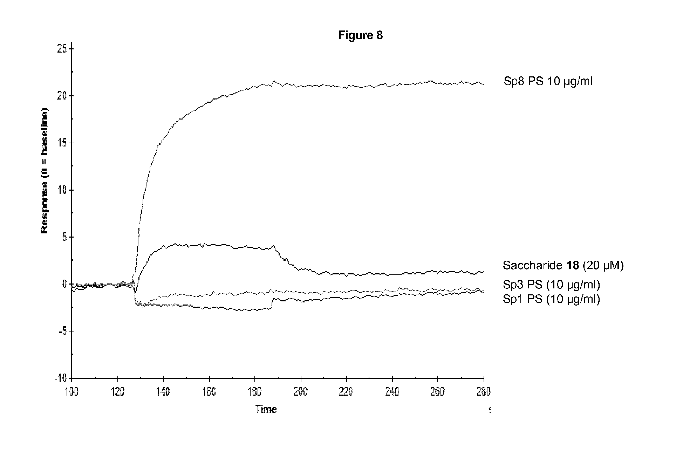

Surface Plasmon Resonance was performed on a Biacore T100 instrument (GE Healthcare, Little Chalfont, UK). Analysis was performed using the Mouse Antibody Capture Kit and Amine Coupling Kit (GE Healthcare) for immobilization. About 10000 response units (RU) of capture antibody were immobilized. A commercial mouse IgG (cat. no. 026502, Invitrogen, Carlsbad, US) was immobilized as a dummy in a reference cell (about 10000 RU). About 800 RU of mAb 1H8 were captured prior to every run using a mAb concentration of 50 µg/mL. Runs were performed using PBS as a running buffer and a flow rate of 30 µL/min with 60 s association and 120 s dissociation periods, respectively. Polysaccharides were used at a concentration of 10 µg/mL and saccharide **18** at 20 µM in PBS. Monoclonal antibody mAb 1H8 specifically recognizes both synthetic saccharide **18** and the native Sp8 polysaccharide (see **Figure 8****).**

### Example 2.8: Immunofluorescence of UV-inactivated S. pneumoniae:

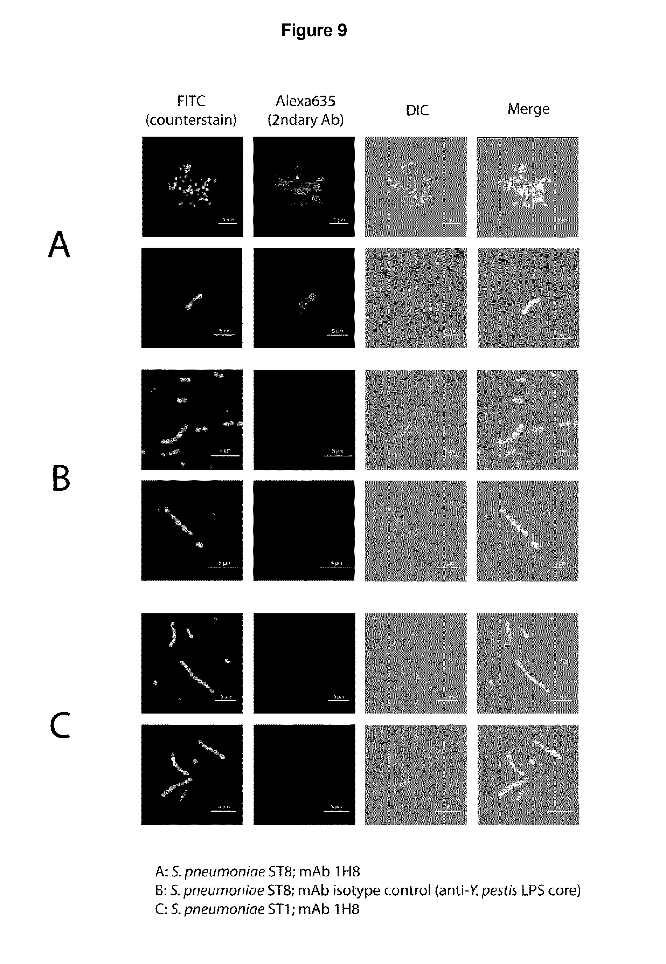

*S. pneumoniae* serotype 8 (ATCC 6308) or serotype 1 (ATCC 6301) (approx. 4x10⁸ cfu/mL) were inactivated by irradiation with 254 nm for 10 min in PBS at room temperature. Cells were harvested by centrifugation, washed once with PBS and frozen in Todd Hewitt Broth containing 0.5% (w/v) yeast extract and 20% (v/v) glycerol. For immunofluorescence, bacteria (8x10⁸ cfu ST8 or 4x10⁸ cfu ST1) were thawed, harvested by centrifugation (16800 g, 15 min, r.t.) and washed once in buffer A (50 mM NaHCO₃, 100 mM NaCl, pH 7.5). Cells were resuspended in buffer A (1 mL) and treated with a fluorescein isothiocyanate (FITC, Sigma-Aldrich) solution (10 mg/mL in DMSO) to a final concentration of 0.1 mg/mL. Bacteria were labeled in the dark for 1 h at 37 °C, harvested by centrifugation and washed twice with 0.25% (w/v) BSA in PBS (1 mL). Labeling was monitored by fluorescence microscopy using an Axio Imager.M2 system equipped with a LSM 700 confocal laser scanning microscope (Carl Zeiss Microscopy GmbH, Jena, Germany). Cells were suspended in 1% (w/v) BSA in PBS (1 mL for ST8, 0.5 mL for ST1) and the suspension was distributed into two aliquots. The suspensions were treated with mAb 1H8 or mAb 1 E12 (IgG1) against *Yersinia pestis* lipopolysaccharide core trisaccharide as an isotype control to a final mAb concentration of 10 µg/mL. Bacteria were incubated in the dark for 16 h at 4 °C under agitation and washed with 1% (w/v) BSA in PBS (0.5 mL). The cells were suspended in a solution of goat anti-mouse IgG-Alexa635 conjugate (1:100 dilution in 200 µL 1% (w/v) BSA in PBS, Invitrogen), incubated in the dark for 1.5 h at room temperature and washed with 1% (w/v) BSA in PBS and PBS (0.5 mL, respectively). Fluorescently labeled bacteria were visualized by fluorescence microscopy and images were processed with using Zen 2011 software (Carl Zeiss Microscopy GmbH). As shown in **Figure 9****,** immunization with conjugate **59** induces the formation of antibodies that recognize the native SP-8 polysaccharide and ST8 bacteria.

## Claims

1. A saccharide of general formula (I)
**V*-[Uₓ₊₃-Uₓ₊₂-Uₓ₊₁-Uₓ]ₙ-V-O-L-NH₂** (I)
wherein
x is an integer selected from 1, 2, 3 and 4;
n is an integer selected from 1, 2 and 3; 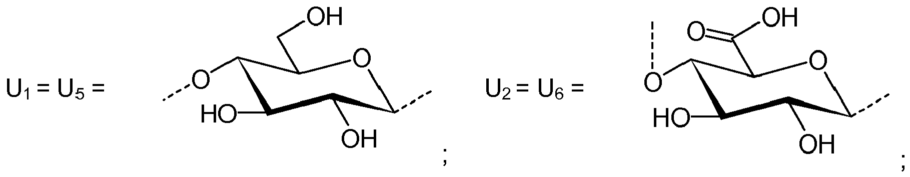 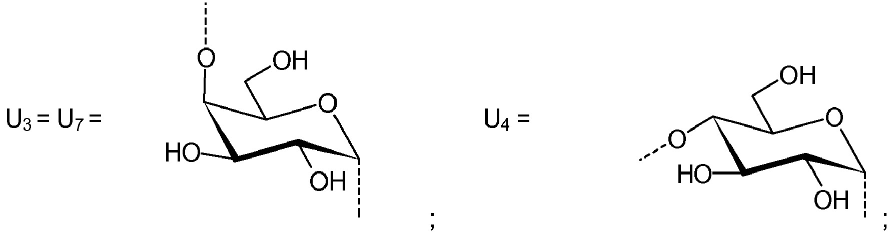
-V- represents a bond, -Uₓ₊₃-, -Uₓ₊₃-Uₓ₊₂- or -Uₓ₊₃-Uₓ₊₂-Uₓ₊₁-; V*- represents H-, H-Uₓ-, H-Uₓ₊₁-Uₓ-, H- Uₓ₊₂-Uₓ₊₁-Uₓ- ;
L represents a linker.

2. The saccharide according to claim 1, of general formula **(II)**
**V*-[Ux₊₃-Uₓ₊₂-Uₓ₊₁-Uₓ]ₙ-O-L-NH₂** (II)
wherein x, n, L, Uₓ, Uₓ₊₁, Uₓ₊₂, Uₓ₊₃ and V* have the meanings as defined in claim 1.

3. The saccharide according to claim 1, of general formula **(III)**
**V*-[Uₓ₊₃-Uₓ₊₂-Uₓ₊₁-Uₓ]ₙ-Uₓ₊₃-O-L-NH₂** (III)
wherein x, n, L, Uₓ, Uₓ₊₁, Uₓ₊₂, Uₓ₊₃ and V* have the meanings as defined in claim 1.

4. The saccharide according to claim 1, of general formula **(IV)**
**V*-[Uₓ₊₃-Uₓ₊₂-Uₓ₊₁-Uₓ]ₙ-Uₓ₊₃-Uₓ₊₂-O-L-NH₂** (IV)
wherein x, n, L, Uₓ, Uₓ₊₁, Uₓ₊₂, Uₓ₊₃ and V* have the meanings as defined in claim 1.

5. The saccharide according to claim 1, of general formula **(V)**
**V*-[Uₓ₊₃-Uₓ₊₂-Uₓ₊₁-Uₓ]ₙ-Uₓ₊₃-Uₓ₊₂-Uₓ₊₁-O-L-NH₂** (V)
wherein x, n, L, Uₓ, Uₓ₊₁, Uₓ₊₂, Uₓ₊₃ and V* have the meanings as defined in claim 1.

6. The saccharide according to any of the claims 1 - 5, wherein x represents 3 and V*- represents H-.

7. The saccharide according to claim 1 selected from the group consisting of:
α-D-glucopyranosyl-(1**→**4)-α-D-galactopyranosyl-(1**→**4)-β-D-glucopyranosyluronic acid-(1 **→**4)-β-D-glucopyranosyl-(1**→**1)-(2-amino)ethanol **(10),**
β-D-glucopyranosyluronic acid-(1 **→**4)-β-D-glucopyranosyl-(1**→**4)-α-D-glucopyranosyl-(1**→**4)-α-D-galactopyranosyl-(1**→**1)-(2-amino)ethanol **(18),**
5-amino pentanyl β-D-glucopyranosyl uronic acid-(1**→**4)-β-D-glucopyranosyl-(1**→**4)-α-D-glucopyranosyl-(1**→**4)-α-D-galactopyranoside **(19),**
5-amino pentanyl α-D-galactopyranosyl-(1**→**4)-β-D-glucopyranosyluronic acid-(1 **→**4)-β-D-glucopyranosyl-(1**→**4)-α-D-glucopyranoside **(20),**
5-amino pentanyl α-D-glucopyranosyl-(1**→**4)-α-D-galactopyranosyl-(1**→**4)-β-D-glucopyranosyluronic acid-(1**→**4)-β-D-glucopyranoside **(21),**
5-amino pentanyl β-D-glucopyranosyl-(1**→**4)-α-D-glucopyranosyl-(1**→**4)-α-D-galactopyranosyl-(1**→**4)-β-D-glucopyranosyl uronic acid **(22),**
α-D-galactopyranosyl-(1**→**4)-β-D-glucopyranosyluronate-(1**→**4)-β-D-glucoyranosyl-α-D-glucopyranosyl-(1**→**4)-α-D-galactopyranosyl-(1**→**1)-(2-amino)ethanol **(55),**
α-D-glucopyranosyl-(1**→**4)-α-D-galactopyranosyl-(1**→**4)-β-D-glucopyranosyluronate-(1**→**4)-β-D-glucoyranosyl-(1**→**4)-α-D-glucopyranosyl-(1**→**4)-α-D-galactopyranosyl-(1**→**1)-(2-amino)ethanol **(57).**

8. A compound of the general formula **(I')**
**V*-[Uₓ₊₃-Uₓ₊₂-Uₓ₊₁-Uₓ]ₙ-V-O-** (I')
covalently linked to an immunogenic carrier through the O atom,
wherein V*, Uₓ₊₃, Uₓ₊₂, Uₓ₊₁, Uₓ, V, x and n have the meanings as defined in claim 1.

9. A saccharide according to any of the claims 1 - 7 or a compound of general formula **(I')** covalently linked to an immunogenic carrier through the O atom according to claim 8 for use in raising a protective immune response in a human and/or animal host.

10. A saccharide according to any of the claims 1 - 7 or a compound of general formula **(I')** covalently linked to an immunogenic carrier through the O atom for use in the prevention and/or treatment of a disease associated with bacteria containing in their capsular polysaccharide one of the following saccharide fragments:
α-D-Glc*p*-(1**→**4)-α-D-Gal*p*-(1**→**4)-β-D-GlcA*p*-(1**→**4)-β-D-Glc*p*,
β-D-Glc*p*-(1**→**4)-α-D-Glc*p*-(1**→**4)-α-D-Gal*p*-(1→4)-β-D-GlcA*p*,
β-D-GlcA*p*-(1**→**4)-β-D-Glc*p*-(1**→**4)-α-D-Glc*p*-(1**→**4)-α-D-Gal*p*,
α-D-Gal*p*-(1**→**4)-β-D-GlcA*p*-(1**→**4)-β-D-Glc*p*-(1**→**4)-α-D-Glc*p*.

11. The saccharide for use or the compound of general formula **(I')** covalently linked to an immunogenic carrier through the O atom for use according to claim 10, wherein the bacteria is *Streptococcus pneumoniae* serotype 8.

12. The saccharide for use or the compound of general formula **(I')** covalently linked to an immunogenic carrier through the O atom for use according to claim 10, wherein the disease associated with bacteria is selected from the group comprising: pneumonia, meningitis, otitis media, bacteremia and acute exacerbation of chronic bronchitis, sinusitis, arthritis and conjunctivitis.

13. A vaccine comprising the saccharide according to any of the claims 1 - 7 and/or the compound of general formula **(I')** covalently linked to an immunogenic carrier through the O atom together with at least one pharmaceutical acceptable adjuvant, cryoprotectant, lyoprotectant, excipient and/or diluent.

14. A saccharide according to any of the claims 1 - 7 for use as marker in immunological assays for detection of antibodies against bacteria containing in their capsular polysaccharide one of the following saccharide fragments:
α-D-Glc*p*-(1**→**4)-α-D-Gal*p*-(1**→**4)-β-D-GlcA*p*-(1**→**4)-β-D-Glc*p*,
β-D-Glc*p*-(1**→**4)-α-D-Glc*p*-(1**→**4)-α-D-Gal*p*-(1**→**4)-β-D-GlcA*p*,
β-D-GlcA*p*-(1**→**4)-β-D-Glc*p*-(1**→**4)-α-D-Glc*p*-(1**→**4)-α-D-Gal*p*,
α-D-Gal*p*-(1**→**4)-β-D-GlcA*p*-(1**→**4)-β-D-Glc*p*-(1**→**4)-α-D-Glc*p*.
